# EUROPEAN PATENT SPECIFICATION

(11) **EP 1 558 729 B1**
(45) Date of publication and mention of the grant of the patent: **09.12.2009**
(21) Application number: 03754599.3
(22) Date of filing: 16.09.2003
(51) Int. Cl.: C12N 9/14, C12N 1/20, C12N 15/00, C12Q 1/00, C12Q 1/34, C12Q 1/68, C12P 21/06, C12P 21/04, A61K 38/44, C07H 21/04, C07K 1/00, C12N 9/62

(54) **RAPIDLY DEGRADED REPORTER FUSION PROTEINS**
SCHNELL ABGEBAUTE REPORTER-FUSIONSPROTEINE
PROTEINES HYBRIDES RAPPORTEURS RAPIDEMENT DEGRADEES

(30) Priority: 16.09.2002 US 411070 P; 20.09.2002 US 412268 P
(43) Date of publication of application: 03.08.2005
(73) Proprietor: PROMEGA CORPORATION, Madison, Wisconsin 53711 (US)
(72) Inventor: ZDANOVSKY, Alexey, Madison, WI 53711 (US); ZDANOVSKAIA, Marina, Madison, WI 53711 (US); MA, Dongping, Madison, WI 53710 (US); WOOD, Keith V., Mount Horeb, WI 53572 (US); ALMOND, Brian D., Fitchburg, WI 53719 (US); WOOD, Monika G., Mount Horeb, WI 53572 (US)
(74) Representative: Icely, Dominic Michael
(86) International application number: PCT/US2003/028939
(87) International publication number: WO 2004/025264

(56) References cited:
- WO-A-00/39314
- WO-A-01/64921
- WO-A-02/072844
- US-A- 5 976 796
- US-A- 6 103 313
- CORISH PETE ET AL: "Attenuation of green fluorescent protein half-life in mammalian cells" PROTEIN ENGINEERING, vol. 12, no. 12, December 1999 (1999-12), pages 1035-1040, XP002378153 ISSN: 0269-2139
- LECLERC G M ET AL: "Development of a destabilized firefly luciferase enzyme for measurement of gene expression" BIOTECHNIQUES, INFORMA LIFE SCIENCES PUBLISHING, WESTBOROUGH, MA, US, vol. 29, no. 3, 2000, pages 590-591,594,59, XP002988846 ISSN: 0736-6205
- GILON ET AL: "Degradation signals for ubiquitin system proteolysis in Saccharomyces cerevisiae" EMBO J, vol. 17, no. 10, 1998, pages 2759-2766,

## Description

### Field of the Invention

This invention relates to the field of biochemical assays and reagents. More specifically, this invention relates to modified reporter proteins, e.g., fluorescent reporter proteins, and to methods for their use.

### Background of the Invention

Luciferases are enzymes that catalyze the oxidation of a substrate (luciferin) with the concomitant release of photons of light. Luciferases have been isolated from numerous species, including Coleopteran arthropods and many sea creatures. Because it is easily detectable and its activity can be quantified with high precision, luciferase/luciferin enzyme/substrate pairs have been used widely to study gene expression and protein localization. Unlike another reporter protein, green fluorescent protein (GFP), which requires up to 30 minutes to form a chromophore, the products of luciferases can be detected immediately upon completion of synthesis of the polypeptide chain (if substrate and oxygen are also present). As luciferase is a useful reporter in numerous species and in a wide variety of cells, luciferases are ideal for monitoring gene up-regulation. However, the stability of native luciferases and native GFP can functionally mask reliable detection of gene down-regulation.

Protein degradation is necessary to rid cells of damaged and non-functioning proteins. Intracellular degradation of proteins is a highly selective process that allows some proteins to survive for hours or days, while other proteins survive for only minutes, inside the cell. In recent years, the processes controlling protein degradation have become an important area of study, further prompted perhaps by reports that the failure of key components in protein degradation can be causative in human disease (Bence et al., 2001; McNaught et al., 2001).

Protein degradation is not limited to the removal of damaged or otherwise abnormal proteins, as a number of regulatory circuits involve proteins with short half-lives (relatively "unstable" proteins). For example, proteolysis plays an important regulatory role in many cellular processes including metabolic control, cell cycle progression, signal transduction and transcription

(Hicke, 1997; Joazeiro et al., 1999; Murray et al., 1989; Salghetti et al., 2001). A great part of selective protein degradation in eukaryotes appears to be carried out in the proteosome, an ATP-dependent multi-protein complex. In many degradation pathways, the covalent conjugation of ubiquitin, a 76 amino acid polypeptide, to proteins destined for degradation, precedes degradation in the proteosome (Hershko et al., 1992).

Ornithine decarboxylase (ODC) is an enzyme which is critical in the biosynthesis of polyamines and is known to have a very short cellular half-life. In fact, murine ornithine decarboxylase (mODC) is one of most short-lived proteins, with a half-life of about 30 minutes (see Ghoda et al., 1989; Ghoda et al. 1992). Rapid degradation of ODC has been attributed to the unique composition of its C-terminus which includes a "PEST" sequence (Rogers et al., 1989; Reichsteiner, 1990). A PEST sequence contains a region enriched with proline (P), glutamic acid (E), serine (S), and threonine (T), that is often flanked by basic amino acids, lysine, arginine, or histidine. The PEST sequence targets the PEST containing protein towards the 26S proteosome without prior ubiquitinization (Gilon et al., 1998; Leclerc et al., 2000; Corish et al., 1999; Li et al., 1998; Li et al., 2000). Leclerc et al., 2000 more particularly discloses a construct in which a coding sequence for a firefly luciferase is linked to a mODC coding sequence including a PEST sequence and reports that such a construct allows for detection of rapid increases or decreases in gene expression. The PEST sequence was shown to reduce the half-life of firefly luciferase from about 3_68 hours to about 0.84 hours.

Deletion of the C-terminal PEST containing region from mODC prevents its rapid degradation (Ghoda et al., 1989). In contrast to mODC, the ODC of *Trypanosoma brucei* (TbODC) does not have a PEST sequence, and is long-lived and quite stable when expressed in mammalian cells (Ghoda et al. 1990). However, fusion of the C-terminus of mODC to TbODC results in an unstable protein. As further illustration of development of destabilized reporter proteins by fusing the coding sequence of the reporter with a destabilization sequence which destabilizes the reporter polypeptide, for instance, the C-terminal region of ODC (C-ODC) has been shown to reduce the half-life of GFP from about 26 hours to about 9.8 hours (Corish, 1999) and, when a mutant form of C-ODC was used, to less than 2 hours (Li, 1998; Li, 2000).

Another group has developed destabilized reporter proteins by alternatively fusing the coding sequence of the reporter with a destabilization sequence which destabilizes mRNA encoding the reporter. Fan et al. (1997) found that the presence of an AU-rich region from a herpes virus RNA conferred instability to that RNA as well as to heterologous RNA, thereby destabilizing the mRNA. Published International Application WO 00/39314 (Novation Pharmaceuticals Inc.) discloses a construct having a coding region for luciferase linked to a mRNA destabilization sequence and its use to screen for compounds that promote RNA degradation.

Peptide signals other than C-ODC that have been used for destabilization of proteins include the cyclin destruction box (Corish et al., 1999; King et al., 1996), the PEST-rich C-terminal region of cyclin (Mateus et al., 2000), CL peptides, e.g., CL1 (Gilon et al., 1998; Bence et al., 2001) and N-degron. Although all of these signals direct proteins containing them towards degradation by the proteosome, the pathways followed by these proteins before they reach the proteosome may be different. For instance, degradation of ODC occurs in the 26S proteosome in the absence of prior ubiquitination (Bercovich et al., 1989; Murakami et al., 1992), while CL peptides channel proteins for degradation via a pathway that depends upon the presence of ubiquitin-conjugating enzymes (Gilon et al., 1998): Degradation of cyclins is also a ubiquitination-dependent process (Glotzer et al., 1991). Nevertheless, cyclins become unstable only when cells exit mitosis (Hunt et al., 1992).

More than fifteen years ago Bachmir et al. (1986) described the N-degron degradation signal. These authors reported that certain amino acid residues, if positioned at the N-terminus of the protein, can stimulate the use of internal protein lysine residues by ubiquitin ligase as a point for attachment of ubiquitin. As a result such N-terminal residues (called destabilizing residues) caused dramatic destabilization of the corresponding protein. The relationship between the identity of the N-terminal residue and the *in vivo* half-life of the corresponding protein has been referred by these authors as the N-end rule (Varshavsky, 1992). This rule has been extensively studied in yeast where species of β-galactosidase with N-terminal Arg, Lys, Phe, Leu, Trp, His, Asp, Asn, Tyr, Gln, Ile or Glu had half-lives of 2-30 minutes. At the same time, β-galactosidase species with any of the other eight amino acid residues had half-lives of more than 20 hours (Varshavsky, 1992). The most efficient destabilizing residue with a corresponding half-life of 2 minutes was found to be arginine. It has been reported that the N-end rule also operates in *E*. *coli,* where an arginine residue was found to be the most effective destabilizing residue (Tobias et al., 1991). In *E*. *coli,* similarly to yeast, positioning of this residue at the N-tenninus of β-galactosidase resulted in a protein that had a half-life of 2 minutes instead of more than 10 hours (Varshavsky, 1992; Tobias et al., 1991). Several groups have reported data supporting the existence of the N-end rule in rabbit, mouse and tobacco (Varshavsky, 1992; Reiss et al., 1988; Kwon et al., 1998; Townsend et al., 1988).

However, what is needed is an improved recombinant reporter protein, e.g., for use in higher eukaryotes.

### Summary of the Invention

The invention provides improved gene products, e.g., reporter proteins, with reduced or decreased, e.g., substantially reduced or decreased, half-lives, of expression, which are useful to determine or detect gene expression, e.g., up- or down-regulation, to monitor promoter activity, to reduce cytotoxicity, and to localize proteins. In one embodiment, the invention provides an isolated nucleic acid molecule (polynucleotide) comprising a nucleic acid sequence comprising an open reading frame for a reporter protein, e.g., a luciferase, GFP, chloramphenicol acetyltransferase, beta-glucuronidase or beta-galactosidase, and at least two heterologous destabilization sequences, wherein one of the heterologous destabilization sequences is a mRNA destabilization sequence and another is a heterologous in frame protein destabizastion sequence that includes at least one CL1, CL2, CL6, CL9, CL10, CL11, CL12, CL15, CL16 or SL17 as defined in Gilon et al., 1998 and listed as SEQ. ID. No. 12 (CL1) and SEQ ID Nos. 91 to 99 (CL2, CL6, CL9, CL10, CL11, CL12, CL15, CL16 and SL17 respectively). As used herein, a "heterologous" destabilization sequence is one which is not found in the wild-type gene for the reporter protein employed in the fusion polypeptide. The presence of one or more destabilization sequences in a nucleic acid molecule of the invention which is introduced to a host cell or to an *in vitro* transcription/translation mixture, results in reporter activity (expression) that is reduced or decreased, e.g., a substantially reduced or decreased half-life of reporter expression, relative to the reporter activity for a corresponding reporter protein gene that lacks one or more of the destabilization sequences. For example, the presence of one or more protein destabilization sequences in a fusion polypeptide encoded by a nucleic acid molecule of the invention results in a reduction or decrease in the half-life of the fusion polypeptide relative to a corresponding protein which lacks the destabilization sequence(s). The presence of one or more RNA destabilization sequences in a nucleic acid molecule of the invention results in a reduction or decrease in the half-life of the mRNA transcribed from that nucleic acid molecule relative to a nucleic acid molecule which lacks the destabilization sequence(s). Preferably, the nucleic acid molecule of the invention comprises sequences which have been optimized for expression in mammalian cells, and more preferably, in human cells (see, e.g., WO 02/16944 which discloses methods to optimize sequences for expression in a cell of interest). For instance, nucleic acid molecules may be optimized for expression in eukaryotic cells by introducing a Kozak sequence and/or one or more introns, and/or by altering codon usage to codons employed more frequently in one or more eukaryotic organisms, e.g., codons employed more frequently in an eukaryotic host cell to be transformed with the nucleic acid molecule.

A protein destabilization sequence includes one or more amino acid residues, which, when present at the N-terminus or C-terminus of a protein of interest, reduces or decreases, e.g., having a reduction or decrease in the half-life of the protein of interest of at least 80%, preferably at least 90%, more preferably at least 95% or more, e.g., 99%, relative to a corresponding protein which lacks the protein destabilization sequence. The presence of the protein destabilization sequence in a fusion polypeptide preferably does not substantially alter other functional properties of the protein of interest. As indicated above, for the purpose of the invention a protein destabilization sequence includes, at least one, CL sequence selected from CL1, CL2, CL6, CL9, CL10, CL11, CL12, CL15, CL16 or SL17.

A mRNA destabilization sequence includes two or more nucleotides, which, when present in a RNA, reduces or decreases, e.g., substantially reduces or decreases, for instance, having a reduction or decrease in the half-life of the mRNA encoding a protein of interest of at least 20%, including 50%, 70% or greater, e.g., 90% or 99%, relative to a RNA that lacks the RNA destabilization sequence and encodes the corresponding protein. In one embodiment, a mRNA destabilization sequence has less than about 100 nucleotides. A mRNA destabilization sequence includes, but is not limited to, a sequence present in the 3' UTR of a mRNA which likely forms a stem-loop, one or more AUUUA or UUAUUUAUU sequences, including the 3' UTR of the bradykinin B1 receptor gene.

In one embodiment, the nucleic acid molecule is present in a vector, e.g., a plasmid. In one embodiment, the nucleic acid molecule encodes a destabilized fusion polypeptide comprising a reporter protein, which nucleic acid molecule comprises SEQ ID NO:47, SEQ ID NO:48, SEQ ID NO:49, SEQ ID NO:66, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78, SEQ ID NO:79; SEQ ID NO:80, or a fragment thereof that encodes a fusion polypeptide with substantially the same activity as the corresponding full-length fusion polypeptide. As used herein, "substantially the same activity" is at least about 70%, e.g., 80%, 90% or more, the activity of a corresponding full-length fusion polypeptide.

As described herein, the combination of two protein degradation (CL1 and mODC) sequences in the same luciferase fusion polypeptide resulted in a reduction of the half-life of firefly luciferase to about 30 minutes and *Renilla* luciferase to about 20 minutes. Also, N-degron and mODC complemented each other in that the combination of these two degradation signals in the same protein resulted in a substantial increase in the rate of degradation of the corresponding protein. Moreover, introduction of a mRNA destabilization 3' to the open reading frame for the luciferase fusion polypeptide decreased the half-life of luciferase expression by destabilizing sequence transcription. Further, the combination of a mRNA and a protein destabilization sequence was shown to be effective in at least 3 different cells (HeLa, CHO and 293 cells) in shortening the expression of two different luciferase proteins. In addition, the presence of mammalian cell-optimized sequences for a fusion polypeptide of the invention, in cells transfected with a plasmid comprising those sequences, enhanced the amount of light emitted by those cells as a result of the more efficient translation of RNA encoding the fusion polypeptide. Thus, the presence of optimized sequences including codon optimized sequences in a nucleic acid molecule encoding a fusion polypeptide of the invention, e.g., optimized sequences for the reporter protein, optimized sequences for the protein destabilization signal(s), or both, can yield an enhanced signal.

In one embodiment, the nucleic acid molecule comprises a nucleic acid sequence encoding a fusion polypeptide comprising at least one and preferably at least two heterologous protein destabilization signals, which fusion polypeptide has a half-life that is substantially reduced or decreased, e.g., having at least a 80%, preferably at least a 90%, more preferably at least a 95% or more, e.g., 99%, reduction or decrease in half-life, relative to the half-life of a corresponding wild-type protein, and/or emits more light as a result of the optimization of the nucleic acid sequences for expression in a desired cell relative to a fusion polypeptide encoded by sequences which are not optimized for expression in that cell. In one embodiment, the reporter protein is a luciferases, for instance, a Coleopteran or anthozoan luciferase such as a firefly luciferase or a *Renilla* luciferase, and the luciferase fusion polypeptide includes at least one heterologous protein destabilization sequence and has a substantially reduced half-life relative to a corresponding wild-type (native or recombinant) luciferase. Preferably, optimized nucleic acid sequences encoding at least the reporter protein are employed, as those optimized sequences can increase the strength of the signal for destabilized reporter proteins.

In another embodiment, the nucleic acid molecule comprises at least one heterologous mRNA destabilization sequence and encodes a fusion polypeptide comprising at least one heterologous protein destabilization sequence. Preferably, the mRNA destabilization sequence is 3' to the nucleic acid sequence encoding the fusion polypeptide. In one embodiment, the expression of the fusion polypeptide is reduced relative to a polypeptide encoded by a nucleic acid molecule which lacks the heterologous destabilization sequences.

In one embodiment, the nucleic acid molecule comprises a nucleic acid sequence comprising an open reading frame for a reporter protein and at least one heterologous destabilization sequence, wherein a majority of codons in the open reading frame for the reporter protein are optimized for expression in a particular host cell, e.g., a mammalian cell such as a human cell. The presence of codon optimized sequences in the nucleic acid molecule can compensate for reduced expression from a corresponding nucleic acid molecule which is not codon optimized.

The invention further includes a vector and host cell comprising a nucleic acid molecule of the invention and kits comprising the nucleic acid molecule, vector or host cell. In particular the invention provides a stable cell line that expresses a rapid turnover reporter protein with an enhanced signal relative to a corresponding stable cell line that expresses a corresponding nondestabilized reporter protein.

A rapid turnover (destabilized) reporter protein such as luciferase can be used in applications where currently available reporter proteins with half-lives of expression at least several hours cannot, such as, as a genetic reporter for analyzing transcriptional regulation and/or cis-acting regulatory elements, as a tool for identifying and analyzing degradation domains of short-lived proteins or to accelerate screening of efficacious compounds. Cells containing a regulatable vector of the invention respond more quickly to induction or repression and show enhanced activation relative to cells containing a vector expressing a corresponding unmodified, e.g., wild-type, reporter protein. Moreover, the presence of a vector of the invention in host cells used for screening is advantageous in that those cells are less sensitive to impaired cell growth or to modification or loss of the vector, and allows for more precise quantification of signal.

Hence, the present invention also provides an expression cassette comprising the nucleic acid sequence of the invention and a vector capable of expressing the nucleic acid sequence in a host cell. Preferably, the expression cassette comprises a promoter, e.g., a constitutive or regulatable promoter, operably linked to the nucleic acid sequence. In one embodiment of the vector, the expression cassette contains an inducible promoter. Also provided is a host cell, e.g., an eukaryotic cell such as a plant or vertebrate cell, e.g., a mammalian cell, including but not limited to a human, non-human primate, canine, feline, bovine, equine, ovine or rodent (e.g., rabbit, rat, ferret or mouse) cell, and a kit which comprises the nucleic acid molecule, expression cassette or vector of the invention.

In another aspect of the invention, there is provided a method of labeling cells with a fusion polypeptide of the invention. In this method, a cell is contacted with a vector comprising a promoter, e.g., a regulatable promoter, and a nucleic acid sequence encoding a fusion polypeptide comprising a protein of interest such as a reporter protein with a substantially decreased half-life of expression relative to a corresponding wild-type protein. In one embodiment, a transfected cell is cultured under conditions in which the promoter induces transient expression of the fusion polypeptide, which provides a transient reporter label.

### Brief Description of the Figures

Figure 1A. Lysates of CHO cells containing plasmid pwtLuc1 (lane 2), pUbiq(Y)Luc 19 (lane 3) or pLuc-PEST10 (lane 4), or a CHO lysate without plasmid (lane 5), were separated on 4-20% SDS-PAGE, transferred on to an ImmobilonP membrane and luciferase species were detected with rabbit anti-firefly luciferase and anti-rabbit antibodies conjugated with alkaline phosphatase. Lane 1 corresponds to See Blue Pre-Stained Standard from Invitrogen.
Figure 1B. Proteins translated with wheat germ extracts containing mRNA obtained using plasmid pwtLuc1 (lane 1) or pETUbiqLuc (lane 2), or without external mRNA (lane 3), were separated on 4-20% SDS-PAGE and the proteins visualized by autoradiography.
Figure 1C. TNT^{®} T7 Coupled Reticulocyte Lysates containing plasmid pETwtLuc1 (lane 1), pT7Ubiq(Y)Luc19.2 (lane 2), pT7 Ubiq(E)Luc19.1 (lane 3) or pT7Luc-PEST10 (lane 4), were separated on 4-20% SDS-PAGE and the proteins visualized by autoradiography.
Figure 2. Plasmids encoding wild-type firefly luciferase and fusion proteins comprising firefly luciferase were expressed in TNT^{®} T7 Coupled Reticulocyte Lysate System. Specific activity was determined as the ratio between total luciferase activity accumulated in each mixture and the amount of [³H]-Leucine incorporated in each protein.
Figure 3. Cells transiently transfected with plasmids encoding wild-type firefly luciferase (pwtLuc1), a ubiquitin-luciferase fusion protein (pUbiq(Y)Luc19 and pT7Ubiq(Y)Luc19.2), or a fusion protein comprising firefly luciferase and a mutant form of C-ODC (mODC) (pLuc-PEST10) were treated with cycloheximide (100 µg/ml) for different periods of time. Upon completion of incubation, and to define stability, cells were lysed, and accumulated luciferase activity was determined using a MLX Microtiter Plate Luminometer.
Figure 4. CHO (A), COS-7 (B), and HeLa (C) cells, transfected with ubiquitin-luciferase fusion protein encoding plasmids, were treated with cycloheximide for different periods of time. Cellular luminescence was measured to determine the stability of the corresponding proteins. Control cells that had not been treated with cycloheximide were used to determine background luciferase activity.
Figure 5. The partial amino acid sequence of ubiquitin-luciferase fusion proteins was evaluated in establishing the relative importance of the N-terminal residue in determining protein half-life. Shadowed/boxed areas mark ubiquitin and luciferase sequences. Thick lines mark the position of deletions.
Figure 6. CHO (A) and COS-7 (B) cells were transiently transfected with plasmids encoding either wild-type firefly luciferase (pwtLuc1) or ubiquitin-luciferase fusion proteins with different N-terminal luciferase amino acid residues. Twenty-four hours after transfection, the cells were treated with cycloheximide (100 µg/ml) for different periods of time and, upon completion of incubation, luminescence of accumulated luciferase was measured.

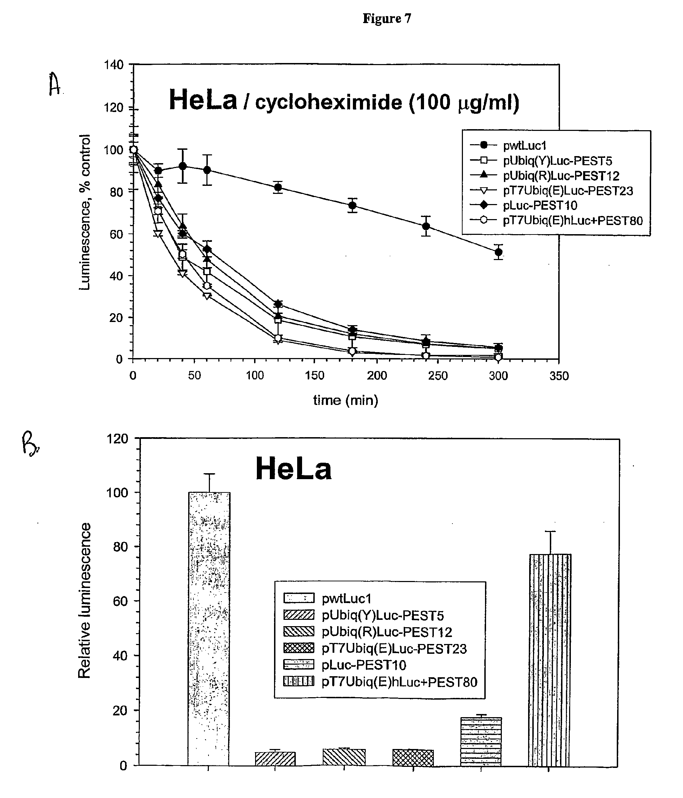

Figure 7. HeLa cells were transfected with plasmids encoding wild-type luciferase (pwtLuc1), a fusion protein comprising luciferase and mODC (pLuc-PEST10), or a fusion protein comprising ubiquitin, firefly luciferase, and mODC (pUbiq(Y)Luc-PEST5, pUbiq(R)Luc-PEST12, pT7Ubiq(E)Luc-PEST23 and pT7Ubiq(E)hLuc+PEST80). Twenty-four hours after transfection, the cells were treated with cycloheximide (100 µg/ml) for different periods of time. Cellular luminescence was measured to determine the stability of the corresponding luciferase (A). Control cells that had not been treated with cycloheximide were used to compare the luciferase activity of different constructs (B).
Figure 8. CHO cells were transiently transfected with various plasmids. Twenty-four hours post-transfection, the cells were treated with cycloheximide (100 µg/ml) for different periods of time. After incubation, luminescence due to accumulated luciferase was measured. Control cells that had not been treated with cycloheximide were used to determine background luciferase activity.
Figure 9. Comparison of luciferase fusion protein properties in a *tet* inducible system after doxycycline (2 µg/ml) (A) or cycloheximide (100 µg/ml) (B) treatment. Luminescence data from control cells that had not been treated with either doxycycline or cycloheximide are depicted in panel C.
Figures 10 A-B. Comparison of luciferase fusion protein properties *Renilla* luciferase (A) and firefly luciferase (B) in a heat shock inducible system.
Figure 11. Schematic of selected vectors.
Figures 12A-B. Induction of luminescence in D293 cells transiently transfected with *Renilla* luciferase vectors with multiple CREs, forskolin (10 µM) and isoproterenol (0.25 µM).
Figure 13A-B. Luminescence profiles of hCG-D293 cells transiently transfected with vectors encoding stable and destabilized versions of firefly luciferase. Cells were treated with isoproterenol (1 µM) and Ro-20-1724 (100 µM) or isoproterenol (1 µM) and Ro-20-1724 (100 µM) followed by treatment with human chorionic gonadotropin (hCG) (10 ng/ml) and Ro-20-1724 (100 µM). Arrows indicate time points when chemicals were added to the cell cultures.
Figure 14. Luminescence versus fold induction in D293 cells stably transfected with destabilized vectors. Cells were treated with forskolin (10 µM) for 7 hours or incubated in forskolin-free media. All vectors were under the control of a cAMP regulated promoter.
Figure 15. Fold induction by isoproterenol and prostaglandin E1 (PGE1) in 293 cells transfected with codon optimized firefly or *Renilla* luciferase in conjunction with destabilization sequences in a CRE system. (A)-(B): PGE1 added 24 hours after Iso/Ro; (C)-(D): PGE1 added 6 hours after Iso/Ro.
Figure 16. Fold induction by isoproterenol in 293 cells transfected with either red (CBR) (B) or green (CBG) (A) click beetle sequences in conjunction with destabilization sequences in a CRE system

### Detailed Description of the Invention

### Definitions

The term "nucleic acid molecule", "gene" or "nucleic acid sequence" as used herein, refers to nucleic acid, DNA or RNA, that comprises coding sequences necessary for the production of a polypeptide or protein precursor. The polypeptide can be encoded by a full-length coding sequence or by any portion of the coding sequence, as long as the desired protein activity is retained.

A "nucleic acid", as used herein, is a covalently linked sequence of nucleotides in which the 3' position of the pentose of one nucleotide is joined by a phosphodiester group to the 5' position of the pentose of the next, and in which the nucleotide residues (bases) are linked in specific sequence, i.e., a linear order of nucleotides. A "polynucleotide", as used herein, is a nucleic acid containing a sequence that is greater than about 100 nucleotides in length. An "oligonucleotide" or "primer", as used herein, is a short polynucleotide or a portion of a polynucleotide. An oligonucleotide typically contains a sequence of about two to about one hundred bases. The word "oligo" is sometimes used in place of the word "oligonucleotide".

Nucleic acid molecules are said to have a "5'-terminus" (5' end) and a "3'-terminus" (3' end) because nucleic acid phosphodiester linkages occur to the 5' carbon and 3' carbon of the pentose ring of the substituent mononucleotides. The end of a polynucleotide at which a new linkage would be to a 5' carbon is its 5' terminal nucleotide. The end of a polynucleotide at which a new linkage would be to a 3' carbon is its 3' terminal nucleotide. A terminal nucleotide, as used herein, is the nucleotide at the end position of the 3'- or 5'-terminus.

DNA molecules are said to have "5' ends" and "3' ends" because mononucleotides are reacted to make oligonucleotides in a manner such that the 5' phosphate of one mononucleotide pentose ring is attached to the 3' oxygen of its neighbor in one direction via a phosphodiester linkage. Therefore, an end of an oligonucleotides referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring.

As used herein, a nucleic acid sequence, even if internal to a larger oligonucleotide or polynucleotide, also may be said to have 5' and 3' ends. In either a linear or circular DNA molecule, discrete elements are referred to as being "upstream" or 5' of the "downstream" or 3' elements. This terminology reflects the fact that transcription proceeds in a 5' to 3' fashion along the DNA strand. Typically, promoter and enhancer elements that direct transcription of a linked gene (e.g., open reading frame or coding region) are generally located 5' or upstream of the coding region. However, enhancer elements can exert their effect even when located 3' of the promoter element and the coding region. Transcription termination and polyadenylation signals are located 3' or downstream of the coding region.

The term "codon" as used herein, is a basic genetic coding unit, consisting of a sequence of three nucleotides that specify a particular amino acid to be incorporation into a polypeptide chain, or a start or stop signal. The term "coding region" when used in reference to structural genes refers to the nucleotide sequences that encode the amino acids found in the nascent polypeptide as a result of translation of a mRNA molecule. Typically, the coding region is bounded on the 5' side by the nucleotide triplet "ATG" which encodes the initiator methionine and on the 3' side by a stop codon (e.g., TAA, TAG, TGA). In some cases the coding region is also known to initiate by a nucleotide triplet "TTG".

By "protein" and "polypeptide" is meant any chain of amino acids, regardless of length or post-translational modification (e.g., glycosylation or phosphorylation). The nucleic acid molecules of the invention may also encode a variant of a naturally-occurring protein or polypeptide fragment thereof. Preferably, such a protein polypeptide has an amino acid sequence that is at least 85%, preferably 90%, and most preferably 95% or 99% identical to the amino acid sequence of the naturally-occurring (native or wild-type) protein from which it is derived.

Polypeptide molecules are said to have an "amino terminus" (N-terminus) and a "carboxy terminus" (C-terminus) because peptide linkages occur between the backbone amino group of a first amino acid residue and the backbone carboxyl group of a second amino acid residue. The terms "N-terminal" and "C-terminal" in reference to polypeptide sequences refer to regions of polypeptides including portions of the N-terminal and C-terminal regions of the polypeptide, respectively. A sequence that includes a portion of the N-terminal region of a polypeptide includes amino acids predominantly from the N-terminal half of the polypeptide chain, but is not limited to such sequences. For example, an N-terminal sequence may include an interior portion of the polypeptide sequence including bases from both the N-terminal and C-terminal halves of the polypeptide. The same applies to C-terminal regions. N-terminal and C-terminal regions may, but need not, include the amino acid defining the ultimate N-terminus and C-terminus of the polypeptide, respectively.

The term "wild-type" as used herein, refers to a gene or gene product that has the characteristics of that gene or gene product isolated from a naturally occurring source. A wild-type gene is that which is most frequently observed in a population and is thus arbitrarily designated the "wild-type" form of the gene. In contrast, the term "mutant" refers to a gene or gene product that displays modifications in sequence and/or functional properties (i.e., altered characteristics) when compared to the wild-type gene or gene product. It is noted that naturally-occurring mutants can be isolated; these are identified by the fact that they have altered characteristics when compared to the wild-type gene or gene product.

The term "recombinant protein" or "recombinant polypeptide" as used herein refers to a protein molecule expressed from a recombinant DNA molecule. In contrast, the term "native protein" is used herein to indicate a protein isolated from a naturally occurring (i.e., a nonrecombinant) source. Molecular biological techniques may be used to produce a recombinant form of a protein with identical properties as compared to the native form of the protein.

The term "fusion polypeptide" refers to a chimeric protein containing a protein of interest (e.g., luciferase) joined to a heterologous sequence (e.g., a non-luciferase amino acid or protein).

The terms "cell," "cell line," "host cell," as used herein, are used interchangeably, and all such designations include progeny or potential progeny of these designations. By "transformed cell" is meant a cell into which (or into an ancestor of which) has been introduced a nucleic acid molecule of the invention, e.g., via transient transfection. Optionally, a nucleic acid molecule synthetic gene of the invention may be introduced into a suitable cell line so as to create a stably-transfected cell line capable of producing the protein or polypeptide encoded by the synthetic gene. Vectors, cells, and methods for constructing such cell lines are well known in the art. The words "transformants" or "transformed cells" include the primary transformed cells derived from the originally transformed cell without regard to the number of transfers. All progeny may not be precisely identical in DNA content, due to deliberate or inadvertent mutations. Nonetheless, mutant progeny that have the same functionality as screened for in the originally transformed cell are included in the definition of transformants.

Nucleic acids are known to contain different types of mutations. A "point" mutation refers to an alteration in the sequence of a nucleotide at a single base position from the wild type sequence. Mutations may also refer to insertion or deletion of one or more bases, so that the nucleic acid sequence differs from the wild-type sequence.

The term "homology" refers to a degree of complementarity. There may be partial homology or complete homology (i.e., identity). Homology is often measured using sequence analysis software (e.g., Sequence Analysis Software Package of the Genetics Computer Group. University of Wisconsin Biotechnology Center. 1710 University Avenue. Madison, WI 53705). Such software matches similar sequences by assigning degrees of homology to various substitutions, deletions, insertions, and other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine.

The term "isolated" when used in relation to a nucleic acid, as in "isolated oligonucleotide" or "isolated polynucleotide" refers to a nucleic acid sequence that is identified and separated from at least one contaminant with which it is ordinarily associated in its source. Thus, an isolated nucleic acid is present in a form or setting that is different from that in which it is found in nature. In contrast, non-isolated nucleic acids (e.g., DNA and RNA) are found in the state they exist in nature. For example, a given DNA sequence (e.g., a gene) is found on the host cell chromosome in proximity to neighboring genes; RNA sequences (e.g., a specific mRNA sequence encoding a specific protein), are found in the cell as a mixture with numerous other mRNAs that encode a multitude of proteins. However, isolated nucleic acid includes, by way of example, such nucleic acid in cells ordinarily expressing that nucleic acid where the nucleic acid is in a chromosomal location different from that of natural cells, or is otherwise flanked by a different nucleic acid sequence than that found in nature. The isolated nucleic acid or oligonucleotide may be present in single-stranded or double-stranded form. When an isolated nucleic acid or oligonucleotide is to be utilized to express a protein, the oligonucleotide contains at a minimum, the sense or coding strand (i.e., the oligonucleotide may be single-stranded), but may contain both the sense and anti-sense strands (i.e., the oligonucleotide may be double-stranded).

The term "isolated" when used in relation to a polypeptide, as in "isolated protein" or "isolated polypeptide" refers to a polypeptide that is identified and separated from at least one contaminant with which it is ordinarily associated in its source. Thus, an isolated polypeptide is present in a form or setting that is different from that in which it is found in nature. In contrast, non-isolated polypeptides (e.g., proteins and enzymes) are found in the state they exist in nature.

The term "purified" or "to purify" means the result of any process that removes some of a contaminant from the component of interest, such as a protein or nucleic acid. The percent of a purified component is thereby increased in the sample.

The term "operably linked" as used herein refer to the linkage of nucleic acid sequences in such a manner that a nucleic acid molecule capable of directing the transcription of a given gene and/or the synthesis of a desired protein molecule is produced. The term also refers to the linkage of sequences encoding amino acids in such a manner that a functional (e.g., enzymatically active, capable of binding to a binding partner, capable of inhibiting, etc.) protein or polypeptide is produced.

The term "recombinant DNA molecule" means a hybrid DNA sequence comprising at least two nucleotide sequences not normally found together in nature. The term "vector" is used in reference to nucleic acid molecules into which fragments of DNA may be inserted or cloned and can be used to transfer DNA segment(s) into a cell and capable of replication in a cell. Vectors may be derived from plasmids, bacteriophages, viruses, cosmids, and the like.

The terms "recombinant vector" and "expression vector" as used herein refer to DNA or RNA sequences containing a desired coding sequence and appropriate DNA or RNA sequences necessary for the expression of the operably linked coding sequence in a particular host organism. Prokaryotic expression vectors include a promoter, a ribosome binding site, an origin of replication for autonomous replication in a host cell and possibly other sequences, e.g. an optional operator sequence, optional restriction enzyme sites. A promoter is defined as a DNA sequence that directs RNA polymerase to bind to DNA and to initiate RNA synthesis. Eukaryotic expression vectors include a promoter, optionally a polyadenlyation signal and optionally an enhancer sequence.

A polynucleotide having a nucleotide sequence encoding a protein or polypeptide means a nucleic acid sequence comprising the coding region of a gene, or in other words the nucleic acid sequence encodes a gene product. The coding region may be present in either a cDNA, genomic DNA or RNA form. When present in a DNA form, the oligonucleotide may be single-stranded (i.e., the sense strand) or double-stranded. Suitable control elements such as enhancers/promoters, splice junctions, polyadenylation signals, etc. may be placed in close proximity to the coding region of the gene if needed to permit proper initiation of transcription and/or correct processing of the primary RNA transcript. Alternatively, the coding region utilized in the expression vectors of the present invention may contain endogenous enhancers/promoters, splice junctions, intervening sequences, polyadenylation signals, etc. In further embodiments, the coding region may contain a combination of both endogenous and exogenous control elements.

The term "transcription regulatory element" or "transcription regulatory sequence" refers to a genetic element or sequence that controls some aspect of the expression of nucleic acid sequence(s). For example, a promoter is a regulatory element that facilitates the initiation of transcription of an operably linked coding region. Other regulatory elements include, but are not limited to, transcription factor binding sites, splicing signals, polyadenylation signals, termination signals and enhancer elements.

Transcriptional control signals in eukaryotes comprise "promoter" and "enhancer" elements. Promoters and enhancers consist of short arrays of DNA sequences that interact specifically with cellular proteins involved in transcription. Promoter and enhancer elements have been isolated from a variety of eukaryotic sources including genes in yeast, insect and mammalian cells. Promoter and enhancer elements have also been isolated from viruses and analogous control elements, such as promoters, are also found in prokaryotes. The selection of a particular promoter and enhancer depends on the cell type used to express the protein of interest. Some eukaryotic promoters and enhancers have a broad host range while others are functional in a limited subset of cell types. For example, the SV40 early gene enhancer is very active in a wide variety of cell types from many mammalian species and has been widely used for the expression of proteins in mammalian cells. Two other examples of promoter/enhancer elements active in a broad range of mammalian cell types are those from the human elongation factor 1 gene (Uetsuki et al., 1989; Kim et al., 1990; and Mizushima and Nagata, 1990) and the long terminal repeats of the Rous sarcoma virus (Gorman et al., 1982); and the human cytomegalovirus (Boshart et al., 1985).

The term "promoter/enhancer" denotes a segment of DNA containing sequences capable of providing both promoter and enhancer functions (i.e., the functions provided by a promoter element and an enhancer element as described above). For example, the long terminal repeats of retroviruses contain both promoter and enhancer functions. The enhancer/promoter may be "endogenous" or "exogenous" or "heterologous." An "endogenous" enhancer/promoter is one that is naturally linked with a given gene in the genome. An "exogenous" or "heterologous" enhancer/promoter is one that is placed in juxtaposition to a gene by means of genetic manipulation (i.e., molecular biological techniques) such that transcription of the gene is directed by the linked enhancer/promoter.

The presence of "splicing signals" on an expression vector often results in higher levels of expression of the recombinant transcript in eukaryotic host cells. Splicing signals mediate the removal of introns from the primary RNA transcript and consist of a splice donor and acceptor site (Sambrook et al., 1989). A commonly used splice donor and acceptor site is the splice junction from the 16S RNA of SV40.

Efficient expression of recombinant DNA sequences in eukaryotic cells requires expression of signals directing the efficient termination and polyadenylation of the resulting transcript. Transcription termination signals are generally found downstream of the polyadenylation signal and are a few hundred nucleotides in length. The term "poly(A) site" or "poly(A) sequence" as used herein denotes a DNA sequence which directs both the termination and polyadenylation of the nascent RNA transcript. Efficient polyadenylation of the recombinant transcript is desirable, as transcripts lacking a poly(A) tail are unstable and are rapidly degraded. The poly(A) signal utilized in an expression vector may be "heterologous" or "endogenous." An endogenous poly(A) signal is one that is found naturally at the 3' end of the coding region of a given gene in the genome. A heterologous poly(A) signal is one which has been isolated from one gene and positioned 3' to another gene. A commonly used heterologous poly(A) signal is the SV40 poly(A) signal. The SV40 poly(A) signal is contained on a 237 bp *Bam*HI/*Bcl* I restriction fragment and directs both termination and polyadenylation (Sambrook et al., 1989).

Eukaryotic expression vectors may also contain "viral replicons "or "viral origins of replication." Viral replicons are viral DNA sequences which allow for the extrachromosomal replication of a vector in a host cell expressing the appropriate replication factors. Vectors containing either the SV40 or polyoma virus origin of replication replicate to high copy number (up to 10⁴ copies/cell) in cells that express the appropriate viral T antigen. In contrast, vectors containing the replicons from bovine papillomavirus or Epstein-Barr virus replicate extrachromosomally at low copy number (about 100 copies/cell).

The term "*in vitro*" refers to an artificial environment and to processes or reactions that occur within an artificial environment. *In vitro* environments include, but are not limited to, test tubes and cell lysates. The term "*in situ*" refers to cell culture. The term "*in vivo*" refers to the natural environment (e.g., an animal or a cell) and to processes or reactions that occur within a natural environment.

The term "expression system" refers to any assay or system for determining (e.g., detecting) the expression of a gene of interest. Those skilled in the field of molecular biology will understand that any of a wide variety of expression systems may be used. A wide range of suitable mammalian cells are available from a wide range of sources (e.g., the American Type Culture Collection, Rockland, MD). The method of transformation or transfection and the choice of expression vehicle will depend on the host system selected. Transformation and transfection methods are described, e.g., in Ausubel et al., 1992. Expression systems include *in vitro* gene expression assays where a gene of interest (e.g., a reporter gene) is linked to a regulatory sequence and the expression of the gene is monitored following treatment with an agent that inhibits or induces expression of the gene. Detection of gene expression can be through any suitable means including, but not limited to, detection of expressed mRNA or protein (e.g., a detectable product of a reporter gene) or through a detectable change in the phenotype of a cell expressing the gene of interest. Expression systems may also comprise assays where a cleavage event or other nucleic acid or cellular change is detected.

All amino acid residues identified herein are in the natural L-configuration. In keeping with standard polypeptide nomenclature, abbreviations for amino acid residues are as shown in the following Table of Correspondence.

| TABLE OF CORRESPONDENCE | | |
|---|---|---|
| 1-Letter | 3-Letter | AMINO ACID |
| Y | Tyr | L-tyrosine |
| G | Gly | L-glycine |
| F | Phe | L-phenylalanine |
| M | Met | L-methionine |
| A | Ala | L-alanine |
| S | Ser | L-serine |
| I | Ile | L-isoleucine |
| L | Leu | L-leucine |
| T | Thr | L-threonine |
| V | Val | L-valine |
| P | Pro | L-proline |
| K | Lys | L-lysine |
| H | His | L-histidine |
| Q | Gln | L-glutamine |
| E | Glu | L-glutamic acid |
| W | Trp | L-tryptophan |
| R | Arg | L-arginine |
| D | Asp | L-aspartic acid |
| N | Asn | L-asparagine |
| C | Cys | L-cysteine |

The invention provides compositions comprising nucleic acid molecules comprising nucleic acid sequences encoding fusion polypeptides, as well as methods for using those molecules to yield fusion polypeptides, comprising a protein of interest with a reduced, e.g., a substantially reduced, half-life of expression relative to a corresponding parental (e.g., wild-type) polypeptide. The invention also provides a fusion polypeptide encoded by such a nucleic acid molecule. The invention may be employed to reduce the half-life of expression of any protein of interest, e.g., the half-life of a reporter protein. In particular, the invention provides an isolated nucleic acid molecule comprising a nucleic acid sequence encoding a fusion polypeptide comprising a protein of interest and a combination of one or more heterologous protein destabilization sequences and one or more heterologous mRNA destabilization sequences, which results in a substantial reduction in the half-life of expression of the encoded fusion polypeptide. Heterologous protein destabilization sequences may be at the N-terminus or the C-terminus, or at the N-terminus and the C-terminus of the protein of interest and will include at least one CL sequence as noted above or the SL17 sequence. A heterologous protein destabilization sequence may include 2 or more, e.g., 3 to 200, or any integer in between 3 and 200, amino acid residues, although not all of the residues in longer sequences, e.g., those greater than 5 residues in length, may be capable of destabilizing a linked amino acid sequence. Multiple copies of any one protein destabilization sequence may also be employed with a protein of interest. In one embodiment, different protein destabilization sequences are employed, e.g., a combination of a CL sequence and a PEST sequence. Heterologous mRNA destabilization sequences are preferably to the coding region for a fusion polypeptide of the invention. A heterologous mRNA destabilization sequence may include 5 or more, e.g., 6 to 100, or any integer in between 6 and 100, nucleotides, although not all of the resides in longer sequences, e.g., those greater than 10 nucleotides, may be capable of destabilizing a linked nucleotide sequence. Multiple copies of any one mRNA destabilization sequence may be employed. In one embodiment, different mRNA destabilization sequences are employed.

Optionally, a second polypeptide may be fused to the N-terminus of a fusion polypeptide comprising a protein of interest and a heterologous protein destabilization sequence, e.g., a destabilization sequence which is present at the N-terminus of the protein of interest. In one embodiment, the second polypeptide is a polypeptide which is cleaved after the C-terminal residue by an enzyme present in a cell or cell extract, yielding a fusion polypeptide comprising a protein of interest with a heterologous protein destabilization sequence, e.g., at its N-termmus. In one embodiment, the second polypeptide is ubiquitin.

In one embodiment, the N-terminal heterologous protein destabilization sequence is a cyclin destruction box or N-degron. In one embodiment, the C-terminal heterologous protein destabilization sequence is a CL peptide, CL1, CL2, CL6, CL9, CL10, CL11, CL12, CL15, CL16, or SL17 (see Table 1 of Gilon et al., 1998, which is specifically incorporated by reference herein), a C-ODC or a mutant C-ODC, e.g., a sequence such as
HGFXXXMXXQXXGTLPMSCAQESGXXRHPAACASARINV
(corresponding to residues 423-461 of mODC), wherein one or more of the residues at positions marked with "X" are not the naturally occurring residue and wherein the substitution results in a decrease in the stability of a protein having that substituted sequence relative to a protein having the nonsubstituted sequence. For instance, a fusion polypeptide comprising a mutant C-ODC which has a non-conservative substitution at residues corresponding to residues 426,427, 428, 430, 431, 433, 434, or 448 of ODC, e.g., from proline, aspartic acid or glutamic acid to alanine, can result in a fusion polypeptide with decreased stability, e.g., relative to a fusion polypeptide with a non-substituted C-ODC.

The invention may be employed with any nucleic acid sequence, e.g., a native sequence such as a cDNA or one which has been manipulated *in vitro,* e.g., but is particularly useful for reporter genes as well as other genes associated with the expression of reporter genes, such as selectable markers. Preferred genes include, but are not limited to, those encoding lactamase (β-gal), neomycin resistance (Neo), CAT, GUS, galactopyranoside, GFP, xylosidase, thymidine kinase, arabinosidase and the like. As used herein, a "marker gene" or "reporter gene" is a gene that imparts a distinct phenotype to cells expressing the gene and thus permits cells having the gene to be distinguished from cells that do not have the gene. Such genes may encode either a selectable or screenable marker, depending on whether the marker confers a trait which one can 'select' for by chemical means, i.e., through the use of a selective agent (e.g., a herbicide, antibiotic, or the like), or whether it is simply a "reporter" trait that one can identify through observation or testing, i.e., by 'screening'. Elements of the present disclosure are exemplified in detail through the use of particular marker genes. Of course, many examples of suitable marker genes or reporter genes are known to the art and can be employed in the practice of the invention. Therefore, it will be understood that the following discussion is exemplary rather than exhaustive. In light of the techniques disclosed herein and the general recombinant techniques which are known in the art, the present invention renders possible the alteration of any gene.

Exemplary genes include, but are not limited to, a *neo* gene, a β-gal gene, a *gus* gene, a *cat* gene, a *gpt* gene, a *hyg* gene, a *his-D* gene, a *ble* gene, a *mprt* gene, a *bar* gene, a nitrilase gene, a mutant acetolactate synthase gene (ALS) or acetoacid synthase gene (AAS), a methotrexate-resistant *dhfr* gene, a dalapon dehalogenase gene, a mutated anthranilate synthase gene that confers resistance to 5-methyl tryptophan (WO 97/26366), an R-locus gene, a β-lactamase gene, a *xyl*E gene, an α-amylase gene, a tyrosinase gene, a luciferase (*luc*) gene, (e.g., a *Renilla reniformis* luciferase gene, a firefly luciferase gene, or a click beetle luciferase (*Pyrophorus plagiophthalamus*) gene, an aequorin gene, or a green fluorescent protein gene. Included within the terms selectable or screenable marker genes are also genes which encode a "secretable marker" whose secretion can be detected as a means of identifying or selecting for transformed cells. Examples include markers which encode a secretable antigen that can be identified by antibody interaction, or even secretable enzymes which can be detected by their catalytic activity. Secretable proteins fall into a number of classes, including small, diffusible proteins detectable, e.g., by ELISA, and proteins that are inserted or trapped in the cell membrane.

In one embodiment, the nucleic acid sequence encoding the fusion polypeptide is optimized for expression in a particular cell. For example, the nucleic acid sequence is optimized by replacing codons in the wild-type sequence with codons which are preferentially employed in a particular (selected) cell. Preferred codons have a relatively high codon usage frequency in a selected cell, and preferably their introduction results in the introduction of relatively few transcription factor binding sites, and relatively few other undesirable structural attributes. Thus, the optimized nucleic acid product has an improved level of expression due to improved codon usage frequency, and a reduced risk of inappropriate transcriptional behavior due to a reduced number of undesirable transcription regulatory sequences.

An isolated nucleic acid molecule of the invention which is optimized may have a codon composition that differs from that of the corresponding wild-type nucleic acid sequence at more than 30%, 35%, 40% or more than 45%, e.g., 50%, 55%, 60% or more of the codons. Preferred codons for use in the invention are those which are employed more frequently than at least one other codon for the same amino acid in a particular organism and, more preferably, are also not low-usage codons in that organism and are not low-usage codons in the organism used to clone or screen for the expression of the nucleic acid molecule: Moreover, preferred codons for certain amino acids (i.e., those amino acids that have three or more codons,), may include two or more codons that are employed more frequently than the other (non-preferred) codon(s). The presence of codons in the nucleic acid molecule that are employed more frequently in one organism than in another organism results in a nucleic acid molecule which, when introduced into the cells of the organism that employs those codons more frequently, is expressed in those cells at a level that is greater than the expression of the wild-type or parent nucleic acid sequence in those cells.

In one embodiment of the invention, the codons that are different are those employed more frequently in a mammal, while in another embodiment the codons that are different are those employed more frequently in a plant. A particular type of mammal, e.g., human, may have a different set of preferred codons than another type of mammal. Likewise, a particular type of plant may have a different set of preferred codons than another type of plant. In one embodiment of the invention, the majority of the codons which differ are ones that are preferred codons in a desired host cell. Preferred codons for mammals (e.g., humans) and plants are known to the art (e.g., Wada et al., 1990). For example, preferred human codons include, but are not limited to, CGC (Arg), CTG (Leu), TCT (Ser), AGC (Ser), ACC (Thr), CCA (Pro), CCT (Pro), GCC (Ala), GGC (Gly), GTG (Val), ATC (Ile), ATT (Ile), AAG (Lys), AAC (Asn), CAG (Gln), CAC (His), GAG (Glu), GAC (Asp), TAC (Tyr), TGC (Cys) and TTC (Phe) (Wada et al., 1990). Thus, in one embodiment, synthetic nucleic acid molecules of the invention have a codon composition which differs from a wild type nucleic acid sequence by having an increased number of the preferred human codons, e.g. CGC, CTG, TCT, AGC, ACC, CCA, CCT, GCC, GGC, GTG, ATC, ATT, AAG, AAC, CAG, CAC, GAG, GAC, TAC, TGC, TTC, or any combination thereof. For example, the nucleic acid molecule of the invention may have an increased number of CTG or TTG leucine-encoding codons, GTG or GTC valine-encoding codons, GGC or GGT glycine-encoding codons, ATC or ATT isoleucine-encoding codons, CCA or CCT proline-encoding codons, CGC or CGT arginine-encoding codons, AGC or TCT serine-encoding codons, ACC or ACT threonine-encoding codon, GCC or GCT alanine-encoding codons, or any combination thereof, relative to the wild-type nucleic acid sequence. Similarly, nucleic acid molecules having an increased number of codons that are employed more frequently in plants, have a codon composition which differs from a wild-type or parent nucleic acid sequence by having an increased number of the plant codons including, but not limited to, CGC (Arg), CTT (Leu), TCT (Ser), TCC (Ser), ACC (Thr), CCA (Pro), CCT (Pro), GCT (Ser), GGA (Gly), GTG (Val), ATC (Ile), ATT (Ile), AAG (Lys), AAC (Asn), CAA (Gln), CAC (His), GAG (Glu), GAC (Asp), TAC (Tyr), TGC (Cys), TTC (Phe), or any combination thereof (Murray et al., 1989). Preferred codons may differ for different types of plants (Wada et al., 1990).

A nucleic acid molecule comprising a nucleic acid sequence encoding a fusion polypeptide of the invention is optionally operably linked to transcription regulatory sequences, e.g., enhancers, promoters and transcription termination sequences to form an expression cassette. The nucleic acid molecule is introduced to a vector, e.g., a plasmid or viral vector, which optionally a selectable marker gene, and the vector introduced to a cell of interest, for example, a plant (dicot or monocot), fungus, yeast or mammalian cell. Preferred host cells are mammalian cells such as CHO, COS, 293, Hela, CV-1, and NIH3T3 cells.

The expression of the encoded fusion polypeptide may be controlled by any promoter, including but not limited to regulatable promoters, e.g., an inducible or repressible promoter such as the *tet* promoter, the hsp70 promoter and a synthetic promoter regulated by CRE. For example, in the tet-regulated system, the luminescent signal for a wild-type luciferase dissipated by 16-17 hours while the signal for a fusion polypeptide comprising a heterologous destabilization sequence dissipated to a similar level by 4 hours.

In one embodiment of the invention, the isolated nucleic acid molecule comprises a nucleic acid sequence encoding a fusion polypeptide comprising a reporter protein and at least two destabilization sequences, wherein the nucleic acid sequence is a synthetic sequence containing codons preferentially found in a particular organism, e.g., in plants or humans, and more preferably in highly expressed proteins, for instance, highly expressed human proteins.

In one preferred embodiment, the invention provides an isolated nucleic acid molecule comprising a nucleic acid sequence encoding a fusion polypeptide comprising a luminescent protein, e.g., a luciferase, and a combination of heterologous protein and mRNA destabilization sequences. Preferably, at least the sequence encoding the luminescent protein is optimized for expression in human cells.

The invention will be further described by the following example in so far as it refers to CL1.

### Example 1

### Materials and Methods

### Bacterial Cells and Plasmids

*Escherichia coli* JM109 cells were used to propagate plasmids. Bacterial cultures were grown routinely in LB broth at 37°C with the addition of 100 µg/ml ampicillin or 30 µg/ml kanamycin when required. Extraction and purification of plasmid DNA were performed using Plasmid Maxi Kit (Qiagen).

pGEM^{®}T Easy Vector (Promega) was used to clone PCR products. Plasmids pGL3-Basic Vector and pSP-luc+NF Fusion Vector were used as the source for cDNA encoding firefly luciferase (Promega).

### DNA modifying enzymes

Restriction enzymes *Age*I, *Apa*I, *Bam*HI, *Bgl*II, *Bst*EII, *Bst*98I, *Eco*RI, *Eco*RV, *Nco*I, *Not*I, *Sca*I, *Xba*I and *Xmn*I as well as T4 DNA polymerase and S I nuclease were obtained from Promega. Rapid DNA Ligation Kit and Expand™ High Fidelity PCR System were supplied by Boehringer Mannheim.

### Oligonucleotides

Oligonucleotides used for polymerase chain reactions as well as oligonucleotides used for cloning and sequencing are listed in Table 1. All the oligonucleotides were synthesized at Promega.

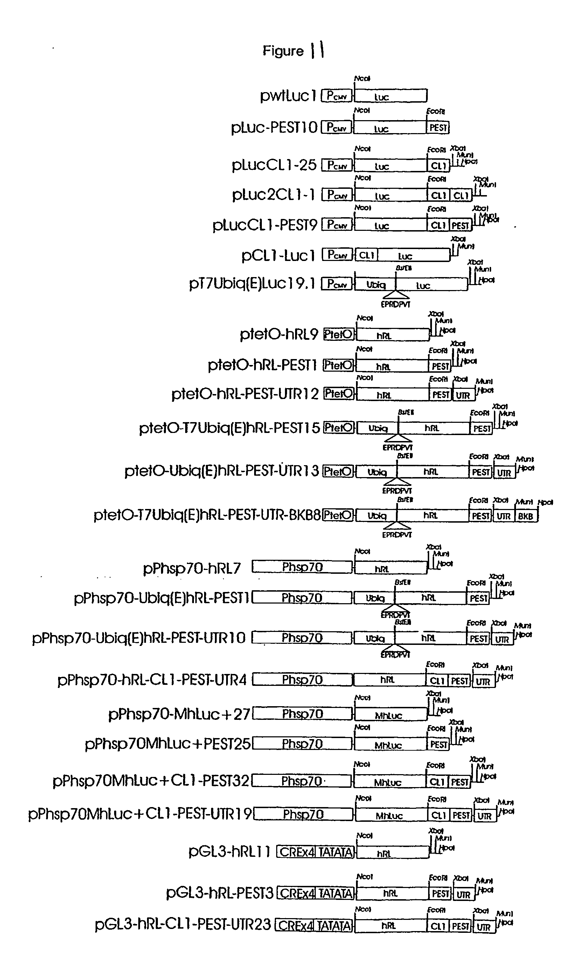

A representative number of these DNA constructs are depicted schematically in Figure 11.
pGEM-Luc5 was constructed by cloning into pGEM^{®}-T Easy Vector a fragment that encodes firefly luciferase, which was amplified from pGL3-Basic Vector using primers LucN and LucC (Table 1).
pLuc11 was constructed by joining the large *Not*I*-Bgl*II fragment of plasmid pUbiqGFP23 with the small *Bgl*II*-Not*I fragment of plasmid pGEM-Luc5.
pETwtLuc1 is a derivative of plasmid pET28b(+) that contains the small *Nco*I-*Eco*RV fragment of plasmid pSP-luc+NF Fusion Vector instead of the *Nco*I*-Ecl*136II fragment of plasmid pET28b(+).
pwtLuc1 was generated by joining the large *Not*I*-Sca*I fragment of plasmid pLuc11 with the small *Sca*I*-Not*I fragment of plasmid pETwtLuc1.
pLuc-PEST10 was generated by joining the large *Not*I*-EcoR*I fragment of plasmid pLuc11 with the synthetic DNA fragment that encodes a mutant C-terminal region of the mouse ornithine decarboxylase (mODC), which synthetic fragment was formed by oligonucleotides PEST-5', PEST-3', 5'-PEST and 3'-PEST (Table 1).
pT7LucPEST10 was generated by joining the large *Bgl*II*-Sca*I fragment of plasmid pLuc-PEST10 with the small *Sca*I*-Bgl*II fragment of plasmid pETwtLuc1.
pLucΔRI17 was constructed by treatment of plasmid pluc11 with *EcoRI,* T4 DNA polymerase and ligase.
pSPUbiqLuc1 was generated by combining BstEII-linearized DNA of plasmid pSP-luc+NF Fusion Vector with the fragment of plasmid pUbiqGFP23 which encodes ubiquitin. The ubiquitin fragment was prepared using PCR and primers Ubiquitin 5' wt/*Bsyt*EII and Ubiquitin 3' w/BstEII (Table 1), and subsequent treatment with *Bst*EII.
pETUbiqLuc was constructed by joining the large *Nco*I*-Ecl*136II fragment of plasmid pETBirA with the small *Nco*I*-Eco*RV fragment of plasmid pSPUbiqLuc1.
pUbiqLuc15 was prepared by joining the large fragment of plasmid pUbiqGFP23, which was generated by treatment of pUbiqGFP23 with *Age*I*,* S1 nuclease and *Xba*I, with the small fragment of plasmid pGL3 Basic Vector, which was generated by treatment of pGL3-Basic Vector with *Nco*I*,* S1 nuclease and *Xba*I.
pUbiq(Y)Luc19 was generated by combining the large *Xba*I *Xmn*I fragment of plasmid pUbiqGFP23 with the small *Xba*I*-Xmn*I fragment of plasmid pSPUbiqLuc1.
pT7Ubiq(I)Luc19.1, pT7Ubiq(E)Luc19.1 and pT7Ubiq(M)Luc19.2 were generated by combining the large *Bam*HI*-Apa*I fragment of plasmid pUbiq(Y)Luc19 with *Bam*HI*-Apa*I treated DNA fragments which had been amplified by PCR using plasmid pUbiqLuc15 and primers Ubi-Luc 5' w/Linker and Ubi-Luc 3' with linker or Ubi-Luc3' w/Linker Glu or Ubi-Luc 3' w/Linker Met, accordingly (Table 1).
pT7Ubiq(Y)Luc 19.2 was generated by joining the large *Bam*HI*-Xmn*I fragment of plasmid pT7Ubiq(I)Luc19.1 with the small *Bam*HI*-Xmn*I fragment of plasmid pUbiq(Y)Luc19.
pUbiq(R)Luc13 was generated by combining the large *Bst*EII*-Xmn*I fragment of plasmid pUbiq(Y)Luc19 with the *Bst*EII*-Xmn*I treated DNA fragments, which had been amplified by PCR using plasmid pUbiq(Y)Luc19 and primers Ubiquitin 5'wt/BsytEII and Ubiq(R) (Table 1).
pUbiq(A)Luc2, pUbiq(Asp2)Luc16, pUbiq(F)Luc10, pUbiq(His2)Luc3, pUbiq(H)Luc11, pUbiq(L)Luc23, pUbiq(K)Luc4, pUbiq(N)Luc25, pUbiq(Q)Luc36 and pUbiq(W)Luc16 were constructed by combining the large *Bst*EII-*Xmn*I fragment of plasmid pUbiq(R)Luc13 with *BstEII-XmnI* treated DNA fragments which had been amplified by PCR using plasmid pUbiq(Y)Luc19 and primers Ubiquitin 5'wt/*Bsy*tEII and Ala or Asp, or Phe, or His2, or His, or Leu, or Lys, or Asn, or Gln, or Trp, respectively (Table 1).
pUbiq(H)ΔLuc18 was constructed by treatment of plasmid pUbiq(H)Luc11 with *Bst*EII, T4 DNA polymerase and ligase.
pUbiq(E)ΔLuc6 was generated by joining the large *Sea*I*-Xmn*I fragment of plasmid pUbiq(H)ΔLuc18 with a *Sca*I*-Xmn*I treated PCR amplified fragments. The fragments were amplified from plasmid pT7Ubiq(E)Luc19.1 as separate DNA fragments using primers Ubiquitin 5'wt/BsytEII and Ubiq(E)de15' or Ubiq(E)de13' and LucC (Table 1) and then those fragments were combined in a separate PCR using primers Ubiquitin 5'wt/BsytEII and LucC.
pT7Ubiq(E)LucPEST23 was generated by joining the large *Bst*98I*-Sca*I fragment of plasmid pT7Ubiq(I)Luc19.1 with the small *Bst*98I-*Sca*I-fragment of plasmid pLuc-PEST10.
pUbiq(R)Luc-PEST12 and pUbiq(Y)Luc-PEST5 were generated by joining the small *Bst*98I*-Sca*I fragment of plasmid pLuc-PEST10 with the large *Bst*98I*-Sca*I fragment of plasmids pUbiq(R)Luc13 and pUbiq(Y)Luc19, accordingly.
pGEMhLuc+5 was constructed by cloning into pGEM^{®-}T Easy Vector a fragment that encodes firefly luciferase, which fragment was amplified using a template with an optimized firefly luciferase sequence and primers Luc+N and Luc+C (Table 1).
phLuc+PEST1 was generated by joining the small *Eco*RI*-Hind*III fragment of plasmid pGEMhLuc+5 with the large *Eco*RI*-Hind*III fragment of plasmid pLuc-PEST10.
pT7Ubiq(E)hLuc+PEST80 was generated by joining the small *BstEII-VspI* fragment of plasmid pT7Ubiq(I)Luc19.1 with the large *BstEII-VspI* fragment of plasmid phLuc+PEST1.
A sequence containing the promoter of the human hsp70 gene (Pₕₛₚ₇₀) was amplified from human chromosomal DNA using PCR and primers 5'-ATTAATCTGATCAATAAAGGGTTTAAGG (SEQ ID NO:1) and 5'-AAAAAGGTAGTGGACTGTCG (SEQ ID NO:2).
A UTR destabilization sequence was assembled using primers: 5'-CTAGATTTATTTATTTATTTCTTCATATGC (SEQ ID NO:3) and 5'-AATTGCATATGAAGAAATAAATAAATAAAT (SEQ ID NO:4).
A BKB destabilization sequence was assembled using primers: 5'-AATTGGGAATTAAAACAGCATTGAACCAAGAAGCTTGGCTTTCT TATCAATTCTTTGTGACATAATAAGTT (SEQ ID NO:5) and 5'-AACTTATTATGTCACAAAGAATTGATAAGAAAGCCAAGCTTCTT GGTTCAATGCTGTTTTAATTCCC (SEQ ID NO:6).
A mutant mODC PEST sequence (HGFPPEMEEQAAGTLPMSCAQESGMDRHPAACASARINV (corresponding to resides 423-461 of mODC; SEQ ID NO:7) was assembled using primers: 5'-AATTCTCATGGCTTCCCGCCGGAGATGGAGGAGCAGGCTGCTGG CACGCTGCCCATGTCTT (SEQ ID NO:8),5'-GTGCCCAGGAGAGCGGGATGGACCGTCACCCTGCAGCCTGTGCT TCTGCTAGGATCAATGTGTAA (SEQ ID NO:9), 5'-GGCCTTACACATTGATCCTAGCAGAAGCACAGGCTGCAGGGTG ACGGTCCATCCCGCTCTCCT (SEQ ID NO:10) and 5'-GGGCACAAGACATGGGCAGCGTGCCAGCAGCCTGCTCCTCCATC TCCGGCGGGAAGCCATGAG (SEQ ID NO:11).
A CL1 sequence (ACKNWFSSLSHFVIHL; SEQ ID NO: 12) was assembled using oligonucleotides: 5'-AATTCAAGTGGATCACGAAGTGGCTCAAGCTGCTGAACCAGTTC TTGCAGGCAGACA (SEQ ID NO: 13) and 5'-AATTTGTCTGCCTGCAAGAACTGGTTCAGCAGCTTGAGCCACTT CGTGATCCACTTG (SEQ ID NO:14).
An optimized PEST sequence (hPEST) has the following sequence: 
CACGGCTTCCCtCCCGAGGTGGAGGAGCAGGCCGCCGGCACCCT
GCCCATGAGCTGCGCCCAGGAGAGCGGCATGGAtaGaCACCCtGCt
GCtTGCGCCAGCGCCAGGATCAACGTcTAA (SEQ ID NO:15).
An optimized CL1 and hPEST with a UTR sequence has the following sequence: 
GCtTGCAAGAACTGGTTCAGtAGCtTaAGCCACTTtGTGATCCACCT
tAACAGCCACGGCTTCCCtCCCGAGGTGGAGGAGCAGGCCGCCG
GCACCCTGCCCATGAGCTGCGCCCAGGAGAGCGGCATGGAtaGaC
ACCCtGCtGCtTGCGCCAGCGCCAGGATCAACGTcTAg (SEQ ID
NO:46).
pGEM-hRL3 was constructed by cloning into pGEM^{®}T Easy Vector a PCR amplified optimized sequence that encodes *Renilla* luciferase, which was amplified from phRL-TK using primers hRLN and hRLC (Table 1).
phRL-PEST15 was generated by joining the large *Hind*III-*Eco*RI fragment of plasmid pLuc-PEST10 with the small *Hind*III*-Eco*RI fragment of plasmid pGEM-hRL3.
phRLΔRI-PEST1 was constructed by treatment of plasmid phRL-PEST15 with *EcoRI,* T4 DNA polymerase and ligase.
pT7Ubiq(E)hRL-PEST65 and pUbiq(R)hRL-PEST45 were generated by joining the large *Bst*EII*-Vsp*I fragment of plasmid phRL-PEST15 with the small *Bst*EII*-Vsp*I fragment of plasmids pT7Ubiq(E)Luc19.1 and pUbiq(R)Luc13, accordingly.
pUbiq(A)hRL1, pUbiq(H)hRL1, pUbiq(F)hRL1 were generated by joining the large *Bst*98I*-Bst*EII fragment of plasmid phRLΔRI-PEST1 with the small *Bst*98I*-Bst*EII fragment of plasmids pUbiq(A)Luc2 or pUbiq(His2)Luc3 or pUbiq(F)Luc10, accordingly.
pUbiq(E)hRL1 and pUbiq(R)hRL1 were created by joining the large *Hind*III-*Eco*RV fragment of plasmid phRLΔRI-PEST1 with the small *Hind*III-*Eco*RV fragment of plasmids pT7Ubiq(E)hRL-PEST65 or pUbiq(R)hRL-PEST45, accordingly.
pGEM-tetO1 was constructed by cloning into pGEM^{®}T Easy Vector a PCR amplified sequence that encodes a hCMV minimal promoter with heptamerized upstream tet-operators (Gossen, 1992), which was amplified from pUHD 10-3 using primers tetO-3' and tetO-5' (Table 1).
ptetO-hRL9 was generated by treatment of plasmid ptetO-hRL1-PEST1 with endonuclease *Eco*RI, T4 DNA polymerase and ligase.
ptetO-hRL-PEST1 was generated by joining the large *Nhe*I*-Vsp*I fragment of plasmid phRL-PEST15 with the small *Nhe*I*-Vsp*I fragment of plasmid pGEM-tetO1.
ptetO-T7Ubiq(E)hRL-PEST15 was generated by joining the large *Nhe*I-*Vsp*I fragment of plasmid pT7Ubiq(E)hRL-PEST65 with the small *Nhe*I*-Vsp*I fragment of plasmid pGEM-tetO1.
ptetO-Ubiq(E)hRL-PEST6 was constructed by treatment of plasmid ptetO-T7Ubiq(E)hRL-PEST15 with *Xba*I and Eco47III, T4 DNA polymerase and ligase.
ptetO-Ubiq(E)hRL-PEST-UTR13 was created by joining the large *Muni-Xba*I fragment of plasmid ptetO-Ubiq(E)hRL-PEST6 with the adaptor formed by oligonucleotides AUUU and anti-AUUU (Table 1).
ptetO-hRL-PEST-UTR12 was created by joining the large *Pst*I*-Kpn*I fragment of plasmid ptetO-Ubiq(E)hRL-PEST-UTR13 with the small *Pst*I*-Kpn*I fragment of plasmid ptetO-hRL-CL1-PEST11.
ptetO-Ubiq(E)hRL-PEST-BKB24 was created by joining the large *Muni-Hpa*I fragment of plasmid ptetO-T7Ubiq(E)hRL-PEST15 with the adaptor formed by oligonucleotides 3'-BKB1 and 5'-BKB1rev (Table 1).
ptetO-T7Ubiq(E)hRL-PEST-UTR-BKB8 was generated by joining the large *Nhe*I*-Muni* fragment of plasmid ptetO-Ubiq(E)hRL-PEST-BKB24 with the small *Nhe*I*-Muni* fragment of plasmid ptetO-Ubiq(E)hRL-PEST-UTR16.
ptetO-Ubiq(E)hRL-PEST-UTR16 was generated by joining the large *Mun*I*-Xba*I fragment of plasmid ptetO-Ubiq(E)hRL-PEST6 with the DNA fragment formed by oligonucleotides AUUU (SEQ ID NO:3) and Anti-AUUU (SEQ ID NO:4).
ptetO-hRL-CL1-PEST11 was generated by joining the large *Eco*RI fragment of plasmid ptetO-hRL-PEST1 with the DNA fragment formed by oligonucleotides CL1-N-final (SEQ ID NO:64) and Rev-CL1-N-final (SEQ ID NO:65).
ptetO-hRL-CL1-PEST-UTR1 was generated by joining the large *Pst*I*-Kpn*I fragment of plasmid ptetO-Ubiq(E)hRL-PEST-UTR16 with the small *Pst*I-KpnI fragment of plasmid ptetO-hRL-CL1-PEST1.
pGEM-Phsp70-3 was constructed by cloning into pGEM^{®}T Easy Vector a PCR amplified sequence which was amplified from human DNA using primers hsp70-5' and hsp70-3' (Table 1).
pPhsp70-hRL-PEST15 was generated by joining the large *NheI-VspI* fragment of plasmid ptetO-hRL-PEST1 with the small *Nhe*I*-Ysp*I fragment of plasmid pGEM-Phsp70-3.
pPhsp70-hRL7 was constructed by treatment of plasmid pPhsp70-hRL-PEST15 with *EcoRI,* T4 DNA polymerase and ligase.
pPhsp70-Ubiq(E)hRL-PEST1 was generated by joining the large *Nhe*I*-Vsp*I fragment of plasmid ptetO-Ubiq(E)hRL-PEST6 with the small *Nhe*I*-Vsp*I fragment of plasmid pGEM-Phsp70-3.
pPhsp70-Ubiq(E)hRL-PEST-UTR10 was generated by joining the large *Nhe*I-*Vsp*I fragment of plasmid ptetO-Ubiq(E)hRL-PEST-UTR16 with the small *NheI-VspI* fragment of plasmid pGEM-Phsp70-3.
pPhsp70-T7Ubiq(E)hRL-PEST-BKB5 was generated by joining the large *Nhe*I-*Vsp*I fragment of plasmid ptetO-T7Ubiq(E)hRL-PEST-BKB24 with the small *NheI-VspI* fragment of plasmid pGEM-Phsp70-3.
pPhsp70-T7Ubiq(E)hRL-PEST-UTR-BKB7 was generated by joining the large *NheI-VspI* fragment of plasmid ptetO-T7Ubiq(E)hRL-PEST-UTR-BKB8 with the small *Nhe*I*-Vsp*I fragment of plasmid pGEM-Phsp70-3.
pLucCL1-25 was generated by joining the large *Eco*RI*-Not*I fragment of plasmid pLuc11 with the DNA fragment formed by oligonucleotides CL1 (SEQ ID. NO:62) and Rev-CL1 (SEQ ID NO:63).
pLucCL1-PEST9 was generated by joining the large *EcoRI* fragment of plasmids pLuc-PEST10 with the DNA fragment formed by oligonucleotides CL1-N-final (SEQ ID NO:64) and Rev-CL1-N-final (SEQ ID NO:65).
pCL1-Luc1 was generated by joining the large *Hind*III-*Bgl*II fragment of plasmid pLucΔRI17 with the DNA fragment formed by oligonucleotides CL1-N (SEQ ID NO:64) and Rev-CL1-N (SEQ ID NO:65).
phLuc+CL1-PEST13 was generated by joining the large *EcoRI* fragment of plasmid phLuc+PEST1 with the DNA fragment formed by oligonucleotides CL1-N-final (SEQ ID NO:64) and Rev-CL1-N-final (SEQ ID NO:65).
pPhsp70hLuc+PEST2 was generated by joining the large *Eco*RI*-Nhe*I fragment of plasmid phLuc+PEST1 with the small *Eco*RI*-Nhe*I fragment of plasmid pPhsp70hRL-PEST15.
pPhsp70hLuc+14 was constructed by treatment of plasmid pPhsp70hLuc+PEST2 with *EcoRI,* T4 DNA polymerase and ligase.
pPhsp70hRL-CL1-PEST-UTR4 was generated by joining the large *Vsp*I*-Nhe*I fragment of plasmid ptetO-hRL-CL1-PEST-UTR1 with the small *Vsp*I-*Nhe*I fragment of plasmid pGEM-Phsp70-3.
pPhsp70hLuc+CL1-PEST12, pPhsp70hLuc+CL1-PEST-UTR5 were generated by joining the small *Eco*RI*-Nhe*I fragment of plasmid phLuc+CL1-PEST13 with large *Eco*RI*-Nhe*I fragments of plasmids pPhsp70hRL-PEST15 and pPhsp70hRL-CL1-PEST-UTR4, respectively.
pPhsp70MhLuc+27, pPhsp70MhLuc+PEST25, pPhsp70MhLuc+CLl-PEST32 and pPhsp70MhLuc+CL1-PEST-UTR19 were constructed by cloning DNA fragment formed by oligonucleotides N-M and M-C (Table.1) into plasmids pPhsp70hLuc+14, pPhsp70hLuc+PEST2, pPhsp70hLuc+CL1-PEST12 and pPhsp70hLuc+CL1-PEST-UTR5, respectively, that were treated with *Bst*EII and *Bgl*II*.*
phRL-PEST14 was constructed by joining the large *Eco*RV-NheI fragment of plasmid phRL-PEST15 with the small *Eco*RV-NheI fragment of plasmid phRL-TK.
pGL3-hRL-PEST3 was constructed by joining the large *Bst*98I-*Xba*I fragment of plasmid pGL3-Ubiq(E)hRL-PEST2 with the small *Bst*98I-*Xba*I fragment of plasmid phRL-PEST14.
pGL3-bRL1 was constructed by joining the large *Bst*98I-*Eco*RV fragment of plasmid pGL3-hRL-PEST3 with the small *Bst*98I-*Eco*RV fragment of plasmid phRL3.
pGL3-hRL-CL1-PEST-UTR23 was constructed by joining the large *Bst*98I-*Eco*RV fragment of plasmid pGL3-hRL-PEST3 with the small *Bst*98I-*Eco*RV fragment of plasmid ptetO-hRL-CL1-PEST-UTR1.
pGL3-hRL-PEST-UTR6 was constructed by joining the large *Bst*98I*-Eco*RV fragment of plasmid pGL3-hRL-PEST3 with the small *Bst*981*-Eco*RV fragment of plasmid ptetO-Ubiq(E)hRL-PEST-UTR16.
pGL3-hRL-CL1-PEST7 was constructed by joining the large *Bst*98I*-Eco*RV fragment of plasmid pGL3-hRL-PEST3 with the small *Bst*98I*-Eco*RV fragment of plasmid ptetO-hRL-CL1-PEST11.

An optimized *Renilla* luciferase DNA has the following sequence:

atggcttccaaggtgtacgaccccgagcaacgcaaacgcatgatcactgggcctcagtggtgggctcgctgcaag
c
aaatgaacgtgctggactccttcatcaactactatgattccgagaagcacgccgagaacgccgtgatttttctgca
tggtaacgctgcctccagctacctgtggaggcacgtcgtgcctcacatcgagcccgtggctagatgcatcatccct
gatctgatcggaatgggtaagtccggcaagagcgggaatggctcatatcgcctcctggatcactacaagtacctca
ccgcttggttcgagctgctgaaccttccaaagaaaatcatctttgtgggccacgactgggggcttgtctggcctt
tcactactcctacgagcaccaagacaagatcaaggccatcgtccatgctgagagtgtcgtggacgtgatcgagtcc
tgggacgagtggcctgacatcgaggaggatatcgccctgatcaagagcgaagagggcgagaaaatggtgcttga
ga
ataacttcttcgtcgagaccatgctcccaagcaagatcatgcggaaactggagcctgaggagttcgctgcctacct
ggagccattcaaggagaagggcgaggttagacggcctaccctctcctggcctcgcgagatccctctcgttaaggg
a
ggcaagcccgacgtcgtccagattgtccgcaactacaacgcctaccttcgggccagcgacgatctgcctaagatgt

tcatcgagtccgaccctgggttcttttccaacgctattgtcgagggagctaagaagttccctaacaccgagttcgt
gaaggtgaagggcctccacttcagccaggaggacgctccagatgaaatgggtaagtacatcaagagcttcgtgga
g
cgcgtgctgaagaacgagcagtaa (SEQ ID NO:47).

An optimized firefly luciferase DNA has the following sequence:

atggccgatgctaagaacattaagaagggccctgctcccttctaccctctggaggatggcaccgctggcgagcag
c
tgcacaaggccatgaagaggtatgccctggtgcctggcaccattgccttcaccgatgcccacattgaggtggacat
cacctatgccgagtacttcgagatgtctgtgcgcctggccgaggccatgaagaggtacggcctgaacaccaacca
c
cgcatcgtggtgtgctctgagaactctctgcagttcttcatgccagtgctgggcgccctgttcatcggagtggccg
tggcccctgctaacgacatttacaacgagcgcgagctgctgaacagcatgggcatttctcagcctaccgtggtgtt
cgtgtctaagaagggcctgcagaagatcctgaacgtgcagaagaagctgcctatcatccagaagatcatcatcatg
gactctaagaccgactaccagggcttccagagcatgtacacattcgtgacatctcatctgcctcctggcttcaacg
agtacgacttcgtgccagagtctttcgacagggacaaaaccattgccctgatcatgaacagctctgggtctaccgg
cctgcctaagggcgtggccctgcctcatcgcaccgcctgtgtgcgcttctctcacgcccgcgaccctatttcggc
aaccagatcatccccgacaccgctattctgagcgtggtgccattccaccacggcttcggcatgttcaccaccctgg
gctacctgatttgcggctttcgggtggtgctgatgtaccgcttcgaggaggagctgttcctgcgcagcctgcaaga
ctacaaaattcagtctgccctgctggtgccaaccctgttcagcttcttcgctaagagcaccctgatcgacaagtac
gacctgtctaacctgcacgagattgcctctggcggcgccccactgtctaaggaggtgggcgaagccgtggccaa
gc
gctttcatctgccaggcatccgccagggctacggcctgaccgagacaaccagcgccattctgattaccccagagg
g
cgacgacaagcctggcgccgtgggcaaggtggtgccattcttcgaggccaaggtggtggacctggacaccggc
aag
accctgggagtgaaccagcgcggcgagctgtgtgtgcgcggccctatgattatgtccggctacgtgaataaccctg
aggccacaaacgccctgatcgacaaggacggctggctgcactctggcgacattgcctactgggacgaggacga
gca
cttcttcatcgtggaccgcctgaagtctctgatcaagtacaagggctaccaggtggccccagccgagctggagtct
atcctgctgcagcaccctaacattttcgacgccggagtggccggcctgcccgacgacgatgccggcgagctgcct
g
ccgccgtcgtcgtgctggaacacggcaagaccatgaccgagaaggagatcgtggactatgtggccagccaggtg
ac
aaccgccaagaagctgcgcggcggagtggtgttcgtggacgaggtgcccaagggcctgaccggcaagctgga
cgcccgcaagatccgcgagatcctgatcaaggctaagaaaggcggcaagatcgccgtgtaa (SEQ ID
NO:48).

An optimized mutant firefly luciferase DNA has the following sequence:

atggccgatgctaagaacattaagaagggccctgctcccttctaccctctggaggatggcaccgctggcgagcag
c
tgcacaaggccatgaagaggtatgccctggtgcctggcaccattgccttcaccgatgcccacattgaggtggacat
cacctatgccgagtacttcgagatgtctgtgcgcctggccgaggccatgaagaggtacggcctgaacaccaacca
c
cgcatcgtggtgtgctctgagaactctctgcagttcttcatgccagtgctgggcgccctgttcatcggagtggccg
tggcccctgctaacgacatttacaacgagcgcgagctgctgaacagcatgggcatttctcagcctaccgtggtgtt
cgtgtctaagaagggcctgcagaagatcctgaacgtgcagaagaagctgcctatcatccagaagatcatcatcatg
gactctaagaccgactaccagggcttccagagcatgtacacattcgtgacatctcatctgcctcctggcttcaacg
agtacgacttcgtgccagagtctttcgacagggacaaaaccattgccctgatcatgaacagctctgggtctaccgg

cctgcctaagggcgtggccctgacccatcgcaacgcctgtgtgcgcttctctcacgcccgcgaccctattttcggc
aaccagatcatccccgacaccgctattctgagcgtggtgccattccaccacggcttcggcatgttcaccaccctgg
gctacctgatttgcggctttcgggtggtgctgatgtaccgcttcgaggaggagctgttcctgcgcagcctgcaaga
ctacaaaattcagtctgccctgctggtgccaaccctgttcagcttcttcgctaagagcaccctgatcgacaagtac
gacctgtctaacctgcacgagattgcctctggcggcgccccactgtctaaggaggtgggcgaagccgtggccaa
gc
gctttcatctgccaggcatccgccagggctacggcctgaccgagacaaccagcgccattctgattaccccagagg
g
cgacgacaagcctggcgccgtgggcaaggtggtgccattcttcgaggccaaggtggtggacctggacaccggc
aag
accctgggagtgaaccagcgcggcgagctgtgtgtgcgcggccctatgattatgtccggctacgtgaataaccctg
aggccacaaacgccctgatcgacaaggacggctggctgcactctggcgacattgcctactgggacgaggacga
gca
cttcttcatcgtggaccgcctgaagtctctgatcaagtacaagggctaccaggtggccccagccgagctggagtct
atcctgctgcagcaccctaacattttcgacgccggagtggccggcctgcccgacgacgatgccggcgagctgcct
g
ccgccgtcgtcgtgctggaacacggcaagaccatgaccgagaaggagatcgtggactatgtggccagccaggtg
ac
aaccgccaagaagctgcgcggcggagtggtgttcgtggacgaggtgcccaagggcctgaccggcaagctgga
cgcccgcaagatccgcgagatcctgatcaaggctaagaaaggcggcaagatcgccgtgtaa (SEQ ID
NO:49).

Other optimized firefly luciferase sequences include hluc+: 
ATGGCCGATGCTAAGAACATTAAGAAGGGCCCTGCTCCCTTCTACCC
TCTGGAGGATGGCACCGCTGGCGAGCAGCTGCACAAGGCCATGAAG
AGGTATGCCCTGGTGCCTGGCACCATTGCCTTCACCGATGCCCACATT
GAGGTGGACATCACCTATGCCGAGTACTTCGAGATGTCTGTGCGCCT
GGCCGAGGCCATGAAGAGGTACGGCCTGAACACCAACCACCGCATC
GTGGTGTGCTCTGAGAACTCTCTGCAGTTCTTCATGCCAGTGCTGGGC
GCCCTGTTCATCGGAGTGGCCGTGGCCCCTGCTAACGACATTTACAA
CGAGCGCGAGCTGCTGAACAGCATGGGCATTTCTCAGCCTACCGTGG
TGTTCGTGTCTAAGAAGGGCCTGCAGAAGATCCTGAACGTGCAGAAG
AAGCTGCCTATCATCCAGAAGATCATCATCATGGACTCTAAGACCGA
CTACCAGGGCTTCCAGAGCATGTACACATTCGTGACATCTCATCTGCC
TCCTGGCTTCAACGAGTACGACTTCGTGCCAGAGTCTTTCGACAGGG
ACAAAACCATTGCCCTGATCATGAACAGCTCTGGGTCTACCGGCCTG
CCTAAGGGCGTGGCCCTGCCCCATCGCACCGCCTGTGTGCGCTTCTCT
CACGCCCGCGACCCTATTTTCGGCAACCAGATCATCCCCGACACCGC
TATTCTGAGCGTGGTGCCATTCCACCACGGCTTCGGCATGTTCACCAC
CCTGGGCTACCTGATTTGCGGCTTTCGGGTGGTGCTGATGTACCGCTT
CGAGGAGGAGCTGTTCCTGCGCAGCCTGCAAGACTACAAAATTCAGT
CTGCCCTGCTGGTGCCAACCCTGTTCAGCTTCTTCGCTAAGAGCACCC
TGATCGACAAGTACGACCTGTCTAACCTGCACGAGATTGCCTCTGGC
GGCGCCCCACTGTCTAAGGAGGTGGGCGAAGCCGTGGCCAAGCGCTT
TCATCTGCCAGGCATCCGCCAGGGCTACGGCCTGACCGAGACAACCA
GCGCCATTCTGATTACCCCAGAGGGCGACGACAAGCCTGGCGCCGTG
GGCAAGGTGGTGCCATTCTTCGAGGCCAAGGTGGTGGACCTGGACAC
CGGCAAGACCCTGGGAGTGAACCAGCGCGGCGAGCTGTGTGTGCGC
GGCCCTATGATTATGTCCGGCTACGTGAATAACCCTGAGGCCACAAA
CGCCCTGATCGACAAGGACGGCTGGCTGCACTCTGGCGACATTGCCT
 
ACTGGGACGAGGACGAGCACTTCTTCATCGTGGACCGCCTGAAGTCT
CTGATCAAGTACAAGGGCTACCAGGTGGCCCCAGCCGAGCTGGAGTC
TATCCTGCTGCAGCACCCTAACATTTTCGACGCCGGAGTGGCCGGCCT
GCCCGACGACGATGCCGGCGAGCTGCCTGCCGCCGTCGTCGTGCTGG
AACACGGCAAGACCATGACCGAGAAGGAGATCGTGGACTATGTGGC
CAGCCAGGTGACAACCGCCAAGAAGCTGCGCGGCGGAGTGGTGTTC
GTGGACGAGGTGCCCAAGGGCCTGACCGGCAAGCTGGACGCCCGCA
AGATCCGCGAGATCCTGATCAAGGCTAAGAAAGGCGGCAAGATCGC
CGTGTAA (SEQ ID NO: 66);
 and hLuc+ (5F2):

ATGGCCGATGCTAAGAACATTAAGAAGGGCCCTGCTCCCTTCTACCC
TCTGGAGGATGGCACCGCTGGCGAGCAGCTGCACAAGGCCATGAAG
AGGTATGCCCTGGTGCCTGGCACCATTGCCTTCACCGATGCCCACATT
GAGGTGGACATCACCTATGCCGAGTACTTCGAGATGTCTGTGCGCCT
GGCCGAGGCCATGAAGAGGTACGGCCTGAACACCAACCACCGCATC
GTGGTGTGCTCTGAGAACTCTCTGCAGTTCTTCATGCCAGTGCTGGGC
GCCCTGTTCATCGGAGTGGCCGTGGCCCCTGCTAACGACATTTACAA
CGAGCGCGAGCTGCTGAACAGCATGGGCATTTCTCAGCCTACCGTGG
TGTTCGTGTCTAAGAAGGGCCTGCAGAAGATCCTGAACGTGCAGAAG
AAGCTGCCTATCATCCAGAAGATCATCATCATGGACTCTAAGACCGA
CTACCAGGGCTTCCAGAGCATGTACACATTCGTGACATCTCATCTGCC
TCCTGGCTTCAACGAGTACGACTTCGTGCCAGAGTCTTTCGACAGGG
ACAAAACCATTGCCCTGATCATGAACAGCTCTGGGTCTACCGGCCTG
CCTAAGGGCGTGGCCCTGACCCATCGCAACGCCTGTGTGCGCTTCTCT
CACGCCCGCGACCCTATTTTCGGCAACCAGATCATCCCCGACACCGC
TATTCTGAGCGTGGTGCCATTCCACCACGGCTTCGGCATGTTCACCAC
CCTGGGCTACCTGATTTGCGGCTTTCGGGTGGTGCTGATGTACCGCTT
CGAGGAGGAGCTGTTCCTGCGCAGCCTGCAAGACTACAAAATTCAGT
CTGCCCTGCTGGTGCCAACCCTGTTCAGCTTCTTCGCTAAGAGCACCC
TGATCGACAAGTACGACCTGTCTAACCTGCACGAGATTGCCTCTGGC
GGCGCCCCACTGTCTAAGGAGGTGGGCGAAGCCGTGGCCAAGCGCTT
TCATCTGCCAGGCATCCGCCAGGGCTACGGCCTGACCGAGACAACCA
GCGCCATTCTGATTACCCCAGAGGGCGACGACAAGCCTGGCGCCGTG
GGCAAGGTGGTGCCATTCTTCGAGGCCAAGGTGGTGGACCTGGACAC
CGGCAAGACCCTGGGAGTGAACCAGCGCGGCGAGCTGTGTGTGCGC
GGCCCTATGATTATGTCCGGCTACGTGAATAACCCTGAGGCCACAAA
CGCCCTGATCGACAAGGACGGCTGGCTGCACTCTGGCGACATTGCCT
ACTGGGACGAGGACGAGCACTTCTTCATCGTGGACCGCCTGAAGTCT
CTGATCAAGTACAAGGGCTACCAGGTGGCCCCAGCCGAGCTGGAGTC
TATCCTGCTGCAGCACCCTAACATTTTCGACGCCGGAGTGGCCGGCCT
GCCCGACGACGATGCCGGCGAGCTGCCTGCCGCCGTCGTCGTGCTGG
AACACGGCAAGACCATGACCGAGAAGGAGATCGTGGACTATGTGGC
CAGCCAGGTGACAACCGCCAAGAAGCTGCGCGGCGGAGTGGTGTTC
GTGGACGAGGTGCCCAAGGGCCTGACCGGCAAGCTGGACGCCCGCA
AGATCCGCGAGATCCTGATCAAGGCTAAGAAAGGCGGCAAGATCGC
CGTGTAA (SEQ ID NO: 49).

An optimized GFP sequence has the following sequence:

ATGGGCGTGATCAAGCCCGACATGAAGATCAAGCTGCGgATGGAGGG
CGCCGTGAACGGCCACAAaTTCGTGATCGAGGGCGACGGgAAaGGCA
AGCCCTTtGAGGGtAAGCAGACtATGGACCTGACCGTGATCGAGGGCG
CCCCCCTGCCCTTCGCtTAtGACATtCTcACCACCGTGTTCGACTACGGt
AACCGtGTcTTCGCCAAGTACCCCAAGGACATCCCtGACTACTTCAAGC
AGACCTTCCCCGAGGGCTACtcgTGGGAGCGaAGCATGACaTACGAGG
ACCAGGGaATCTGtATCGCtACaAACGACATCACCATGATGAAGGGtGT
GGACGACTGCTTCGTGTACAAaATCCGCTTCGACGGgGTcAACTTCCCt
GCtAAtGGCCCgGTgATGCAGCGCAAGACCCTaAAGTGGGAGCCCAGtA
CCGAGAAGATGTACGTGCGgGACGGCGTaCTGAAGGGCGAtGTtAAtAT
GGCaCTGCTctTGGAGGGaGGCGGCCACTACCGCTGCGACTTCAAGACC
ACCTACAAaGCCAAGAAGGTGGTGCAGCTtCCCGACTACCACTTCGTG
GACCACCGCATCGAGATCGTGAGCCACGACAAGGACTACAACAAaGT
cAAGCTGTACGAGCACGCCGAaGCCCACAGCGGaCTaCCCCGCCAGGC
CggcTAA (SEQ ID NO: 68).

An optimized firefly luciferase (hluc+(5f2))-optimized PEST sequence (hluc+(5f2)-hPEST) has the following sequence:

atggccgatgctaagaacattaagaagggccctgctcccttctaccctctggaggatggcaccgctggcgagcag
ctgcacaaggccatgaagaggtatgccctggtgcctggcaccattgccttcaccgatgcccacattgaggtggaca
tcacctatgccgagtacttcgagatgtctgtgcgcctggccgaggccatgaagaggtacggcctgaacaccaacc
accgcatcgtggtgtgctctgagaactctctgcagttcttcatgccagtgctgggcgccctgttcatcggagtggccg
tggcccctgctaacgacatttacaacgagcgcgagctgctgaacagcatgggcatttctcagcctaccgtggtgttc
gtgtctaagaagggcctgcagaagatcctgaacgtgcagaagaagctgcctatcatccagaagatcatcatcatgg
actctaagaccgactaccagggcttccagagcatgtacacattcgtgacatctcatctgcctcctggcttcaacgagt
acgacttcgtgccagagtctttcgacagggacaaaaccattgccctgatcatgaacagctctgggtctaccggcctg
cctaagggcgtggccctgacccatcgcaacgcctgtgtgcgcttctctcacgcccgcgaccctattttcggcaacc
agatcatccccgacaccgctattctgagcgtggtgccattccaccacggcttcggcatgttcaccaccctgggctac
ctgatttgcggctttcgggtggtgctgatgtaccgcttcgaggaggagctgttcctgcgcagcctgcaagactacaa
aattcagtctgccctgctggtgccaaccctgttcagcttcttcgctaagagcaccctgatcgacaagtacgacctgtct
aacctgcacgagattgcctctggcggcgccccactgtctaaggaggtgggcgaagccgtggccaagcgctttcat
ctgccaggcatccgccagggctacggcctgaccgagacaaccagcgccattctgattaccccagagggcgacg
acaagcctggcgccgtgggcaaggtggtgccattcttcgaggccaaggtggtggacctggacaccggcaagac
cctgggagtgaaccagcgcggcgagctgtgtgtgcgcggccctatgattatgtccggctacgtgaataaccctga
ggccacaaacgccctgatcgacaaggacggctggctgcactctggcgacattgcctactgggacgaggacgag
cacttcttcatcgtggaccgcctgaagtctctgatcaagtacaagggctaccaggtggccccagccgagctggagt
ctatcctgctgcagcaccctaacattttcgacgccggagtggccggcctgcccgacgacgatgccggcgagctgc
ctgccgccgtcgtcgtgctggaacacggcaagaccatgaccgagaaggagatcgtggactatgtggccagccag
gtgacaaccgccaagaagctgcgcggcggagtggtgttcgtggacgaggtgcccaagggcctgaccggcaag
ctggacgcccgcaagatccgcgagatcctgatcaaggctaagaaaggcggcaagatcgccgtgaattctCAC
GGCTTCCCtCCCGAGGTGGAGGAGCAGGCCGCCGGCACCCTGCCCAT
GAGCTGCGCCCAGGAGAGCGGCATGGAtaGaCACCCtGCtGCtTGCGCC
AGCGCCAGGATCAACGTcTAA (SEQ ID NO: 69).

An optimized firefly luciferase(hluc+(5f2))-optimized CL1-optimzed PEST sequence (hluc+(5f2)-hCL1-hPEST) has the following sequence:

atggccgatgctaagaacattaagaagggccctgctcccttctaccctctggaggatggcaccgctggcgagcag
ctgcacaaggccatgaagaggtatgccctggtgcctggcaccattgccttcaccgatgcccacattgaggtggaca

tcacctatgccgagtacttcgagatgtctgtgcgcctggccgaggccatgaagaggtacggcctgaacaccaacc
accgcatcgtggtgtgctctgagaactctctgcagttcttcatgccagtgctgggcgccctgttcatcggagtggccg
tggcccctgctaacgacatttacaacgagcgcgagctgctgaacagcatgggcatttctcagcctaccgtggtgttc
gtgtctaagaagggcctgcagaagatcctgaacgtgcagaagaagctgcctatcatccagaagatcatcatcatgg
actctaagaccgactaccagggcttccagagcatgtacacattcgtgacatctcatctgcctcctggcttcaacgagt
acgacttcgtgccagagtctttcgacagggacaaaaccattgccctgatcatgaacagctctgggtctaccggcctg
cctaagggcgtggccctgacccatcgcaacgcctgtgtgcgcttctctcacgcccgcgaccctattttcggcaacc
agatcatccccgacaccgctattctgagcgtggtgccattccaccacggcttcggcatgttcaccaccctgggctac
ctgatttgcggctttcgggtggtgctgatgtaccgcttcgaggaggagctgttcctgcgcagcctgcaagactacaa
aattcagtctgccctgctggtgccaaccctgttcagcttcttcgctaagagcaccctgatcgacaagtacgacctgtct
aacctgcacgagattgcctctggcggcgccccactgtctaaggaggtgggcgaagccgtggccaagcgctttcat
ctgccaggcatccgccagggctacggcctgaccgagacaaccagcgccattctgattaccccagagggcgacg
acaagcctggcgccgtgggcaaggtggtgccattcttcgaggccaaggtggtggacctggacaccggcaagac
cctgggagtgaaccagcgcggcgagctgtgtgtgcgcggccctatgattatgtccggctacgtgaataaccctga
ggccacaaacgccctgatcgacaaggacggctggctgcactctggcgacattgcctactgggacgaggacgag
cacttcttcatcgtggaccgcctgaagtctctgatcaagtacaagggctaccaggtggccccagccgagctggagt
ctatcctgctgcagcaccctaacattttcgacgccggagtggccggcctgcccgacgacgatgccggcgagctgc
ctgccgccgtcgtcgtgctggaacacggcaagaccatgaccgagaaggagatcgtggactatgtggccagccag
gtgacaaccgccaagaagctgcgcggcggagtggtgttcgtggacgaggtgcccaagggcctgaccggcaag
ctggacgcccgcaagatccgcgagatcctgatcaaggctaagaaaggcggcaagatcgccgtgaattctGCtT
GCAAGAACTGGTTCAGtAGCtTaAGCCACTTtGTGATCCACCTtAACAGC
CACGGCTTCCCtCCCGAGGTGGAGGAGCAGGCCGCCGGCACCCTGCC
CATGAGCTGCGCCCAGGAGAGCGGCATGGAtaGaCACCCtGCtGCtTGCG
CCAGCGCCAGGATCAACGTcTAg (SEQ ID NO: 70).

An optimized firefly luciferase (hluc+)-optimized PEST sequence (hluc+-hPEST) has the following sequence:

atggccgatgctaagaacattaagaagggccctgctcccttctaccctctggaggatggcaccgctggcgagcag
ctgcacaaggccatgaagaggtatgccctggtgcctggcaccattgccttcaccgatgcccacattgaggtggaca
tcacctatgccgagtacttcgagatgtctgtgcgcctggccgaggccatgaagaggtacggcctgaacaccaacc
accgcatcgtggtgtgctctgagaactctctgcagttcttcatgccagtgctgggcgccctgttcatcggagtggccg
tggcccctgctaacgacatttacaacgagcgcgagctgctgaacagcatgggcatttctcagcctaccgtggtgttc
gtgtctaagaagggcctgcagaagatcctgaacgtgcagaagaagctgcctatcatccagaagatcatcatcatgg
actctaagaccgactaccagggcttccagagcatgtacacattcgtgacatctcatctgcctcctggcttcaacgagt
acgacttcgtgccagagtctttcgacagggacaaaaccattgccctgatcatgaacagctctgggtctaccggcctg
cctaagggcgtggccctgcctcatcgcaccgcctgtgtgcgcttctctcacgcccgcgaccctatttcggcaacca
gatcatccccgacaccgctattctgagcgtggtgccattccaccacggcttcggcatgttcaccaccctgggctacc
tgatttgcggctttcgggtggtgctgatgtaccgcttcgaggaggagctgttcctgcgcagcctgcaagactacaaa
attcagtctgccctgctggtgccaaccctgttcagcttcttcgctaagagcaccctgatcgacaagtacgacctgtcta
acctgcacgagattgcctctggcggcgccccactgtctaaggaggtgggcgaagccgtggccaagcgctttcatc
tgccaggcatccgccagggctacggcctgaccgagacaaccagcgccattctgattaccccagagggcgacga
caagcctggcgccgtgggcaaggtggtgccattcttcgaggccaaggtggtggacctggacaccggcaagacc
ctgggagtgaaccagcgcggcgagctgtgtgtgcgcggccctatgattatgtccggctacgtgaataaccctgag
gccacaaacgccctgatcgacaaggacggctggctgcactctggcgacattgcctactgggacgaggacgagca
cttcttcatcgtggaccgcctgaagtctctgatcaagtacaagggctaccaggtggccccagccgagctggagtct
atcctgctgcagcaccctaacattttcgacgccggagtggccggcctgcccgacgacgatgccggcgagctgcct
gccgccgtcgtcgtgctggaacacggcaagaccatgaccgagaaggagatcgtggactatgtggccagccaggt
gacaaccgccaagaagctgcgcggcggagtggtgttcgtggacgaggtgcccaagggcctgaccggcaagct
ggacgcccgcaagatccgcgagatcctgatcaaggctaagaaaggcggcaagatcgccgtgaattctCACG
GCTTCCCtCCCGAGGTGGAGGAGCAGGCCGCCGGCACCCTGCCCATG

AGCTGCGCCCAGGAGAGCGGCATGGAtaGaCACCCtGCtGCtTGCGCCA
GCGCCAGGATCAACGTcTAA (SEQ ID NO: 71).

An optimized firefly luciferase (hluc+)-optimized CL1-optimized PEST sequence (hluc+-hCL1-hPEST) has the following sequence:

atggccgatgctaagaacattaagaagggccctgctcccttctaccctctggaggatggcaccgctggcgagcag
ctgcacaaggccatgaagaggtatgccctggtgcctggcaccattgccttcaccgatgcccacattgaggtggaca
tcacctatgccgagtacttcgagatgtctgtgcgcctggccgaggccatgaagaggtacggcctgaacaccaacc
accgcatcgtggtgtgctctgagaactctctgcagttcttcatgccagtgctgggcgccctgttcatcggagtggccg
tggcccctgctaacgacatttacaacgagcgcgagctgctgaacagcatgggcatttctcagcctaccgtggtgttc
gtgtctaagaagggcctgcagaagatcctgaacgtgcagaagaagctgcctatcatccagaagatcatcatcatgg
actctaagaccgactaccagggcttccagagcatgtacacattcgtgacatctcatctgcctcctggcttcaacgagt
acgacttcgtgccagagtctttcgacagggacaaaaccattgccctgatcatgaacagctctgggtctaccggcctg
cctaagggcgtggccctgcctcatcgcaccgcctgtgtgcgcttctctcacgcccgcgaccctattttcggcaacca
gatcatccccgacaccgctattctgagcgtggtgccattccaccacggcttcggcatgttcaccaccctgggctacc
tgatttgcggctttcgggtggtgctgatgtaccgcttcgaggaggagctgttcctgcgcagcctgcaagactacaaa
attcagtctgccctgctggtgccaaccctgttcagcttcttcgctaagagcaccctgatcgacaagtacgacctgtcta
acctgcacgagattgcctctggcggcgccccactgtctaaggaggtgggcgaagccgtggccaagcgctttcatc
tgccaggcatccgccagggctacggcctgaccgagacaaccagcgccattctgattaccccagagggcgacga
caagcctggcgccgtgggcaaggtggtgccattcttcgaggccaaggtggtggacctggacaccggcaagacc
ctgggagtgaaccagcgcggcgagctgtgtgtgcgcggccctatgattatgtccggctacgtgaataaccctgag
gccacaaacgccctgatcgacaaggacggctggctgcactctggcgacattgcctactgggacgaggacgagca
cttcttcatcgtggaccgcctgaagtctctgatcaagtacaagggctaccaggtggccccagccgagctggagtct
atcctgctgcagcaccctaacatttcgacgccggagtggccggcctgcccgacgacgatgccggcgagctgcct
gccgccgtcgtcgtgctggaacacggcaagaccatgaccgagaaggagatcgtggactatgtggccagccaggt
gacaaccgccaagaagctgcgcggcggagtggtgttcgtggacgaggtgcccaagggcctgaccggcaagct
ggacgcccgcaagatccgcgagatcctgatcaaggctaagaaaggcggcaagatcgccgtgaattctGCtTG
CAAGAACTGGTTCAGtAGCtTaAGCCACTTtGTGATCCACCTtAACAGCC
ACGGCTTCCCtCCCGAGGTGGAGGAGCAGGCCGCCGGCACCCTGCCC
ATGAGCTGCGCCCAGGAGAGCGGCATGGAtaGaCACCCtGCtGCtTGCGC
CAGCGCCAGGATCAACGTcTAg (SEQ ID NO: 72).

An optimized *Renilla* luciferase-optimized PEST sequence (hRenilla-hPEST) has the following sequence:

atggcttccaaggtgtacgaccccgagcaacgcaaacgcatgatcactgggcctcagtggtgggctcgctgcaag
caaatgaacgtgctggactccttcatcaactactatgattccgagaagcacgccgagaacgccgtgatttttctgcat
ggtaacgctgcctccagctacctgtggaggcacgtcgtgcctcacatcgagcccgtggctagatgcatcatccctg
atctgatcggaatgggtaagtccggcaagagcgggaatggctcatatcgcctcctggatcactacaagtacctcac
cgcttggttcgagctgctgaaccttccaaagaaaatcatctttgtgggccacgactgggggcttgtctggcctttca
ctactcctacgagcaccaagacaagatcaaggccatcgtccatgctgagagtgtcgtggacgtgatcgagtcctgg
gacgagtggcctgacatcgaggaggatatcgccctgatcaagagcgaagagggcgagaaaatggtgcttgaga
ataacttcttcgtcgagaccatgctcccaagcaagatcatgcggaaactggagcctgaggagttcgctgcctacctg
gagccattcaaggagaagggcgaggttagacggcctaccctctcctggcctcgcgagatccctctcgttaaggga
ggcaagcccgacgtcgtccagattgtccgcaactacaacgcctaccttcgggccagcgacgatctgcctaagatgt
tcatcgagtccgaccctgggttcttttccaacgctattgtcgagggagctaagaagttccctaacaccgagttcgtga
aggtgaagggcctccacttcagccaggaggacgctccagatgaaatgggtaagtacatcaagagcttcgtggagc
gcgtgctgaagaacgagcagaattctCACGGCTTCCCtCCCGAGGTGGAGGAGCAGG
CCGCCGGCACCCTGCCCATGAGCTGCGCCCAGGAGAGCGGCATGGAta

GaCACCCtGCtGCtTGCGCCAGCGCCAGGATCAACGTcTAA (SEQ ID NO:
73).

An optimized *Renilla* luciferase-optimized CL1-optimized PEST sequence (hRenilla-hCL1-hPEST) has the following sequence:

atggcttccaaggtgtacgaccccgagcaacgcaaacgcatgatcactgggcctcagtggtgggctcgctgcaag
caaatgaacgtgctggactccttcatcaactactatgattccgagaagcacgccgagaacgccgtgatttttctgcat
ggtaacgctgcctccagctacctgtggaggcacgtcgtgcctcacatcgagcccgtggctagatgcatcatccctg
atctgatcggaatgggtaagtccggcaagagcgggaatggctcatatcgcctcctggatcactacaagtacctcac
cgcttggttcgagctgctgaaccttccaaagaaaatcatctttgtgggccacgactgggggcttgtctggcctttca
ctactcctacgagcaccaagacaagatcaaggccatcgtccatgctgagagtgtcgtggacgtgatcgagtcctgg
gacgagtggcctgacatcgaggaggatatcgccctgatcaagagcgaagagggcgagaaaatggtgcttgaga
ataacttcttcgtcgagaccatgctcccaagcaagatcatgcggaaactggagcctgaggagttcgctgcctacctg
gagccattcaaggagaagggcgaggttagacggcctaccctctcctggcctcgcgagatccctctcgttaaggga
ggcaagcccgacgtcgtccagattgtccgcaactacaacgcctaccttcgggccagcgacgatctgcctaagatgt
tcatcgagtccgaccctgggttcttttccaacgctattgtcgagggagctaagaagttccctaacaccgagttcgtga
aggtgaagggcctccacttcagccaggaggacgctccagatgaaatgggtaagtacatcaagagcttcgtggagc
gcgtgctgaagaacgagcagaattctGCtTGCAAGAACTGGTTCAGtAGCtTaAGCCAC
TTtGTGATCCACCTtAACAGCCACGGCTTCCCtCCCGAGGTGGAGGAGC
AGGCCGCCGGCACCCTGCCCATGAGCTGCGCCCAGGAGAGCGGCATG
GAtaGaCACCCtGCtGCtTGCGCCAGCGCCAGGATCAACGTcTAg (SEQ ID
NO: 74).

An optimized *Renilla* luciferase-optimized CL1-optimized PEST-UTR sequence (hRluc-hCL1-hPEST-UTR) has the following sequence:

ATGGCTTCCAAGGTGTACGACCCCGAGCAACGCAAACGCATGATCAC
TGGGCCTCAGTGGTGGGCTCGCTGCAAGCAAATGAACGTGCTGGACT
CCTTCATCAACTACTATGATTCCGAGAAGCACGCCGAGAACGCCGTG
ATTTTTCTGCATGGTAACGCTGCCTCCAGCTACCTGTGGAGGCACGTC
GTGCCTCACATCGAGCCCGTGGCTAGATGCATCATCCCTGATCTGATC
GGAATGGGTAAGTCCGGCAAGAGCGGGAATGGCTCATATCGCCTCCT
GGATCACTACAAGTACCTCACCGCTTGGTTCGAGCTGCTGAACCTTCC
AAAGAAAATCATCTTTGTGGGCCACGACTGGGGGGCTTGTCTGGCCT
TTCACTACTCCTACGAGCACCAAGACAAGATCAAGGCCATCGTCCAT
GCTGAGAGTGTCGTGGACGTGATCGAGTCCTGGGACGAGTGGCCTGA
CATCGAGGAGGATATCGCCCTGATCAAGAGCGAAGAGGGCGAGAAA
ATGGTGCTTGAGAATAACTTCTTCGTCGAGACCATGCTCCCAAGCAA
GATCATGCGGAAACTGGAGCCTGAGGAGTTCGCTGCCTACCTGGAGC
CATTCAAGGAGAAGGGCGAGGTTAGACGGCCTACCCTCTCCTGGCCT
CGCGAGATCCCTCTCGTTAAGGGAGGCAAGCCCGACGTCGTCCAGAT
TGTCCGCAACTACAACGCCTACCTTCGGGCCAGCGACGATCTGCCTA
AGATGTTCATCGAGTCCGACCCTGGGTTCTTTTCCAACGCTATTGTCG
AGGGAGCTAAGAAGTTCCCTAACACCGAGTTCGTGAAGGTGAAGGG
CCTCCACTTCAGCCAGGAGGACGCTCCAGATGAAATGGGTAAGTACA
TCAAGAGCTTCGTGGAGCGCGTGCTGAAGAACGAGCAGAATTCTGCT
TGCAAGAACTGGTTCAGTAGCTTAAGCCACTTTGTGATCCACCTTAAC
AGCCACGGCTTCCCTCCCGAGGTGGAGGAGCAGGCCGCCGGCACCCT
GCCCATGAGCTGCGCCCAGGAGAGCGGCATGGATAGACACCCTGCTG

CTTGCGCCAGCGCCAGGATCAACGTCTAGGGCGCGGACTTTATTTATT
TATTTCTT (SEQ ID NO:75)

An optimized firefly luciferase-optimized CL1-optimized PEST-UTR sequence (hluc+-hCL1-hPEST-UTR) has the following sequence:

ATGGCCGATGCTAAGAACATTAAGAAGGGCCCTGCTCCCTTCTACCC
TCTGGAGGATGGCACCGCTGGCGAGCAGCTGCACAAGGCCATGAAG
AGGTATGCCCTGGTGCCTGGCACCATTGCCTTCACCGATGCCCACATT
GAGGTGGACATCACCTATGCCGAGTACTTCGAGATGTCTGTGCGCCT
GGCCGAGGCCATGAAGAGGTACGGCCTGAACACCAACCACCGCATC
GTGGTGTGCTCTGAGAACTCTCTGCAGTTCTTCATGCCAGTGCTGGGC
GCCCTGTTCATCGGAGTGGCCGTGGCCCCTGCTAACGACATTTACAA
CGAGCGCGAGCTGCTGAACAGCATGGGCATTTCTCAGCCTACCGTGG
TGTTCGTGTCTAAGAAGGGCCTGCAGAAGATCCTGAACGTGCAGAAG
AAGCTGCCTATCATCCAGAAGATCATCATCATGGACTCTAAGACCGA
CTACCAGGGCTTCCAGAGCATGTACACATTCGTGACATCTCATCTGCC
TCCTGGCTTCAACGAGTACGACTTCGTGCCAGAGTCTTTCGACAGGG
ACAAAACCATTGCCCTGATCATGAACAGCTCTGGGTCTACCGGCCTG
CCTAAGGGCGTGGCCCTGCCTCATCGCACCGCCTGTGTGCGCTTCTCT
CACGCCCGCGACCCTATTTTCGGCAACCAGATCATCCCCGACACCGC
TATTCTGAGCGTGGTGCCATTCCACCACGGCTTCGGCATGTTCACCAC
CCTGGGCTACCTGATTTGCGGCTTTCGGGTGGTGCTGATGTACCGCTT
CGAGGAGGAGCTGTTCCTGCGCAGCCTGCAAGACTACAAAATTCAGT
CTGCCCTGCTGGTGCCAACCCTGTTCAGCTTCTTCGCTAAGAGCACCC
TGATCGACAAGTACGACCTGTCTAACCTGCACGAGATTGCCTCTGGC
GGCGCCCCACTGTCTAAGGAGGTGGGCGAAGCCGTGGCCAAGCGCTT
TCATCTGCCAGGCATCCGCCAGGGCTACGGCCTGACCGAGACAACCA
GCGCCATTCTGATTACCCCAGAGGGCGACGACAAGCCTGGCGCCGTG
GGCAAGGTGGTGCCATTCTTCGAGGCCAAGGTGGTGGACCTGGACAC
CGGCAAGACCCTGGGAGTGAACCAGCGCGGCGAGCTGTGTGTGCGC
GGCCCTATGATTATGTCCGGCTACGTGAATAACCCTGAGGCCACAAA
CGCCCTGATCGACAAGGACGGCTGGCTGCACTCTGGCGACATTGCCT
ACTGGGACGAGGACGAGCACTTCTTCATCGTGGACCGCCTGAAGTCT
CTGATCAAGTACAAGGGCTACCAGGTGGCCCCAGCCGAGCTGGAGTC
TATCCTGCTGCAGCACCCTAACATTTTCGACGCCGGAGTGGCCGGCCT
GCCCGACGACGATGCCGGCGAGCTGCCTGCCGCCGTCGTCGTGCTGG
AACACGGCAAGACCATGACCGAGAAGGAGATCGTGGACTATGTGGC
CAGCCAGGTGACAACCGCCAAGAAGCTGCGCGGCGGAGTGGTGTTC
GTGGACGAGGTGCCCAAGGGCCTGACCGGCAAGCTGGACGCCCGCA
AGATCCGCGAGATCCTGATCAAGGCTAAGAAAGGCGGCAAGATCGC
CGTGAATTCTGCTTGCAAGAACTGGTTCAGTAGCTTAAGCCACTTTGT
GATCCACCTTAACAGCCACGGCTTCCCTCCCGAGGTGGAGGAGCAGG
CCGCCGGCACCCTGCCCATGAGCTGCGCCCAGGAGAGCGGCATGGAT
AGACACCCTGCTGCTTGCGCCAGCGCCAGGATCAACGTCTAGGGCGC
GGACTTTATTTATTTATTTCTT (SEQ ID NO:76)

Optimized click beetle sequences include:
CBRluc-hPEST 
ATGGTAAAGCGTGAGAAAAATGTCATCTATGGCCCTGAGCCTCTCCA
TCCTTTGGAGGATTTGACTGCCGGCGAAATGCTGTTTCGTGCTCTCCG
CAAGCACTCTCATTTGCCTCAAGCCTTGGTCGATGTGGTCGGCGATGA
ATCTTTGAGCTACAAGGAGTTTTTTGAGGCAACCGTCTTGCTGGCTCA
GTCCCTCCACAATTGTGGCTACAAGATGAACGACGTCGTTAGTATCT
GTGCTGAAAACAATACCCGTTTCTTCATTCCAGTCATCGCCGCATGGT
ATATCGGTATGATCGTGGCTCCAGTCAACGAGAGCTACATTCCCGAC
GAACTGTGTAAAGTCATGGGTATCTCTAAGCCACAGATTGTCTTCACC
ACTAAGAATATTCTGAACAAAGTCCTGGAAGTCCAAAGCCGCACCAA
CTTTATTAAGCGTATCATCATCTTGGACACTGTGGAGAATATTCACGG
TTGCGAATCTTTGCCTAATTTCATCTCTCGCTATTCAGACGGCAACAT
CGCAAACTTTAAACCACTCCACTTCGACCCTGTGGAACAAGTTGCAG
CCATTCTGTGTAGCAGCGGTACTACTGGACTCCCAAAGGGAGTCATG
CAGACCCATCAAAACATTTGCGTGCGTCTGATCCATGCTCTCGATCCA
CGCTACGGCACTCAGCTGATTCCTGGTGTCACCGTCTTGGTCTACTTG
CCTTTCTTCCATGCTTTCGGCTTTCATATTACTTTGGGTTACTTTATGG
TCGGTCTCCGCGTGATTATGTTCCGCCGTTTTGATCAGGAGGCTTTCT
TGAAAGCCATCCAAGATTATGAAGTCCGCAGTGTCATCAACGTGCCT
AGCGTGATCCTGTTTTTGTCTAAGAGCCCACTCGTGGACAAGTACGA
CTTGTCTTCACTGCGTGAATTGTGTTGCGGTGCCGCTCCACTGGCTAA
GGAGGTCGCTGAAGTGGCCGCCAAACGCTTGAATCTTCCAGGGATTC
GTTGTGGCTTCGGCCTCACCGAATCTACCAGTGCGATTATCCAGACTC
TCGGGGATGAGTTTAAGAGCGGCTCTTTGGGCCGTGTCACTCCACTC
ATGGCTGCTAAGATCGCTGATCGCGAAACTGGTAAGGCTTTGGGCCC
GAACCAAGTGGGCGAGCTGTGTATCAAAGGCCCTATGGTGAGCAAG
GGTTATGTCAATAACGTTGAAGCTACCAAGGAGGCCATCGACGACGA
CGGCTGGTTGCATTCTGGTGATTTTGGATATTACGACGAAGATGAGC
ATTTTTACGTCGTGGATCGTTACAAGGAGCTGATCAAATACAAGGGT
AGCCAGGTTGCTCCAGCTGAGTTGGAGGAGATTCTGTTGAAAAATCC
ATGCATTCGCGATGTCGCTGTGGTCGGCATTCCTGATCTGGAGGCCG
GCGAACTGCCTTCTGCTTTCGTTGTCAAGCAGCCTGGTACAGAAATTA
CCGCCAAAGAAGTGTATGATTACCTGGCTGAACGTGTGAGCCATACT
AAGTACTTGCGTGGCGGCGTGCGTTTTGTTGACTCCATCCCTCGTAAC
GTAACAGGCAAAATTACCCGCAAGGAGCTGTTGAAACAATTGTTGGT
GAAGGCCGGCGGGAATTCTCACGGCTTCCCTCCCGAGGTGGAGGAGC
AGGCCGCCGGCACCCTGCCCATGAGCTGCGCCCAGGAGAGCGGCATG
GATAGACACCCTGCTGCTTGCGCCAGCGCCAGGATCAACGTCTAA
(SEQ ID NO:77)
CBRluc-hCL1-hPEST-UTR 
ATGGTAAAGCGTGAGAAAAATGTCATCTATGGCCCTGAGCCTCTCCA
TCCTTTGGAGGATTTGACTGCCGGCGAAATGCTGTTTCGTGCTCTCCG
CAAGCACTCTCATTTGCCTCAAGCCTTGGTCGATGTGGTCGGCGATGA
ATCTTTGAGCTACAAGGAGTTTTTTGAGGCAACCGTCTTGCTGGCTCA
GTCCCTCCACAATTGTGGCTACAAGATGAACGACGTCGTTAGTATCT
GTGCTGAAAACAATACCCGTTTCTTCATTCCAGTCATCGCCGCATGGT
ATATCGGTATGATCGTGGCTCCAGTCAACGAGAGCTACATTCCCGAC
GAACTGTGTAAAGTCATGGGTATCTCTAAGCCACAGATTGTCTTCACC
ACTAAGAATATTCTGAACAAAGTCCTGGAAGTCCAAAGCCGCACCAA
CTTTATTAAGCGTATCATCATCTTGGACACTGTGGAGAATATTCACGG
 
TTGCGAATCTTTGCCTAATTTCATCTCTCGCTATTCAGACGGCAACAT
CGCAAACTTTAAACCACTCCACTTCGACCCTGTGGAACAAGTTGCAG
CCATTCTGTGTAGCAGCGGTACTACTGGACTCCCAAAGGAGTCATG
CAGACCCATCAAAACATTTGCGTGCGTCTGATCCATGCTCTCGATCCA
CGCTACGGCACTCAGCTGATTCCTGGTGTCACCGTCTTGGTCTACTTG
CCTTTCTTCCATGCTTTCGGCTTTCATATTACTTTGGGTTACTTTATGG
TCGGTCTCCGCGTGATTATGTTCCGCCGTTTTGATCAGGAGGCTTTCT
TGAAAGCCATCCAAGATTATGAAGTCCGCAGTGTCATCAACGTGCCT
AGCGTGATCCTGTTTTTGTCTAAGAGCCCACTCGTGGACAAGTACGA
CTTGTCTTCACTGCGTGAATTGTGTTGCGGTGCCGCTCCACTGGCTAA
GGAGGTCGCTGAAGTGGCCGCCAAACGCTTGAATCTTCCAGGGATTC
GTTGTGGCTTCGGCCTCACCGAATCTACCAGTGCGATTATCCAGACTC
TCGGGGATGAGTTTAAGAGCGGCTCTTTGGGCCGTGTCACTCCACTC
ATGGCTGCTAAGATCGCTGATCGCGAAACTGGTAAGGCTTTGGGCCC
GAACCAAGTGGGCGAGCTGTGTATCAAAGGCCCTATGGTGAGCAAG
GGTTATGTCAATAACGTTGAAGCTACCAAGGAGGCCATCGACGACGA
CGGCTGGTTGCATTCTGGTGATTTTGGATATTACGACGAAGATGAGC
ATTTTTACGTCGTGGATCGTTACAAGGAGCTGATCAAATACAAGGGT
AGCCAGGTTGCTCCAGCTGAGTTGGAGGAGATTCTGTTGAAAAATCC
ATGCATTCGCGATGTCGCTGTGGTCGGCATTCCTGATCTGGAGGCCG
GCGAACTGCCTTCTGCTTTCGTTGTCAAGCAGCCTGGTACAGAAATTA
CCGCCAAAGAAGTGTATGATTACCTGGCTGAACGTGTGAGCCATACT
AAGTACTTGCGTGGCGGCGTGCGTTTTGTTGACTCCATCCCTCGTAAC
GTAACAGGCAAAATTACCCGCAAGGAGCTGTTGAAACAATTGTTGGT
GAAGGCCGGCGGAATTCTGCTTGCAAGAACTGGTTCAGTAGCTTAA
GCCACTTTGTGATCCACCTTAACAGCCACGGCTTCCCTCCCGAGGTGG
AGGAGCAGGCCGCCGGCACCCTGCCCATGAGCTGCGCCCAGGAGAG
CGGCATGGATAGACACCCTGCTGCTTGCGCCAGCGCCAGGATCAACG
TCTAGGGCGCGGACTTTATTTATTTATTTCTT (SEQ ID NO:78)
CBG99luc-hPEST 
ATGGTGAAGCGTGAGAAAAATGTCATCTATGGCCCTGAGCCTCTCCA
TCCTTTGGAGGATTTGACTGCCGGCGAAATGCTGTTTCGTGCTCTCCG
CAAGCACTCTCATTTGCCTCAAGCCTTGGTCGATGTGGTCGGCGATGA
ATCTTTGAGCTACAAGGAGTTTTTTGAGGCAACCGTCTTGCTGGCTCA
GTCCCTCCACAATTGTGGCTACAAGATGAACGACGTCGTTAGTATCT
GTGCTGAAAACAATACCCGTTTCTTCATTCCAGTCATCGCCGCATGGT
ATATCGGTATGATCGTGGCTCCAGTCAACGAGAGCTACATTCCCGAC
GAACTGTGTAAAGTCATGGGTATCTCTAAGCCACAGATTGTCTTCACC
ACTAAGAATATTCTGAACAAAGTCCTGGAAGTCCAAAGCCGCACCAA
CTTTATTAAGCGTATCATCATCTTGGACACTGTGGAGAATATTCACGG
TTGCGAATCTTTGCCTAATTTCATCTCTCGCTATTCAGACGGCAACAT
CGCAAACTTTAAACCACTCCACTTCGACCCTGTGGAACAAGTTGCAG
CCATTCTGTGTAGCAGCGGTACTACTGGACTCCCAAAGGGAGTCATG
CAGACCCATCAAAACATTTGCGTGCGTCTGATCCATGCTCTCGATCCA
CGCGTGGGCACTCAGCTGATTCCTGGTGTCACCGTCTTGGTCTACTTG
CCTTTCTTCCATGCTTTCGGCTTTAGCATTACTTTGGGTTACTTTATGG
TCGGTCTCCGCGTGATTATGTTCCGCCGTTTTGATCAGGAGGCTTTCT
TGAAAGCCATCCAAGATTATGAAGTCCGCAGTGTCATCAACGTGCCT
AGCGTGATCCTGTTTTTGTCTAAGAGCCCACTCGTGGACAAGTACGA
 
CTTGTCTTCACTGCGTGAATTGTGTTGCGGTGCCGCTCCACTGGCTAA
GGAGGTCGCTGAAGTGGCCGCCAAACGCTTGAATCTTCCAGGGATTC
GTTGTGGCTTCGGCCTCACCGAATCTACCAGCGCTAACATTCACTCTC
TCGGGGATGAGTTTAAGAGCGGCTCTTTGGGCCGTGTCACTCCACTC
ATGGCTGCTAAGATCGCTGATCGCGAAACTGGTAAGGCTTTGGGCCC
GAACCAAGTGGGCGAGCTGTGTATCAAAGGCCCTATGGTGAGCAAG
GGTTATGTCAATAACGTTGAAGCTACCAAGGAGGCCATCGACGACGA
CGGCTGGTTGCATTCTGGTGATTTTGGATATTACGACGAAGATGAGC
ATTTTTACGTCGTGGATCGTTACAAGGAGCTGATCAAATACAAGGGT
AGCCAGGTTGCTCCAGCTGAGTTGGAGGAGATTCTGTTGAAAAATCC
ATGCATTCGCGATGTCGCTGTGGTCGGCATTCCTGATCTGGAGGCCG
GCGAACTGCCTTCTGCTTTCGTTGTCAAGCAGCCTGGTAAAGAAATTA
CCGCCAAAGAAGTGTATGATTACCTGGCTGAACGTGTGAGCCATACT
AAGTACTTGCGTGGCGGCGTGCGTTTTGTTGACTCCATCCCTCGTAAC
GTAACAGGCAAAATTACCCGCAAGGAGCTGTTGAAACAATTGTTGGA
GAAGGCCGGCGGGAATTCTCACGGCTTCCCTCCCGAGGTGGAGGAGC
AGGCCGCCGGCACCCTGCCCATGAGCTGCGCCCAGGAGAGCGGCATG
GATAGACACCCTGCTGCTTGCGCCAGCGCCAGGATCAACGTCTAA
(SEQ ID NO:79)
CBG99luc-hCL1-hPEST-UTR

ATGGTGAAGCGTGAGAAAAATGTCATCTATGGCCCTGAGCCTCTCCA
TCCTTTGGAGGATTTGACTGCCGGCGAAATGCTGTTTCGTGCTCTCCG
CAAGCACTCTCATTTGCCTCAAGCCTTGGTCGATGTGGTCGGCGATGA
ATCTTTGAGCTACAAGGAGTTTTTTGAGGCAACCGTCTTGCTGGCTCA
GTCCCTCCACAATTGTGGCTACAAGATGAACGACGTCGTTAGTATCT
GTGCTGAAAACAATACCCGTTTCTTCATTCCAGTCATCGCCGCATGGT
ATATCGGTATGATCGTGGCTCCAGTCAACGAGAGCTACATTCCCGAC
GAACTGTGTAAAGTCATGGGTATCTCTAAGCCACAGATTGTCTTCACC
ACTAAGAATATTCTGAACAAAGTCCTGGAAGTCCAAAGCCGCACCAA
CTTTATTAAGCGTATCATCATCTTGGACACTGTGGAGAATATTCACGG
TTGCGAATCTTTGCCTAATTTCATCTCTCGCTATTCAGACGGCAACAT
CGCAAACTTTAAACCACTCCACTTCGACCCTGTGGAACAAGTTGCAG
CCATTCTGTGTAGCAGCGGTACTACTGGACTCCCAAAGGGAGTCATG
CAGACCCATCAAAACATTTGCGTGCGTCTGATCCATGCTCTCGATCCA
CGCGTGGGCACTCAGCTGATTCCTGGTGTCACCGTCTTGGTCTACTTG
CCTTTCTTCCATGCTTTCGGCTTTAGCATTACTTTGGGTTACTTTATGG
TCGGTCTCCGCGTGATTATGTTCCGCCGTTTTGATCAGGAGGCTTTCT
TGAAAGCCATCCAAGATTATGAAGTCCGCAGTGTCATCAACGTGCCT
AGCGTGATCCTGTTTTTGTCTAAGAGCCCACTCGTGGACAAGTACGA
CTTGTCTTCACTGCGTGAATTGTGTTGCGGTGCCGCTCCACTGGCTAA
GGAGGTCGCTGAAGTGGCCGCCAAACGCTTGAATCTTCCAGGGATTC
GTTGTGGCTTCGGCCTCACCGAATCTACCAGCGCTAACATTCACTCTC
TCGGGGATGAGTTTAAGAGCGGCTCTTTGGGCCGTGTCACTCCACTC
ATGGCTGCTAAGATCGCTGATCGCGAAACTGGTAAGGCTTTGGGCCC
GAACCAAGTGGGCGAGCTGTGTATCAAAGGCCCTATGGTGAGCAAG
GGTTATGTCAATAACGTTGAAGCTACCAAGGAGGCCATCGACGACGA
CGGCTGGTTGCATTCTGGTGATTTTGGATATTACGACGAAGATGAGC
ATTTTTACGTCGTGGATCGTTACAAGGAGCTGATCAAATACAAGGGT
AGCCAGGTTGCTCCAGCTGAGTTGGAGGAGATTCTGTTGAAAAATCC
ATGCATTCGCGATGTCGCTGTGGTCGGCATTCCTGATCTGGAGGCCG
GCGAACTGCCTTCTGCTTTCGTTGTCAAGCAGCCTGGTAAAGAAATTA
CCGCCAAAGAAGTGTATGATTACCTGGCTGAACGTGTGAGCCATACT
AAGTACTTGCGTGGCGGCGTGCGTTTTGTTGACTCCATCCCTCGTAAC
GTAACAGGCAAAATTACCCGCAAGGAGCTGTTGAAACAATTGTTGGA
GAAGGCCGGCGGAATTCTGCTTGCAAGAACTGGTTCAGTAGCTTAA
GCCACTTTGTGATCCACCTTAACAGCCACGGCTTCCCTCCCGAGGTGG
AGGAGCAGGCCGCCGGCACCCTGCCCATGAGCTGCGCCCAGGAGAG
CGGCATGGATAGACACCCTGCTGCTTGCGCCAGCGCCAGGATCAACG
TCTAGGGCGCGGACTTTATTTATTTATTTCTT (SEQ ID NO:80)

**Table 1**

| Name of the primer | Sequence of the primer |
|---|---|
| LucN | 5'- AGATCTGCGATCTAAGTAAGCTTGG; SEQ ID NO:16 |
| LucC | 5'- ACTCTAGAATICACGGCGATCTTTCC; SEQ ID NO: 17 |
| HRLN | 5'-GGCGAAGCTTGGGTCACCTCCAAGGTGTACGACCCCGAGC; SEQ ID NO:18 |
| HRLC | 5'- GCTCTAGAATGAATTCTGCTCGTTCCAGCACGCGCT; SEQ ID NO:19 |
| PEST-5' | 5'-AATTCTCATGGCTTCCCGCCGGAGATGGAGGAGCAGGCT GCTGGCACGCTGCCCATGTCTT; SEQ ID NO:8 |
| PEST-3' | 5'-GTGCCCAGGAGAGCGGATGGACCGTCACCCTGCAGCCT GTGCTTCTGCTAGGATCAATGTGTAA; SEQ ID NO:9 |
| 5'-PEST | 5'-GGCCTTACACATTGATCCTAGCAGAAGCACAGGCTGCAG GGTGACGGTCCATCCCGCTCTCCT; SEQ ID NO:10 |
| 3'-PEST | 5'-GGGCACAAGACATGGGCAGCGTGCCAGCAGCCTGCTCCT CCATCTCCGGCGGGAAGCCATGAG; SEQ ID NO:11 |
| Ubiquitin 5'wt/BsytEII | 5'-TAGCATGGTCACCCAGATTTTCGTGAAAACCCTTACG; SEQ ID NO:20 |
| Ubiquitin 3'w/BstEII | 5'-ATGCTAGGTGACCGGATCCCGCGGATAACCACCA; SEQ ID NO:21 |
| | 5'-CCATGGGACATCATCACCATCACCACGGGGATCCACAAG CTTATGAAGAAATTAGCAA; SEQ ID NO:22 |
| Ubi-Luc 3' w/ Linker | 5'-TTCTGGATCCCGCGGTATACCACCACGAAGACTCAACAC; SEQ ID NO:23 |
| Ubi-Luc 3' w/ Linker Met | 5'-TTCTGGATCCCGCGGCATACCACCACGAAGACTCAACAC; SEQ ID NO:24 |
| Ubi-Luc3' w/Linker Glu | 5'-TTCTGGATCCCGCGGCTCACCACCACGAAGACTCAACAC; SEQ ID NO:25 |
| Ubi-Luc 5' w/Linker | 5'-TATGGGCCCTTAATACGACTCACTATAGGGGAATTGTGA GCGGATAACAATTCCCCTCTAGAAATAATTTTGTTTAACTTTA AGAAGGAGATATAC CATGCAGATTTTCGTGAAAACC; SEQ ID NO:26 |
| Ubiq(R) | 5'- TTTTGGCGTCGGTGACCGGATCCCGCGGTCGACCACCAC GAAG; SEQ ID NO:27 |
| Ala | 5'-TTTTGGCGTCGGTGACCGGATCCCGCGGTGCACCACCAC GAAG; SEQ ID NO:28 |
| Asn | 5'-TTTTGGCGTCGGTGACCGGATCCCGCGGGTTACCACCAC GAAG; SEQ ID NO:29 |
| Asp | 5'-TTTTGGCGTCGGTGACCGGATCCCGCGGATCACCACCAC GAAG; SEQ ID NO:30 |
| Phe | 5'-TTTTGGCGTCGGTGACCGGATCCCGCGGAAACCACCAC GAAG; SEQ ID NO:31 |
| His2 | 5'-TTTTGGCGTCGGTGACCGGATCCCGCGGATGACCACCAC GAAG; SEQ ID NO:32 |
| His | 5'- TTTTGGCGTCGGTGACCGGATCCCGCGGGTGACCACCAC GAAG; SEQ ID NO:33 |
| Leu | 5'-TTTTGGCGTCGGTGACCGGATCCCGCGGAGACCACCAC GAAG; SEQ ID NO:34 |
| Lys | 5'-TTTTGGCGTCGGTGACCGGATCCCGCGGCTTACCACCACG AAG; SEQ ID NO:35 |
| Gln | 5'-TTTTGGCGTCGGTGACCGGATCCCGCGGTTGACCACCAC GAAG; SEQ ID NO:36 |
| Trp | 5'-TTTTGGCGTCGGTGACCGGATCCCGCGGCCAACCACCAC GAAG; SEQ ID NO:37 |
| Ubiq(E)del5' | 5'- GTTTTTGGCGTCGGTGACCTCACCACCACGAAGACTC; SEQ ID NO:38 |
| Ubiq(E)del3' | 5'- GAGTCTTCGTGGTGGTGAGGTCACCGACGCCAAAAAC; SEQ ID NO:39 |
| Luc 5' | 5'-GTTCCAGGAACCAGGGCGTATCTC; SEQ ID NO:40 |
| Luc 3' | 5'-CGCGGAGGAGTTGTGTTTGTGGAC; SEQ ID NO:41 |
| Luc+N | 5'-GGCGAAGCTTGGGTCACCGATGCTAAGAACATTAAGAA GGG; SEQ ID NO:42 |
| Luc+C | 5'-GCTCTAGAATGAATTCACGGCGATCTTGCCGCC; SEQ ID NO:43 |
| tetO-5' | 5'-GATTAATGGCCCTTTCGTCCTTCGAGTT; SEQ ID NO:50 |
| tetO-3' | 5'-AGCTAGCGAGGCTGGATCGGTCCCGGT; SEQ ID NO:51 |
| AUUU | 5'- CTAGATTTATTTATTTATTTCTTCATATGC; SEQ ID NO:52 |
| Anti-AUUU | 5'- AATTGCATATGAAGAAATAAATAAATAAAT; SEQ ID NO:53 |
| hsp70-5' | 5'-ATTAATCTGATCAATAAAGGGTTTAAGG; SEQ ID NO:54 |
| hsp70-3' | 5'-AAAAAGGTAGTGGACTGTCG; SEQ ID NO:55 |
| 3'-BKB1 | 5'-AATTGGGAATTAAAACAGCATTGAACCAAGAAGCTTGGCT TTCTTATCAATTCTTTGTGACATAATAAGTT; SEQ ID NO:56 |
| 5'-BKB1rev | 5'-AACTTATTATGTCACAAAGAATTGATAAGAAAGCCAAGCT TCTTGGTTCAATGCTGTTTTAATTCCC; SEQ ID NO:57 |
| N-M | 5'-GATCTGCGGCCGCATATATG; SEQ ID NO:58 |
| M-C | 5'-GTGACCATATATGCGGCCGCA; SEQ ID NO:59 |
| CL1-RI-final | 5'-AATTTGTCTGCCTGCAAGAACTGGTTCAGCAGCTTGAGCCA CTTCGTGATCCACTTG; SEQ ID NO:60 |
| Rev-CL1-RI-final | 5'-AATTCAAGTGGATCACGAAGTGGCTCAAGCTGCTGAACCA GTTCTTGCAGGCAGACA; SEQ ID NO:61 |
| CL1 | 5'-AATTCTGCCTGCAAGAACTGGTTCAGCAGCTTGAGCCACTT CGTGATCCACTTGTAAGC; SEQ ID NO:62 |
| Rev-CL1 | 5'-GGCCGCTTACAAGTGGATCACGAAGTGGCTCAAGCTGCT GAACCAGTTCTTGCAGGCAG; SEQ ID NO:63 |
| CL1-N | 5'-GATCTTATGTCTGCCTGCAAGAACTGGTTCAGCAGCTTGAG CCACTTCGTGATCCACTTGCA; SEQ ID NO:64 |
| Rev-CL1-N | 5'-AGCTTGCAAGTGGATCACGAAGTGGCTCAAGCTGCTGAA CCAGTTCTTGCAGGCAGACATAA; SEQ ID NO:65 |
| AUUU | 5'-CTAGATTTATTTATTTATTTCTTCATATGC; SEQ ID NO :3 |
| Anti-AUUU | 5'-AATTGCATATGAAGAAATAAATAAATAAAT; SEQ ID NO :4 |

### DNA sequencing

Plasmid DNA sequences were confirmed by DNA sequencing which was performed on ABI Prizm Model 377 using either Luc5' or Luc3' primers (Table 1).

### Expression in vitro

Both TNT^{®} SP6 Coupled Wheat Germ Extract System and TNT^{®} T7 Coupled Reticulocyte Lysate System (Promega) were used to express firefly luciferase and fusion proteins thereof *in vitro.* [³H]-Leucine was included in the reaction mixture. Upon completion, the reaction mixtures were separated into two portions. The first portion was used to determine luciferase activity as described in the section entitled "Luciferase assay conditions" and the second portion was used to determine the quantity of synthesized luciferase. Proteins contained in the second portion were separated by SDS gel electrophoresis using 4-20% Tris-glycine gels (Novex). After completion of the electrophoresis, the location of the protein of interest on the gel was determined by autoradiography. Then bands containing proteins of interest were cut from the gel and the amounts of incorporated radioactivity determined by liquid scintillation. The ratio between luminescence data and the amount of radioactivity was used to characterize specific activity.

### Mammalian cells

Human adenocarcinoma cell line HeLa, African green monkey kidney cell line COS-7, Chinese hamster ovary cell line CHO-K1, and human embryonic kidney 293 cells were obtained from ATCC. All cell lines were maintained in RPMI-1640 medium containing 5% fetal bovine serum and a mixture of antibiotics (penicillin, 100 µg/ml; streptomycin, 100 µg/ml; amphotericin B, 0.25 µg/ml).

### Transient transfection and treatment with cycloheximide

For transfection, cells were grown to confluence in T25 flasks (Falkon, Becton Dickinson, Oxford). Transfection was conducted in 1 ml of serum-free RPMI-1640 that was mixed with 8 µg of plasmid DNA and 20 µl of Lipofectamine^{™}2000 (GIBCO BRL). CHO cells were incubated in the transfection media for 30 minutes, HeLa cells for 1 hour, and COS-7 cells for 5 hours. Following incubation, cells were trypsinized with Trypsin-EDTA (GIBCO BRL) and collected by centrifugation. Cells collected from each T25 flask were resuspended in 10 ml of RPMI-1640 medium containing 5% fetal bovine serum and a mixture of antibiotics, transferred to 96 well plates (100 µl/well), and allowed to grow for 12-16 hours prior to treatment with different agents. Cycloheximide (Sigma) or doxycycline was added to different wells at different times (the final concentration was 100 µg/ml and 2 µg/ml, respectively). For thermoinduction, cells were transferred to 42°C for 1 hour and then were incubated at 37°C for different periods. After incubation, plates with cells expressing derivatives of firefly luciferase were transferred to 70°C. In the case of cells expressing *Renilla* luciferase, the culture media was substituted with lysis buffer from the *Renilla* Luciferase Assay System (Promega). Luciferase assay conditions

To determine firefly luciferase activity, cells were lysed by freeze/thawing. Lysate from each well (100 µl) was transferred into corresponding wells of opaque 96 well plates (Falkon, Becton Dickinson, Oxford) containing 100 µl of Bright-Glo^{™} Luciferase Assay System (Promega) and luminescence intensity was determined using MLX-Microtiter Plate Luminometer (Dynex). The activity of *Renilla* luciferase was measured by mixing 40 µl samples with 100 µl of assay reagent from the *Renilla* Luciferase Assay System (Promega).

### Tet-Off system

A DNA fragment containing the CMV minimal promotor with heptamerized upstream tet-operators (Gossen et al., 1992) was amplified from plasmid pVHD10-3 by PCR with primers:
AGCTAGCGAGGCTGGATCGGTCCCGGT (SEQ ID NO:44) and GATTAATGGCCCTTTCGTCCTCGAGTT (SEQ ID NO:45). The amplified fragment was used to substitute the CMV promoter into *Renilla* luciferase encoding plasmids. HeLa cells were transfected with a mixture containing a *Renilla* luciferase encoding plasmid and plasmid pUHD15-1 in ratio of 4.5:1. Plasmid pUHD15-1 encodes a hybrid transactivator that contains the tetracycline repressor and the C-terminal domain of VP16 from HSV which stimulates minimal promoters fused to tetracycline operator sequences. In the presence of doxycycline, activity of the hybrid transactivator is inhibited.

### CRE system

D293 cells are an isolated subpopulation of 293 cells that produce a significant amount of cAMP upon induction. pGL-3 plasmids contain multiple CREs which respond to cAMP induction by increasing transcription. D293 cells were trypsin treated and 7.5 x10³ cells were added to wells of a 96-well plate. After an overnight incubation, the media from transfected cells was removed and replaced with media containing isoproterenol (Iso, Calbiochem #420355, final concentration 1 µM) and RO (Ro-20-1724, Calbiochem #557502, final concentration 100 µM). Iso induces the cAMP pathway and RO prevents degradation of cAMP. The plates were returned to the incubator. Samples from time points t = 0, 3, 6, 9 and 12 hours post-Iso/RO were collected. At 6 or 24 hours post-Iso/RO addition, prostaglandin E1 (PGE1, Calbiochem #538903, final concentration 1.0 µM) was added to the cells. Samples from time points 24, 27, 30, 33, and 36 hours post-Iso/RO addition (i.e., experimental start) were then collected.

For click beetle experiments, D293 cells were transiently transfected with codon optimized red (CBR) or green (CBG) click beetle sequences in conjunction with destabilization sequences. Two days post-transfection, the cells were treated with trypsin and 7.5 x 10³ cells were added to wells of a 96-well plate. After an overnight incubation, the media from transfected cells was removed and replaced with media containing isoproterenol. Samples from time points t = 0, 1.5, 3, 4.5, 6, and 7.5 hours post-induction were collected.

To determine the relative light units (RLU) for the samples, 20 µl of passive lysis buffer was added to each well, the Dual Luciferase Assay was performed, and the RLU were collected. In order to determine the fold-induction, the RLU from the drug treated wells were divided by RLU from non-treated wells.

### Stable Cell Lines

D293 cells were transfected with plasmids and then grown in media containing 600 µg/ml of G418. Individual lines of stably transfected cells were generated by seeding individual cells from the population grown in the G418-containing media into wells of a 96-well plate and growing the seeded cells in the G418-containing media.

### Results

### Construction and analysis of deubiqutination of ubiquitin-firefly luciferase fusions

To create luciferase species that would have at their N-termini amino acid residues of choice, the approach described earlier by Varshavsky and coauthors was used (Bachmair et al., 1986). This approach utilizes the ability of ubiquitin-specific processing proteases in cells to cleave fusion proteins containing ubiquitin at the N-terminus. Such cleavage occurs immediately after the last amino acid residue of ubiquitin. According to Bachmair et al. (1986) such deubiquitination occurs irrespective of the identity of the residue located immediately after the cleavage site.

Plasmids pUbiq(Y)Luc 19 and pSPUbiqLuc1 encode ubiquitin-firefly luciferase fusion proteins containing a tyrosine residue immediately after the ubiquitin sequence. Plasmid pUbiq(Y)Luc19 was designed to be expressed in mammalian cells and possesses an early promoter of CMV upstream and an SV40 polyadenylation signal downstream of the sequence encoding the fusion protein. Plasmid pSPUbiqLuc1 encodes the same protein as pUbiq(Y)Luc19 but possesses a promoter recognized by the DNA polymerase of bacteriophage SP6. Therefore, plasmid pSPUbiqLuc1 can be used for *in vitro* production of mRNA encoding the fusion protein. Both of these plasmids were used to confirm that in eukaryotic cells, and in mammalian cells specifically, ubiquitin-firefly luciferase fusion proteins undergo deubiquitination.

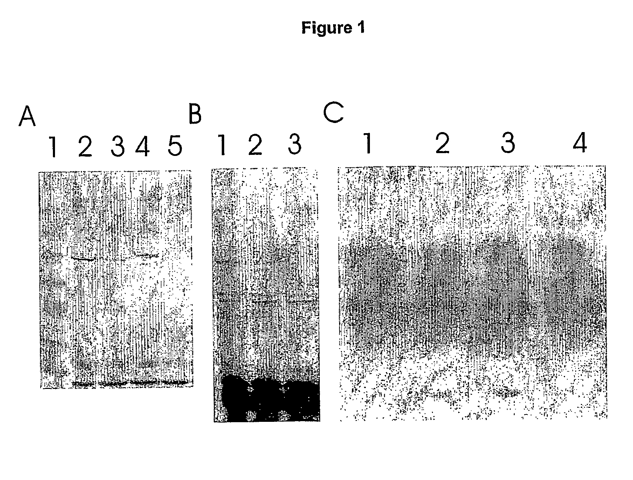

As shown in Figure 1, both in a wheat germ based *in vitro* translation system as well as in transiently transfected mammalian cells, expression of recombinant genes encoding ubiquitin-firefly luciferase fusion proteins resulted in accumulation of proteins that had molecular masses expected for wild-type firefly luciferase. The control wild-type firefly luciferase in these experiments was encoded either by plasmid pETUbiqLuc or pwtLuc1. In addition to the major band, a minor band was also present on the autoradiograph of proteins generated in the wheat germ based system. This minor band corresponds to the full-size recombinant protein that was not deubiquitinated. In several experiments performed using CHO cells, the minor band was either much weaker than in the wheat germ based system or was not detectable at all, suggesting that within mammalian cells, deubiquitination of luciferase happens very quickly and efficiently.

### Comparison of specific activities of wild type luciferase and its derivatives

Sung et al. (1995) reported that modifications of the firefly luciferase N-terminal region could interfere with enzymatic activity. Thus, luciferase species generated as a result of deubiquitination process might have different enzymatic activities than that of wild-type luciferase. To assess specific activities of luciferase fusion proteins and compare them with the specific activity of wild-type luciferase, plasmids pT7Ubiq(Y)Luc19.2 and pT7 Ubiq(E)Luc19.1 were constructed so that, in addition to an eukaryotic promoter, they have a promoter of bacteriophage T7. As a result, these plasmids direct the synthesis of luciferase fusion proteins in an *in vitro* transcription/translation system. Plasmid pT7Ubiq(Y)Luc19.2 encodes exactly the same protein as that encoded by plasmid pUbiq(Y)Luc19. Plasmid pT7 Ubiq(E)Luc19.1 encodes a ubiquitin-firefly luciferase fusion protein that differs from the protein encoded by plasmid pT7Ubiq(Y)Luc19.2 in only one position. In this position, located immediately after the last amino acid residue of ubiquitin, the protein encoded by plasmid pT7Ubiq(E)Luc19.1 has a glutamic acid residue in place of a tyrosine residue. Additionally, plasmids pETwtLuc1 and pT7Luc-PEST10 were constructed that halve a promoter of bacteriophage T7 and encode wild-type firefly luciferase or a fusion protein comprising firefly luciferase and a mutant form of C-ODC, respectively.

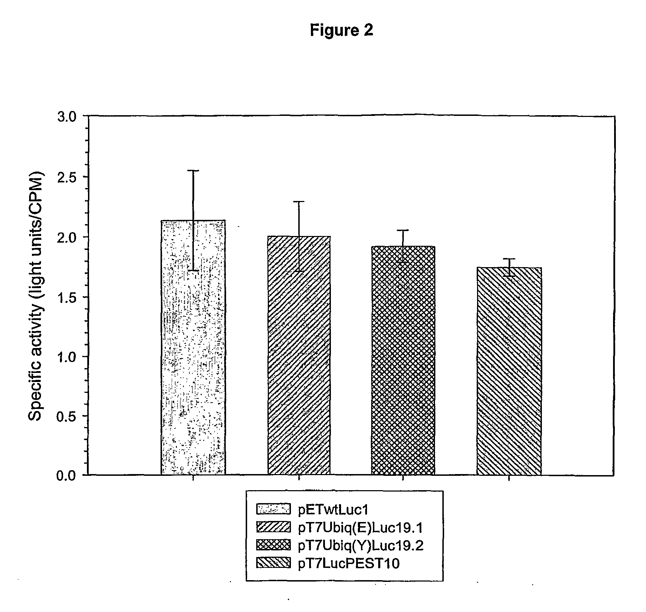

Plasmids encoding wild-type luciferase as well as a luciferase fusion protein were used in a rabbit reticulocyte *in vitro* transcription/translation system to determine luciferase activities accumulated in each reaction mixture and normalize these activities by the amount of radioactive leucine incorporated in corresponding luciferase species. Data presented in Figure 1 (panel C) demonstrate that, similarly to that found in CHO cells, only deubiquitinated forms of luciferase were accumulated in rabbit reticulocyte *in vitro* transcription/translation systems supplemented with either plasmid pT7Ubiq(Y)Luc19.2 or pT7Ubiq(E)Luc19.1. The presence in these mixtures of small proteins with electrophoretic mobilities of free ubiquitin evidences that full-size ubiquitin-luciferase fusions were produced in these reactions and were efficiently deubiquitinated. On the contrary, in the case of plasmid pT7Luc-PEST10, accumulation of the full-size protein was observed. Nevertheless, the data presented in Figure 2 demonstrate that both deubiquitinated forms of luciferase have essentially the same specific activity as that of wild-type luciferase and the protein encoded by plasmid pT7Luc-PEST10.

### Comparison of efficiencies of luciferase destabilization by N-degron and a mutant of the C-terminus of m-ODC

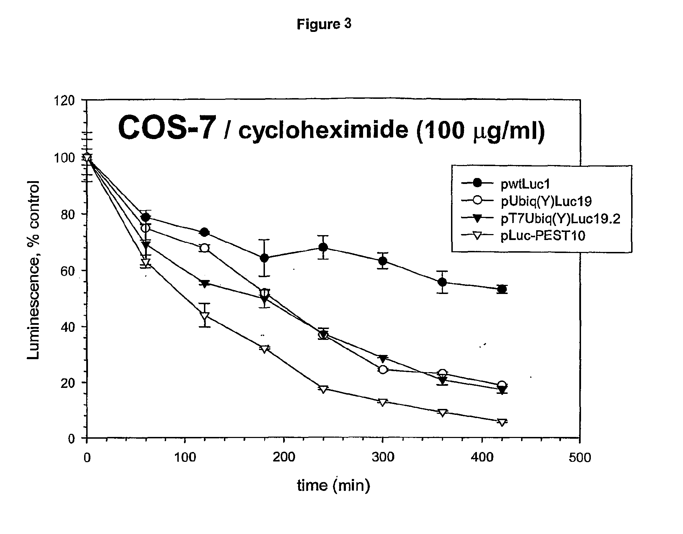

The half-life of the protein encoded by plasmid pUbiq(Y)Luc19 was determined in mammalian cells and compared to the half-lives of wild-type luciferase as well as fusion proteins comprising firefly luciferase and a mutant form of the C-ODC (Luc-PEST10). This was done by evaluating the luminescence emitted by cells that were transiently transfected with either plasmid pUbiq(Y)Luc19 or pwtLuc1 or pLuc-PEST10, respectively, and then, for different periods of time, were exposed to the protein synthesis inhibitor cycloheximide. In this experiment, cycloheximide was used at a concentration that caused complete inhibition of protein synthesis within 7.5 minutes of exposure (100 µg/ml). Results presented in Figure 3 demonstrate that in COS-7 cells, the Ubiq(Y)Luc fusion protein has an intermediate half-life compared to that of wild-type luciferase and a Luc-PEST fusion protein. The estimated half life of Ubiq(Y)Luc in these cells is about 170-200 minutes, which is far longer than the half-lives reported by Varshavsky (1992) for β-galactosidase containing a tyrosine residue at the N-terminus both in *E*. *coli* and *S. cerevisiae* (2 and 10 minutes, respectively). At the same time, the half-life of wild-type luciferase in COS-7 cells (about 7 hours) was shorter than the half-life of the wild-type β-galactosidase both in E. *coli* and in *S*. *cerevisiae* (more than 10 and 20 hours, respectively), suggesting that in COS-7 cells, N-degron initiated degradation occurs less efficiently than in yeast and bacteria. The fact that the half-life of Luc-PEST in these cells (about 120 minutes) is shorter than the half-life of Ubiq(Y)Luc suggests that the protein degrading capacity of the proteosome is not a limiting parameter for determining the half-life of Ubiq(Y)Luc. When the same analysis was performed using either CHO or HeLa cells, it was found that degradation of all three proteins in these cells occurs slightly faster than in COS-7 cells (see Figure 7).

### Analysis of the N-end rule in different cell lines

To understand to what degree in mammalian cells the N-end rule might differ from the same rule in yeast and *E*. *coli,* a set of plasmids was created that encode an ubiquitin-firefly luciferase fusion protein, namely pUbiq(Y)Luc19, pT7Ubiq(Y)Luc19.2 and pT7Ubiq(E)Luc19.1 (for a list of plasmids see Table 2). Proteins encoded by these plasmids have only one difference in their sequences; the amino acid residue located immediately after the ubiquitin sequence. Therefore, upon deubiquitination, these proteins should generate firefly luciferase species that are different only in the first N-terminal amino acid residue. Plasmids by themselves have additional differences in the region located upstream of the fusion protein coding region. Some of these plasmids have an additional bacteriophage T7 promoter (plasmids designated pT7Ubiq(X)Luc). Nevertheless, as shown in Figure 3 (see curves for plasmids pT7Ubiq(Y)Luc19.2 and pUbiq(Y)Luc19), the presence or the absence of bacteriophage T7 promoter had no effect on the stability of corresponding proteins.

**Table 2.**

| Plasmid | Amino acid residue following immediately after ubiquitin sequence |
|---|---|
| PT7Ubiq(M)Luc19.2 | Met |
| PT7Ubiq(E)Luc19.1 | Glu |
| PT7Ubiq(I)Luc19.1 | Ile |
| pUbiq(R)Luc13 | Arg |
| pUbiq(F)Luc10 | Phe |
| pUbiq(A)Luc2 | Ala |
| pUbiq(N)Luc25 | Asn |
| pUbiq(W)Luc16 | Trp |
| pUbiq(L)Luc23 | Leu |
| pUbiq(Q)Luc36 | Gln |
| pUbiq(Y)Luc19 | Tyr |
| pUbiq(Asp3)Luc16 | Asp |
| pUbiq(His2)Luc3 | His |
| pUbiq(K)Luc4 | Lys |

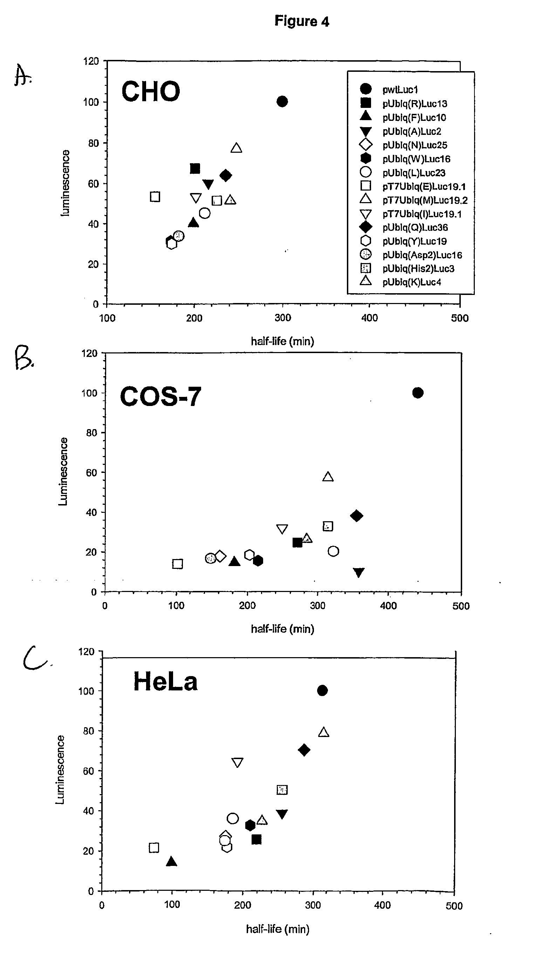

The stabilities of proteins encoded by plasmids listed in Table 2 were analyzed using cell lines derived from three different species, hamster (CHO), monkey (COS-7) and human (HeLa). It was observed that with one exception, Ubiq(M)Luc in HeLa cells, all ubiquitin containing firefly luciferase fusion proteins were degraded faster than wild-type luciferase (Figure 4). Patterns of relationships between amino acid residues positioned at the N-terminus of luciferase and the half-life of the corresponding protein remained essentially unchanged from experiment to experiment. For all three cell lines, a glutamic acid residue was found to be the most efficient destabilizing residue among all residues tested. Aspartic acid was also found to be a quite efficient destabilizing residue. At the same time, in all three cell lines, basic amino acid residues were found to have relatively weak destabilizing properties. In CHO cells, the difference between the half-lives of the most stable and the least stable constructs was almost two times smaller than the same differences determined in COS-7 and HeLa cells. Thus, in different cell lines, the N-end rule might have a different role in determining the fate of the protein.

In general, a correlation was observed between the half-life of the specific protein and the luminescence of cells producing this protein. Indeed, in Figure 4, most of the symbols characterizing the relationship between luminescence intensities and half-lives of firefly luciferase fusion protein tend to locate along straight lines. Nevertheless, in some cases such a correlation was not observed. For example, in COS-7 cells, the protein encoded by plasmid pUbiq(A)Luc2 produced much less luminescence than would be expected based on its half-life in these cells. Moreover, when the same protein was produced in CHO or HeLa cells, a dramatic deviation from the general rule was not observed. The potential role of inconsistencies in transfection efficiency in generating this phenomena was addressed by introducing, in addition to the plasmid encoding the firefly luciferase fusion protein, the same amount of plasmid encoding *Renilla* luciferase to each transfection mixture. *Renilla* luminescence in the transfected cells was used as a measure of transfection efficiency and to normalize firefly luminescence produced by the same cells. Normalization did not eliminate the observed inconsistencies, suggesting that such inconsistencies might reflect biological characteristics of the specific protein-cell pairs.

### Dependence of the N-end rule on the protein structure

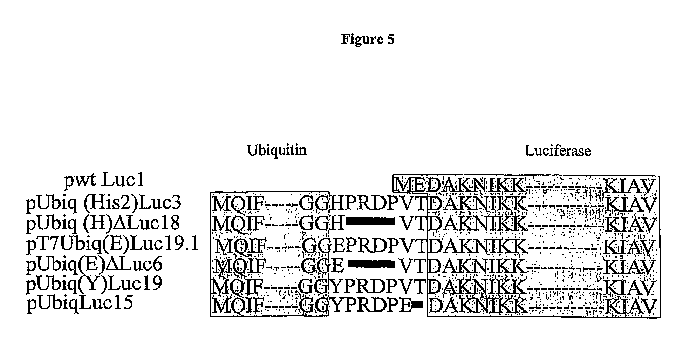

To understand dominance of the N-terminal residue in determining the fate of the protein within mammalian cells, the stabilities of luciferase fusion proteins having the same amino acid residue at the N-terminus, but different peptide sequences attached to the firefly luciferase, were compared. As shown in Figure 5, proteins encoded by plasmids pUbiq(E)ΔLuc6 and pUbiq(H)ΔLuc18 have four amino acid deletions when compared to proteins encoded by plasmids pT7Ubiq(E)Luc19.1 and pUbiq(His2)Luc3, respectively. In the protein encoded by plasmid pUbiqLuc15, one glutamic acid residue substitutes for residues found in the protein encoded by pUbiq(Y)Luc19.

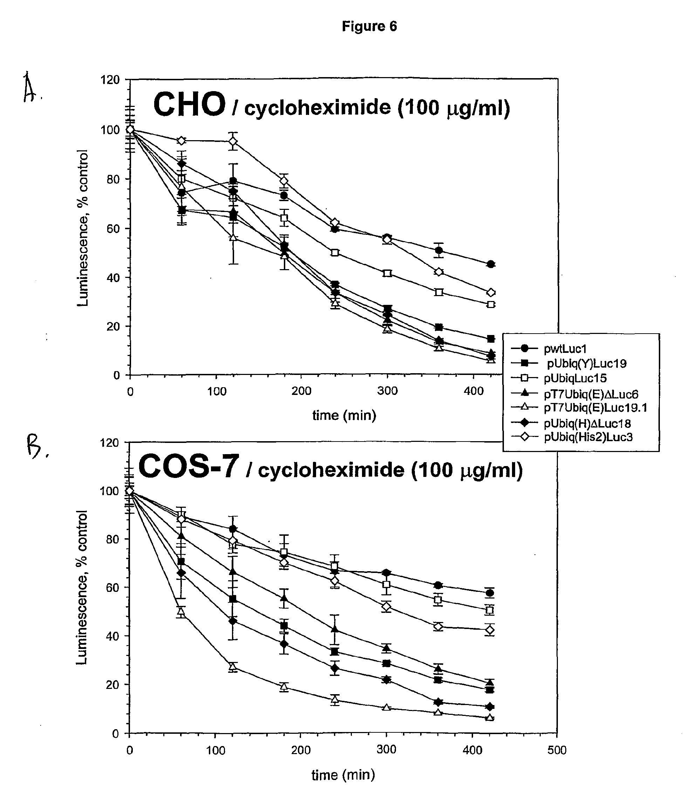

Data presented in Figure 6 reveal that proteins with the same N-terminal residue could have dramatically different half-lives depending on the structure of the following sequence. For example, in COS-7 cells, the half-life of the protein encoded by plasmid pUbiq(Y)Luc19 was about 140 minutes while the half-life of the protein encoded by plasmid pUbiqLuc15 was almost 7 hours. Depending on the cells used for the experiment, this difference could be less dramatic. Indeed, the same proteins when analyzed in CHO cells had half-lives of about 190 minutes and about 240 minutes, respectively. Surprisingly, depending on the amino acid residue located at the N-terminus, the effect of changes in the following sequence could be different. Indeed, deletion of four amino acid residues in a protein that has a histidine residue at the N-terminus resulted in destabilization of the protein (in COS-7 cells the half-life was reduced from about 300 minutes to about 120 minutes and in CHO cells, from about 320 minutes to about 200 minutes). At the same time, when the N-terminal position was occupied by an aspartic acid residue, the same deletion resulted in stabilization of the protein in COS-7 cells (the half-life changed from about 60 minutes to about 200 minutes) and had essentially no effect on the stability of the corresponding protein in CHO cells. Thus, even though the N-terminal residue plays an important role in the determining protein stability, it is not the dominant factor.

### Optimization of the reporter properties of firefly luciferase

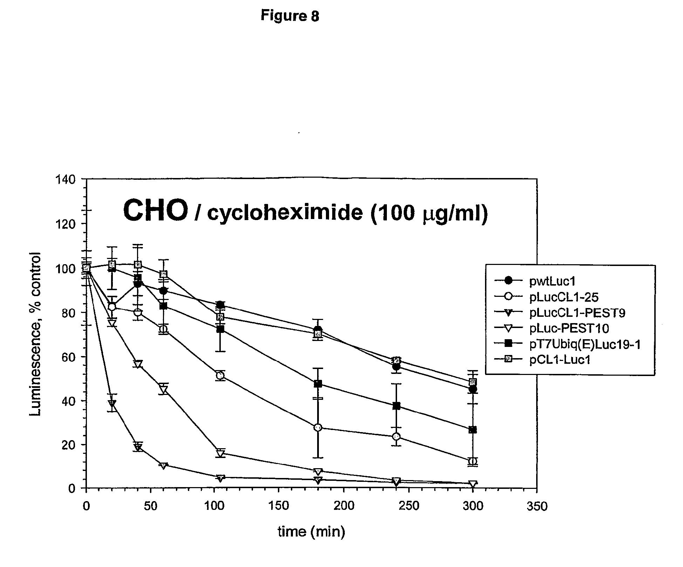

It had been reported that degradation of ODC occurs in the 26S proteosome without prior ubiquitination (Bercovich et al., 1989; Murakami et al., 1992), while degradation directed by N-degron was reported to be a ubiquitin-dependent process. To determine whether the combination of N-degron and C-ODC in the same protein might direct the recombinant protein towards degradation in the proteosome by two different pathways, thus decreasing the half-life of the protein even further, three plasmids, pUbiq(Y)Luc-PEST5, pUbiq(R)Luc-PEST12 and pT7Ubiq(E)Luc-PEST23, were constructed. Sequences of proteins encoded by these plasmids are different only in the position that follows immediately after the last amino acid residue of the ubiquitin sequence. In that position, the protein encoded by plasmid pUbiq(Y)Luc-PEST5 has a tyrosine, the protein encoded by plasmid pUbiq(R)Luc-PEST12 has an arginine, and the protein encoded by plasmid pT7Ubiq(E)Luc-PEST23 has a glutamic acid residue. The stabilities of proteins encoded by these plasmids were tested in HeLa (Figure 7), COS-7 and CHO cells. The analysis revealed that in all cell lines, proteins with two degradation signals were less stable than proteins that contained only one signal. For example, in HeLa cells, ubiquitin-luciferase fusion proteins Ubiq(E)Luc or Luc-PEST10 had half-lives of 70 and 65 minutes, respectively (Figure 4 and Figure 7). At the same time, the half-lives of proteins encoded by plasmids pUbiq(Y)Luc-PEST5, pUbiq(R)Luc-PEST12 and pT7Ubiq(E)Luc-PEST23 were 55, 40 and 30 minutes, respectively. Additionally, a combination of two degradation signals in the same protein resulted in more consistent degradation from cell line to cell line. For example, when three different cell lines were used to determine the half-life of the protein Ubiq(E)Luc that contains only the N-degron, the value varied from 70 to 150 minutes (Figure 4). When the same cell lines were used to determine the half-life of the protein encoded by plasmid pT7Ubiq(E)Luc-PEST23, the half-life was 30 minutes for HeLa cells (Figure 7), 45 minutes for COS-7 cells and 40 minutes for CHO cells. Luminescence data from cells transfected with plasmids encoding luciferase with another combination of protein destabilization sequences are shown in Figure 8. The presence of CL-1 and PEST in a luciferase fusion protein resulted in a protein that had a reduced half-life relative to a luciferase fusion protein with either CL-1 or a PEST sequence.

To determine whether optimized codon sequences can enhance the amount of light emitted in cells transfected with a plasmid encoding a luciferase fusion protein, plasmid pT7Ubiq(E)hLuc+PEST80 was constructed, which encodes the same protein as that encoded by plasmid pT7Ubiq(E)Luc-PEST23, except that it contains a luciferase encoding sequence that has been optimized for expression in human cells. As a result, translation of the fusion protein proceeds more efficiently when this protein is encoded by plasmid pT7Ubiq(E)hLuc+PEST80 rather than by plasmid pT7Ubiq(E)Luc-PEST23. As shown in Figure 7, the luminescence of cells transfected with plasmid pT7Ubiq(E)hLuc+PEST80 becomes comparable to that of cells producing wild-type firefly luciferase. Therefore, codon optimized sequences compensate for loss of expression when incorporated with a destabilization sequence as compared to a destablilization sequence in conjunction with a non-optimized codon sequence.

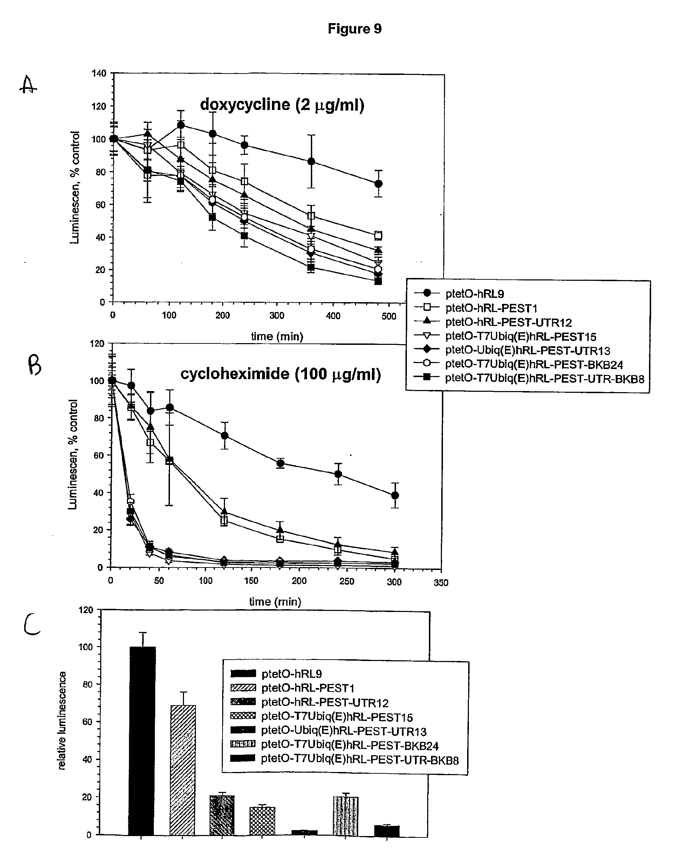

To determine whether a mRNA destabilization sequence in the mRNA for a fusion polypeptide comprising a luciferase and a protein destabilization sequence could further decrease the half-life of expression of a luciferase encoded by an optimized sequence, plasmids with promoters linked to optimized *Renilla* luciferase sequences and various combinations of destabilization sequences were tested (Figure 9). Generally, the greater the number of destabilization sequences, the shorter the half-life of expression of the encoded protein.

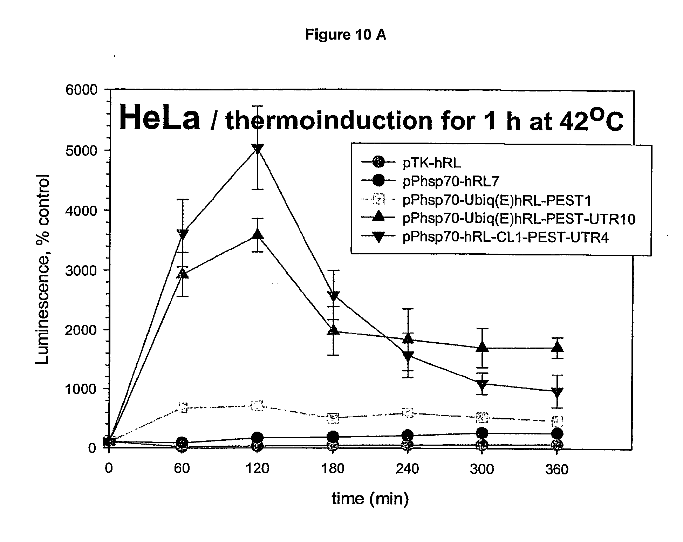

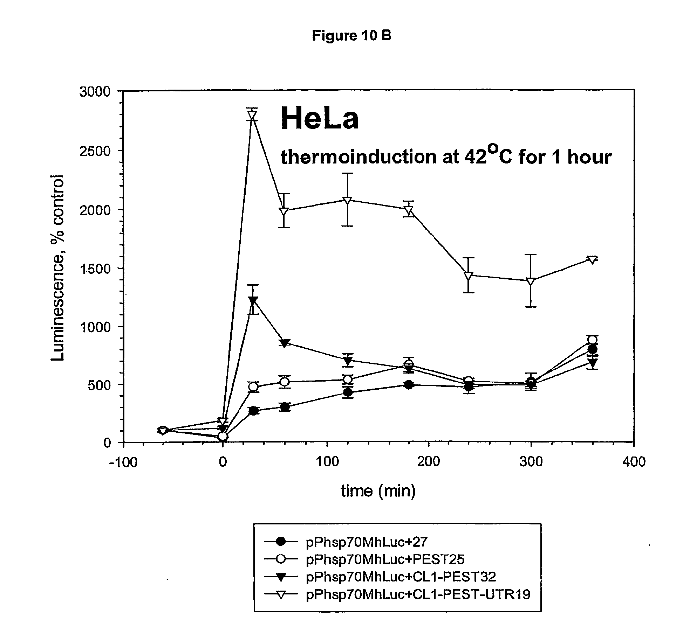

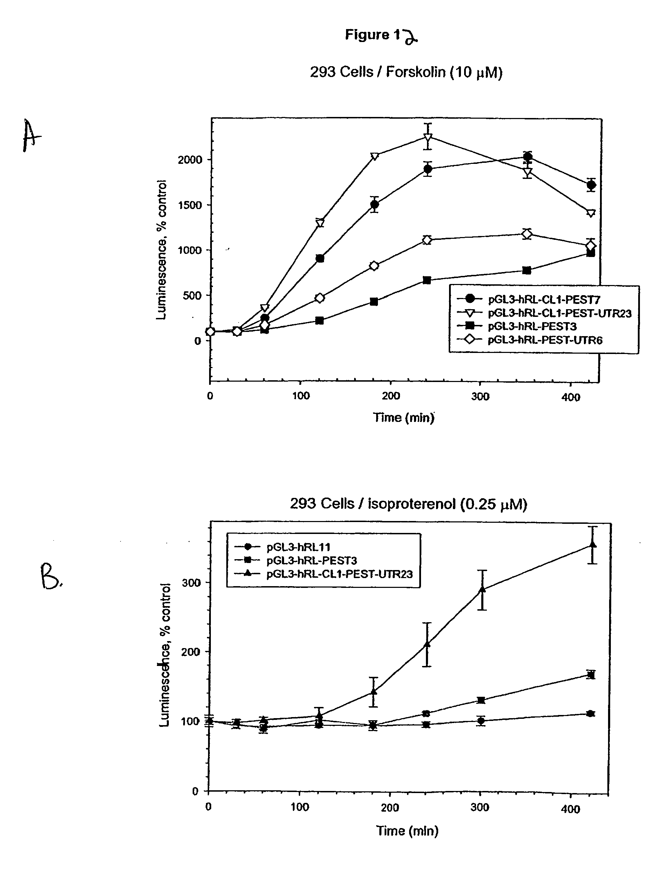

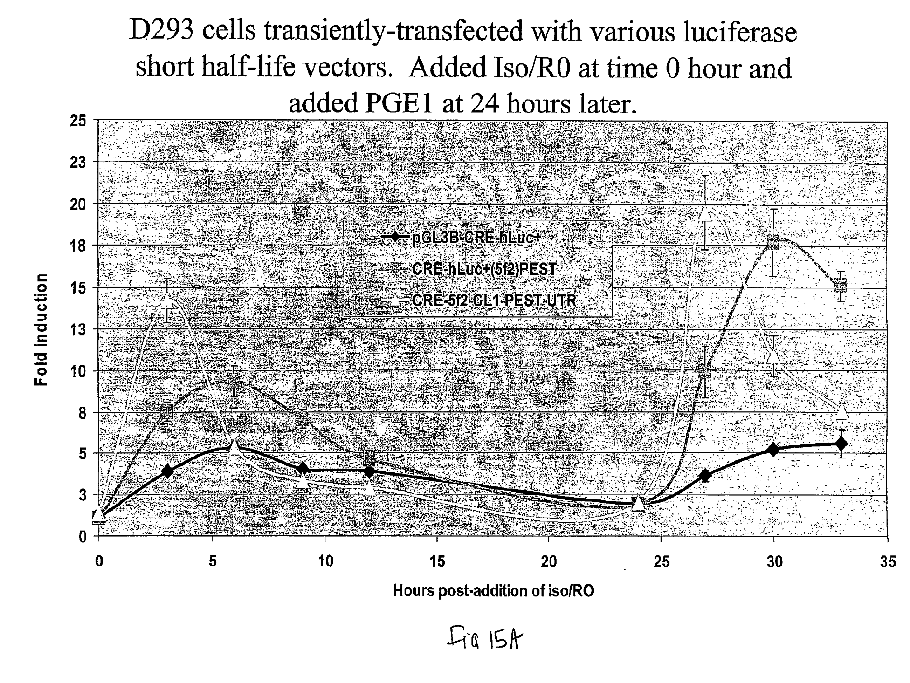

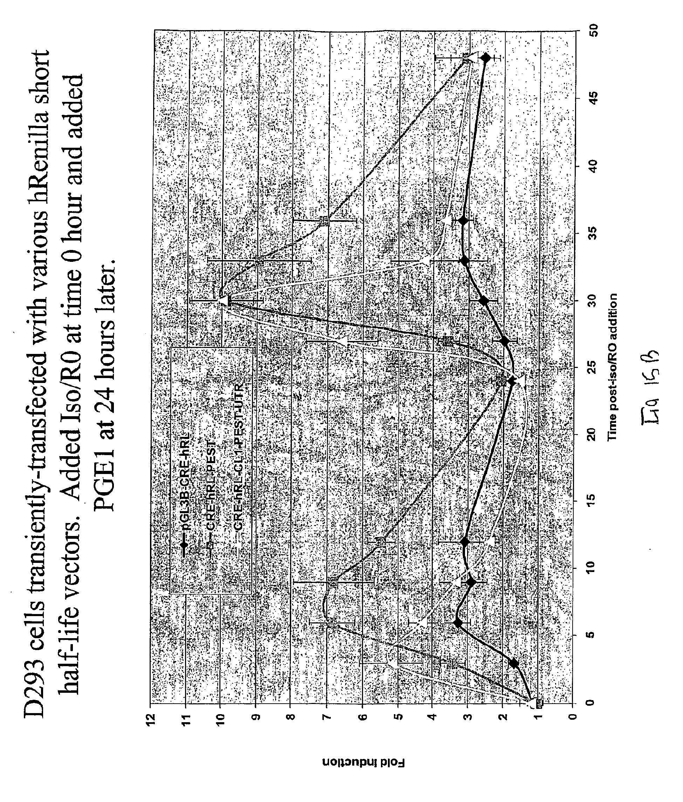

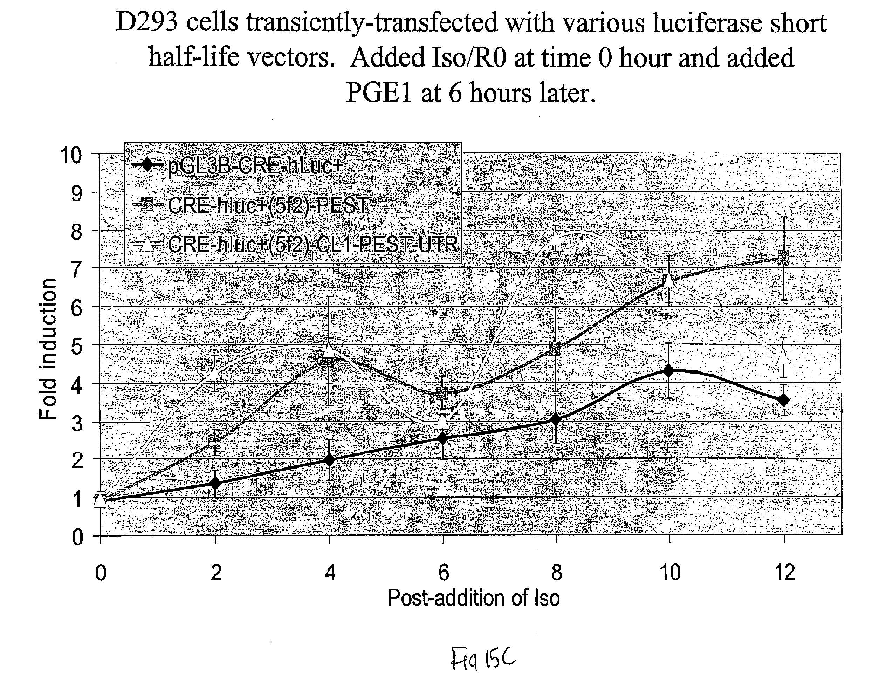

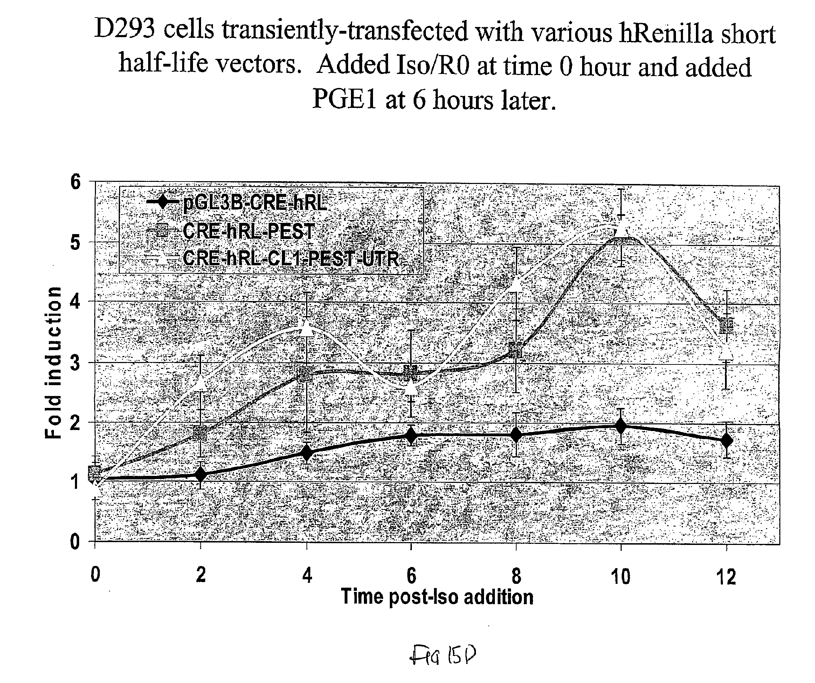

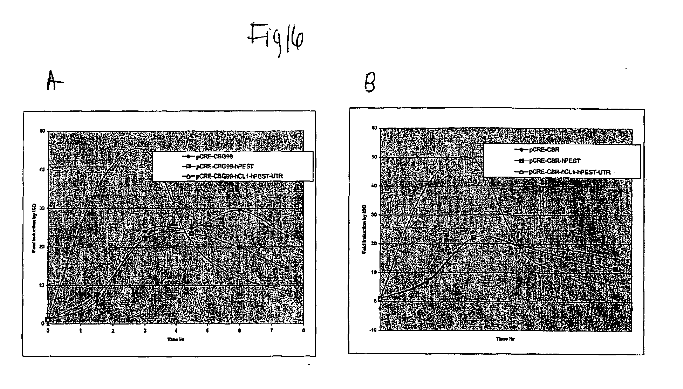

Figures 10 and 12-16 show luminescence after the induction of expression of optimized *Renilla,* firefly or click beetle luciferase sequences from plasmids having various combinations of destabilization sequences. Plasmids with more destabilization sequences generally had better response profiles than those with no or fewer destabilization sequences, i.e., destabilized reporters respond faster and their relative activation is higher than that of more stable derivatives.

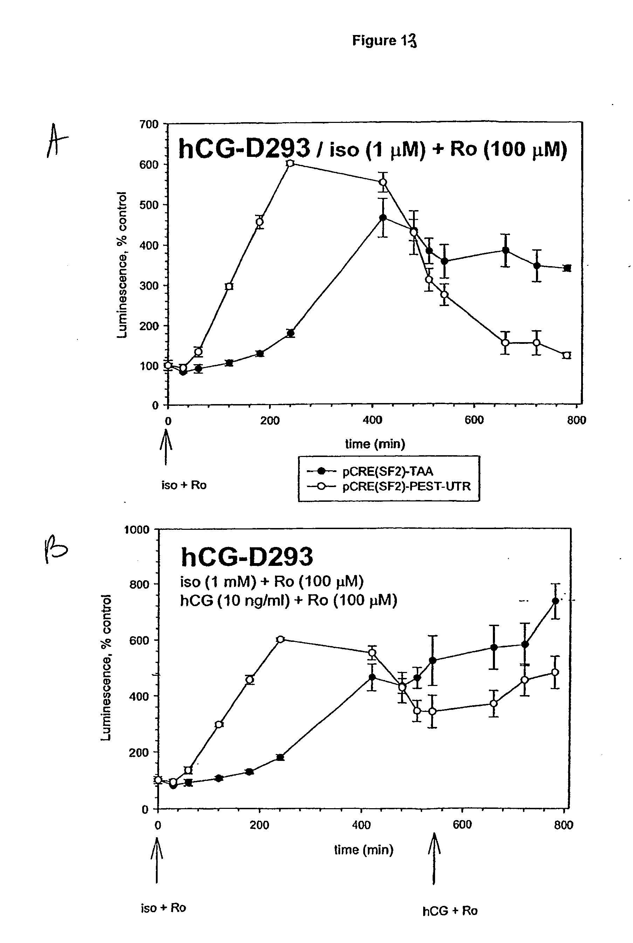

Figure 13 demonstrates that reporters can respond to two subsequent stimuli and that destabilized reporters are more suitable than stable reporters for detection of subsequent stimuli (when two stimuli occur in a relative short period of time) because a stable reporter does not have time to react. In the bottom graph of Figure 13, the curve corresponding to the stable version of optimized firefly luciferase continues to increase. At the same time, the curve corresponding to the destabilized protein, after reaching a maximum, begins to decrease, and only after the addition of hCG begins to increase again.

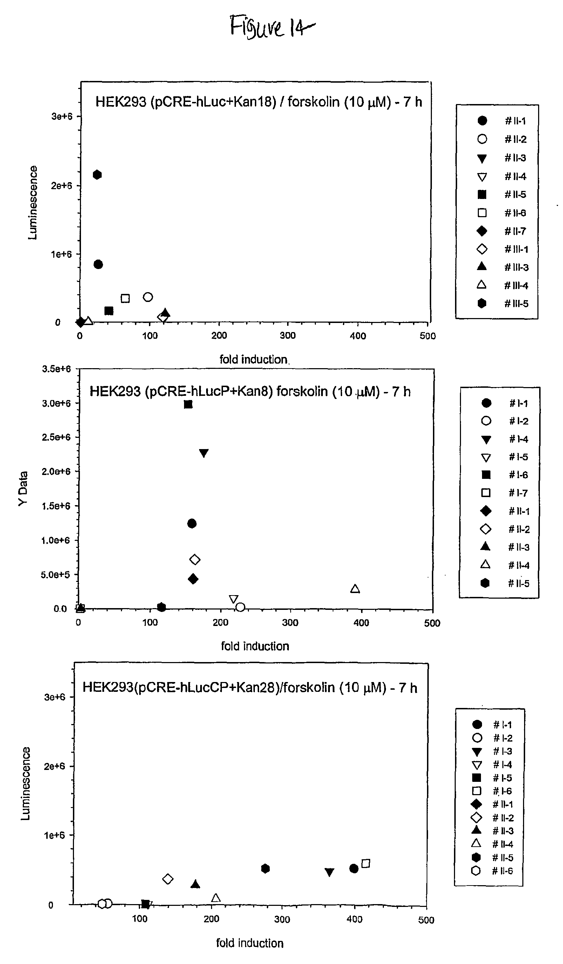

To determine whether destabilized reporter proteins in stably transfected cell lines could be detected, D293 cells were transfected with plasmids containing luciferase encoding sequences under the control of a cAMP regulated promoter. Plasmid pCRE-hLuc+Kan18 encodes a stable version of firefly luciferase, plasmid pCRE-hLucP+Kan8 encodes a luciferase fusion that has a PEST sequence at the C-terminus, and plasmid pCRE-hLucCP+Kan28 encodes a firefly luciferase fusion polypeptide that has CL1-PEST sequences as well as mRNA that has a mRNA destabilization sequence (UTR). G418-resistant clones were treated for 7 hours with 10 µM of forskolin or incubated for the same period of time in forskolin-free media. After the completion of the incubation period, luminescence was determined using Bright-Glo reagent (Figure 14). Stable clones with destabilized constructs were generally as bright as stable clones with a nondestabilized construct.

### Discussion

Varshavsky and coauthors determined that both in yeast and *E*. *coli* cells there is a definite correlation between the identity of the N-terminal residue and the half-life of the corresponding protein (the N-end rule). These findings suggested that the residue located at the N-terminus of the protein might play an important role in determining the fate of the protein inside bacterial and yeast cells. The data herein demonstrate that in different mammalian cells, depending on the identity of the N-terminal residue, the half-life of firefly luciferase fusion proteins might vary from 420 to 70 minutes, suggesting that the N-end rule functions in mammalian cells. Nevertheless, the N-end rule in mammalian cells is surprisingly different from the N-end rule described earlier for yeast and E. *coli.* Indeed, arginine and lysine residues were identified as the most destabilizing residues both in yeast and bacteria (Varshavsky, 1992). In contrast, these residues were just moderately destabilizing in mammalian cells. In all cell lines tested, a glutamic acid residue had the most dramatic effect on the stability of firefly luciferase. At the same time, according to Varshavsky (1992), this residue in yeast had a moderate destabilizing effect and in *E. coli* glutamic acid had no destabilizing effect at all.

The data demonstrate that, in addition to the nature of the host cells, the structure of the protein may have an effect on the N-end rule. Indeed, by introducing a small deletion in the area that, after deubiquitination, becomes the N-terminus, the relative destabilizing properties of histidine and glutamic acid residues could be changed. As a result, in COS-7 cells, luciferase fusion proteins with a N-terminal histidine residue became even more unstable than the corresponding protein with a glutamic acid residue at the N-terminus. It is important to mention that this effect was cell-specific. In CHO cells, the same deletion reduced the half-life of the protein possessing a histidine residue at the N-terminus but did not change the half-life of the protein possessing a glutamic acid residue at the N-terminus (see Figure 6) and, as a result, did not change the status of glutamic acid as a more efficient destabilizing residue than histidine.

Moreover, the addition of a mODC fragment to the C-terminus of firefly luciferase changed the half-life of corresponding proteins but at the same time did not alter the destabilizing effect of a glutamic acid residue relative to arginine and tyrosine. Further, glutamic acid appears to be the most efficient destabilizing residue in the case of *Renilla* luciferase (data not presented). Therefore, the N-end rule determined for one protein may provide a useful guidance to destabilization of other proteins.

In mammalian cells, the N-degron dependent degradation pathway may function less efficiently than it does in yeast cells. Indeed, by positioning Arg, Lys, Phe, Leu, Trp, His, Asp or Asn at the N-terminus of β-galactosidase, Varshavsky and coauthors (Bachmair et al., 1986) were able to reduce the half life of β-galactosidase in yeast from 20 hours to 2-3 minutes. At the same time, in a mammalian cell, even with glutamic acid at the N-terminus, firefly luciferase had a half-life of greater than 45-50 minutes. When compared to the protein degradation signal contained within the C-ODC, N-degron alone does not provide a superior approach to the destabilization of proteins. Nevertheless, the data demonstrate that N-degron and C-ODC can complement each other and the combination of these two degradation signals on the same protein results in an increased rate of protein degradation. Using this combination of degradation signals, a firefly luciferase was generated that in mammalian cells has the shortest half-life among currently described reporter proteins. In contrast, a protein having a degradation signal from listeriolysin 0 and from murine C-ODC had a rate of protein degradation which was similar to a protein having a degradation signal from murine C-ODC (data not shown).

The rapid turnover of reporter proteins in the cell may be invaluable for monitoring fast processes in the cell. Additionally, destabilized reporters can allow for a substantial reduction of time in high-throughput screening experiments. One major disadvantage of destabilized reporter proteins is related to the fact that, because of reduced quantities of such proteins in the cell, the signal available for detection and analysis is weaker than the signal generated by wild-type reporter proteins. For example, cells producing firefly luciferase fusion proteins that possess both N-degron and C-ODC emit almost ten times less light than the same cells producing wild-type luciferase (see Figure 7). Nevertheless, optimization of the sequence encoding reporter protein provides a useful approach to overcome this limitation. Indeed, by using this approach, the signal emitted by cells producing destabilized firefly luciferase was increased almost eight-fold without affecting the half-life of the reporter.

### References

Ausubel, et al., Current Protocols in Molecular Biology. John Wiley & Sons, New York. 1992
Bachmair et al., Science, 234:179 (1986).
Bence et al., Science, 292:1552 (2001).
Bercovich et al., J. Biol. Chem., 264:15949 (1989).
Boshart et al., Cell, 41:521 (1985).
Corish et al., Protein Eng., 12:1035 (1999).
Ghoda et al., Science, 243:1493 (1989).
Ghoda et al., J. Biol. Chem., 265:11823 (1990).
Ghoda et al., Mol. Cell. Biol., 12:2178 (1992).
Gilon et al., Embo J., 17:2759 (1998).
Glotzer et al., Nature, 349:132 (1991).
Gomez et al., PNAS, 79:6777 (1982).
Gossen et al., PNAS, 89:5547 (1992).
Hersko et al., Annu. Rev. Biochem., 61 :761 (1992).
Hicke Faseb J., 11:1215 (1997).
Hunt et al., J. Cell Biol., 116:707 (1992).
Joazeiro et al., Science, 286:309 (1999).
Kim et al., Gene, 91:217 (1990).
King et al., Mol. Biol. Cell, 7:1343 (1996).
Kwon et al., Proc. Natl. Acad. Sci. USA, 95:7898 (1998).
Leclerc et al., Biotechniques, 29:590 (2000).
Li et al., J. Biol. Chem., 273:34970 (1998).
Li et al., Clontech Laboratories, Inc., Palo Alto, Calif. (2000).
Mateus et al., Yeast, 16:1313 (2000).
McNaught et al., Nat. Rev. Neurosci., 2:589 (2001).
Mizushima et al., Nucl. Acids Res., 18:5322 (1990).
Murakami et al., Nature, 360:597 (1992).
Murray et al., Nature, 339:280 (1989).
Reichsteiner et al., Seminars in Cell Biology, 1:433 (1990).
Reiss et al., J. Biol. Chem., 263:2693 (1988).
Rogers et al., Science, 234:364 (1989).
Salghetti et al., Science, 293:1651 (2001).
Sambrook et al., Molecular Cloning: A Laboratory Manual, 2nd ed., Cold Spring Harbor Laboratory Press, New York , 1989
Sung et al., Photochem. Photobiol., 68:749 (1998).
Tobias et al., Science 254:1374 (1991).
Townsend et al., J. Exp. Med., 168:1211 (1988).
Varshavsky, CeM. 69:723 (1992).
Wada et al., Nucl. Acids Res., 15:2367 (1990).
Zdanovskaia et al., J. Protein Chem., 19:699 (2000).

## Claims

1. An isolated nucleic acid molecule comprising a nucleic acid sequence comprising an open reading frame for a reporter protein and at least two heterologous destabilization sequences, wherein one of the heterologous destabilization sequences is a mRNA destabilization sequence and another is a heterologous in frame protein destabilization sequence that includes at least one of CL1, CL2, CL6, CL9, CL10, CL11, CL12, CL15, CL16, or S17, as defined below:
| | |
|---|---|
| CL1 | ACKNWFSSLSHFVIHL |
| CL2 | SLISLPLPTRVKFSSLLLIRIMKITTMTTKKLRS |
| CL6 | FYYPIWFARVLLVHYQ |
| CL9 | SNPFSSLFGASLLIDSVSLKSNWDTSSSSCLISFFSSVMFSSTTRS |
| CL10 | CRQRFSCHLTASYPQSTVTPFLAFLRRDFFFLRHNSSAD |
| CL11 | GAPHVVLFDFELRITNPLSHIQSVSLQITLIFCSLPSLILSKFLQV |
| CL12 | NTPLFSKSFSTTCGVAKKTLLLAQISSLFFLLLSSNIAV |
| CL15 | PTVKNSPKIFCLSSSPYLAFNLEYLSLRIFSTLSKCSNTLLTSLS |
| CL16 | SNQLKRLWLWLLEVRSFDTLRRPWIHLPS |
| SL17 | SISFVIRSHASIRMGASNDFFHKLYFTKCLTSVILSKFLIHLLLRSTPRV |

2. An isolated nucleic acid molecule according to claim 1, wherein the heterologous protein destabilization sequence is CL1, as shown in SEQ ID NO: 12.

3. An isolated nucleic acid molecule according to claim 1, further comprising a promoter operably linked to the nucleic acid sequence.

4. An isolated nucleic acid molecule according to claim 3, wherein the promoter is a regulatable promoter.

5. An isolated nucleic acid molecule according to claim 4, wherein the promoter is an inducible promoter.

6. An isolated nucleic acid molecule according to claim 5, wherein the promoter is a repressible promoter.

7. An isolated nucleic acid molecule according to claim 1, wherein the mRNA destabilization sequence is 3' to said nucleic acid sequence encoding the reporter protein.

8. An isolated nucleic acid molecule according to claim 1, wherein the nucleic acid sequence encoding at least the reporter protein is optimized for expression in a host cell.

9. An isolated nucleic acid molecule according to claim 1, wherein the reporter protein encodes a luciferase.

10. An isolated nucleic acid molecule according to claim 9, wherein the reporter protein encodes a beetle luciferase.

11. An isolated nucleic acid molecule according to claim 10, wherein the reporter protein encodes a click beetle luciferase.

12. An isolated nucleic acid molecule according to claim 9, wherein the reporter protein encodes an anthozoan luciferase protein.

13. An isolated nucleic acid molecule according to claim 1, wherein the nucleic acid sequence comprises SEQ ID NO:47, SEQ ID NO:48, SEQ ID NO:49, SEQ ID NO:66, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78, SEQ ID NO:79, SEQ ID NO:80, or a fragment thereof that encodes a fusion polypeptide with substantially the same activity as the corresponding full-length fusion polypeptide encoded by SEQ ID NO:47, SEQ ID NO:48, SEQ ID NO:49, SEQ ID NO:66, SEQ ID NO:69, SEQ ID NO:70, SEQ ID NO.71, SEQ ID NO:72, SEQ ID NO:73, SEQ ID NO:74, SEQ ID NO:75, SEQ ID NO:76, SEQ ID NO:77, SEQ ID NO:78, SEQ ID NO:79 or SEQ ID No:80.

14. An isolated nucleic acid molecule according to claim 1, wherein one heterologous protein destabilization sequence is a PEST sequence.

15. An isolated nucleic acid molecule according to claim 1, wherein one heterologous protein destabilization sequence is from the C-terminus of a mammalian ornithine decarboxylase.

16. An isolated nucleic acid molecule according to claim 1, wherein one heterologous protein destabilization sequence is a mutant ornithine decarboxylase sequence.

17. An isolated nucleic acid molecule according to claim 16, wherein the mutant ornithine decarboxylase sequence has an amino acid substitution at a position corresponding to position 426, 427, 428, 430, 431, 433, 434, 439 or 448 of murine ornithine decarboxylase, wherein residues 423 to 461 correspond to SEQ ID NO: 81.

18. An isolated nucleic acid molecule according to claim 1, wherein one heterologous protein destabilization sequence is at the C-terminus of the reporter protein.

19. An isolated nucleic acid molecule according to claim 8, which encodes a fusion polypeptide with a half-life of expression of about 20 minutes.

20. An isolated nucleic acid molecule according to claim 8, which encodes a fusion polypeptide with a half life of expression of about 30 minutes.

21. A vector comprising the nucleic acid molecule according to claim 1.

22. A vector according to claim 21, wherein the nucleic acid molecule is operably linked to a regulatable promoter.

23. A vector according to claim 21, wherein the promoter is a repressible promoter.

24. A vector according to claim 21, wherein the nucleic acid molecule comprises SEQ ID NO:49, SEQ ID NO:75, SEQ ID NO:76, SEQ ID NO:77, SEQ ID NO:78, SEQ ID NO:79, SEQ ID NO:80 or a fragment thereof that encodes a fusion polypeptide with substantially the same activity as the corresponding full-length fusion polypeptide encoded by SEQ ID NO:49, SEQ ID NO:75, SEQ ID NO:76, SEQ ID NO:77, SEQ ID NO:78, SEQ ID NO:79 or SEQ ID NO:80.

25. A fusion polypeptide encoded by the nucleic acid molecule according to claim 1, wherein the reporter protein is a luciferase, chloramphenicol acetyltransferase or beta-glucuronidase.

26. A fusion polypeptide according to claim 25, wherein the reporter protein is a luciferase.

27. A host cell comprising the vector according to claim 21.

28. A host cell according to claim 27, which is stably transfected with the vector that encodes a fusion polypeptide comprising a luminescent protein.

29. A host cell according to claim 28, wherein the signal emitted by the host cell comprising the vector is greater than the signal emitted by a corresponding host cell comprising a vector which lacks one or more of the destabilization sequences.

30. A stable cell line comprising the vector according to claim 21 wherein the signal emitted by the reporter protein is equal to or greater than a signal emitted by a corresponding stable cell line comprising a vector which lacks one or more of the heterologous destabilization sequences.

31. A method to detect a reporter protein in a cell, comprising:
a) contacting a cell with the vector according to claim 21; and
b) detecting or determining the presence or amount of the reporter protein in the cell or a lysate thereof.

## Patentansprüche

1. Isoliertes Nucleinsäuremolekül, umfassend eine Nueleinsäuresequenz, umfassend einen offenen Leserahmen für ein Reporterprotein und mindestens zwei heterologe Destabilisierungssequenzen, wobei eine von den heterologen Destabilisierungssequenzen eine mRNA-Destabilisierungssequenz ist und eine andere eine heterologe Protein-Destabilisierungssequenz im Rahmen ist, die mindestens eine von CL1, CL2, CL6, CL9, CL10, CL11, CL12, CL15, CL16 oder SL17, wie nachstehend definiert, einschließt:
| | |
|---|---|
| CL1 | ACKNOSSLSHFVIHL |
| CL2 | SLISLPLPTRVKFSSLLLIRIMKRTMTFKKKLRS |
| CL6 | FYYPIWFARVLLVHYQ |
| CL9 | SNPFSSLFGASLLIDSVSLKSVSLKNWDTSSSSCLISFFSSVMFSSTTRS |
| CL10 | CRQMCHLTASYPQSTVTPFLAFLRXDFFFLRHNSSAD |
| CL11 | GAPHVVLFDFELRITNPLSHIQSVSLQITLlFCSLPSLILSKILQV |
| CL12 | NTPLFSKSFSTTCGVAKKTLLLAQISSLPFLLLSSNIAV |
| CL15 | PTVKNSPKIFCLSSSPYLAFNLEYLSLRIFSTLSKCSNTLLTSLS |
| CL16 | SNQLKRLWLWLLBVRSFDTLRRPWIHLPS |
| SL17 | SISFVIRSHASIRMGASNDFFHXLYFTTGCLTSYITSKFLIFILLLITSPRV |

2. Isoliertes Nucleinsäuremolekül gemäß Anspruch 1, wobei die heterologe Protein-Destabilisierungssequenz CL1, wie angegeben in SEQ ID NO: 12, ist.

3. Isoliertes Nucleinsäuremolekül gemäß Anspruch 1, weiterhin umfassend einen Promotor, operabel verknüpft mit der Nucleinsäuresequenz.

4. Isoliertes Nucleinsämemolekül gemäß Anspruch 3, wobei der Promotor ein regulierbarer Promotor ist.

5. Isoliertes Nucleinsäuremoleküt gemäß Anspruch 4, wobei der Promotor ein induzierbarer Promotor ist.

6. Isoliertes Nucleinsäuremolekül gemäß Anspruch 5, wobei der Promotor ein reprimierbarer Promotor ist.

7. Isoliertes Nucleinsäuremolekül gemäß Anspruch 1, wobei die mRNA-Destabilisierungssequenz 3' zu der Nucleinsäuresequenz, codierend das Reporterprotein, ist.

8. Isoliertes Nucleinsäuremolekül gemäß Anspruch 1, wobei die Nucleinsäuresequenz, codierend zumindest das Reporterprotcin, für Expression in einer Wirtszelle optimiert ist.

9. Isoliertes Nueteinsäuremolekül gemäß Anspruch 1, wobei das Reporterprotein eine Luciferase codiert.

10. Isoliertes Nucleinsäuremolekül gemäß Anspruch 9, wobei das Reporterprotein eine Käufer-Luciferasc codiert.

11. Isoliertes Nucleinsäuremolekül gemäß Anspruch 10, wobei das Reporterprotein eine Schnellkäfer-Luciferase codiert.

12. Isoliertes Nucleinsäuremolekül gemäß Anspruch 9, wobei das Reporterprotein ein Anthozoen-Luciferase-Protein codiert.

13. Isoliertes Nucleinsäuremolekül gemäß Anspruch 1, wobei die Nucleinsäuresequenz SEQ ID NO:47, SEQ ID NO:48, SEQ ID NO:49, SEQ ID NO:66, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID N0:77, SEQ ID NO:78, SEQ ID NO:79, SEQ ID NO:80 umfasst, oder ein Fragment davon, das ein Fusionspolypeptid mit im wesentlichen der gleichen Aktivität wie das entsprechende Fusionspolypeptid mit voller Länge, codiert durch SEQ ID NO:47, SEQ ID NO:48, SEQ ID NO:49, SEQ ID NO:66, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78, SEQ ID NO:79 oder SEQ ID NO:80, codiert.

14. Isoliertes Nucleinsäuremolekül gemäß Anspruch 1, wobei eine heterologe Protein-Destabilisierwgssequenz eine PEST-Sequenz ist.

15. Isoliertes Nucleinsäuremolekül gemäß Anspruch 1, wobei eine heterologe Protein-Destabilisicrungssequenz von dem C-Terminus einer Säuger-Ornithin-Decarboxylase ist.

16. Isoliertes Nucleinsäuremolekül gemäß Anspruch 1, wobei eine heterologe Protein-Destabilisierungssequenz eine mutante Ornithin-Decarboxylase-Sequenz ist.

17. Isoliertes Nucleinsäuremolekül gemäß Anspruch 16, wobei die mutante Ornithin-Decarboxylase Sequenz eine Aminosäuresubstitution an einer Position entsprechend Position 426, 427, 428, 430, 431, 433, 434, 439 oder 448 von muriner Ornithin-Decarboxylase hat, wobei die Reste 423 bis 461 SEQ ID NO: 81 entsprechen.

18. Isoliertes Nucleinsäuremolekül gemäß Anspruch 1, wobei eine heterologe Protein-Destabilisiertingssequenz an dem C-Terminus des Reporter-Proteins ist.

19. Isoliertes Nucleinsäuremolekül gemäß Anspruch 8, welches ein Fusionspolypeptid mit einer Halbwertszeit der Expression von etwa 20 Minuten codiert.

20. Isoliertes Nucleinsäuremolekül gemäß Anspruch 8, welches ein Fusionspolypeptid mit einer Halbwertszeit der Expression von etwa 30 Minuten codiert.

21. Vektor, umfassend das Nucleinsäuremolekül gemäß Anspruch 1.

22. Vektor gemäß Anspruch 21, wobei das Nucleinsäuremolekül operabel mit einem regulierbaren Promotor verknüpft ist.

23. Vektor gemäß Anspruch 21, wobei der Promotor ein reprimierbarer Promotor ist.

24. Vektor gemäß Anspruch 21, wobei das Nucleinsäuremolekül SEQ ID NO:49. SEQ ID NO:75, SEQ ID NO:76, SEQ ID NO:77, SEQ ID NO:78, SEQ ID NO:79, SEQ ID NO:80 umfasst, oder ein Fragment davon, das ein Fusionspolypeptid mit im wesentlichen der gleichen Aktivität wie das entsprechende Fusionspolypeptid mit voller Länge, codiert durch SEQ ID NO:49, SEQ ID NO:75, SEQ ID NO:76, SEQ ID NO:77, SEQ ID NO:78, SEQ ID NO:79 oder SEQ ID NO:80, codiert.

25. Fusionspolypeptid, codiert durch das Nucleinsäuremolekül gemäß Anspruch 1, wobei das Reporterprotein eine Luciferase, Chloramphenicol-Acetyltransferase oder beta-Glucuronidase ist.

26. Fusionspolypeptid gemäß Anspruch 25, wobei das Reporterprotein eine Luciferase ist.

27. Wirtszelle, umfassend den Vektor gemäß Anspruch 21.

28. Wirtszelle gemäß Anspruch 27, welche mit dem Vektor stabil transfiziert ist, der ein Fusionspolypeptid, umfassend ein lumineszentes Protein, codiert.

29. Wirtszelle gemäß Anspruch 28, wobei das Signal, emittiert von der Wirtszelle, umfassend den Vektor, größer ist als das Signal, emittiert von einer entsprechenden Wirtszelle, umfassend einen Vektor, welchem eine oder mehrere der Destabilisierungssequenzen fehlen.

30. Stabile Zell-Linie, umfassend den Vektor gemäß Anspruch 21, wobei das Signal, emittiert von dem Reporterprotein, gleich ist oder größer als ein Signal, emittiert von einer entsprechenden stabilen Zell-Linie, umfassend einen Vektor, welchem eine oder mehrere der heterologen Destabilisierungssequenzen fehlen.

31. Verfahren, um ein Reportcrprotein in einer Zelle nachzuweisen, umfassend:
a) Inkontaktbringen einer Zelle mit dem Vektor gemäß Anspruch 21; und
b) Nachweisen oder Bestimmen der Anwesenheit oder Menge des Reporterproteins in der Zelle oder einem Lysat davon.

## Revendications

1. Molécule d'acide nucléique isolé comprenant une séquence d'acide nucléique comprenant un cadre de lecture ouvert pour une protéine rapporteur et au moins deux séquences hétérologues de déstabilisation, dans laquelle l'une des séquences hétérologues de déstabilisation est une séquence de déstabilisation d'ARNm et l'autre est une séquence hétérologue de déstabilisation de protéine en cadre qui comprend au moins l'une d'entre CL1, CL2, CL6, CL9, CL10, CL11, CL12, CL15, CL16 ou SL17, telles que définies ci-dessous:
| | |
|---|---|
| CL1 | ACKNWFSSLSHFVIHL |
| CL2 | SLISLPLPTRVKFSSLLLIRIMKIITMTFKKLRS |
| CL6 | FYYPIWFARVLLVHYQ |
| CL9 | SNPFSSLFGASLLIDSVSLKSNWDTSSSSCLISFFSSVMFSSTTRS |
| CL10 | CRQRFSCHLTASYPQSTYTPFLAFLRRDFFFLRHNSSAD |
| CL11 | GAPHVVLFDFELRITNPLSHIQSVSLQITLIFCSLPSLILSKFLQV |
| CL12 | NTPLFSKSFSTTCGVAKKTLLLAQISSLFFLLLSSNIAV |
| CL15 | PTVKNSPKIFCLSSSPYLAFNLEYLSLRIFSTLSKCSNTLLTSLS |
| CL16 | SNQLKRLWLWLLEVRSFDTLRRPWIHLPS |
| SL17 | SISFVIRSHASIRMGASNDFFHKLYFTKCLTSVILSKFLIHLLLRSTPRV |

2. Molécule d'acide nucléique isolé selon la revendication 1, dans laquelle la séquence hétérologue de déstabilisation de protéine est CL1, telle qu'illustrée dans la SEQ ID NO:12.

3. Une molécule d'acide nucléique isolé selon la revendication 1, comprenant en outre un promoteur lié de manière opérationnelle à la séquence d'acide nucléique.

4. Molécule d'acide nucléique isolé selon la revendication 3, dans laquelle le promoteur est un promoteur régulable.

5. Molécule d'acide nucléique isolé selon la revendication 4, dans laquelle le promoteur est un promoteur inductible.

6. Molécule d'acide nucléique isolé selon la revendication 5, dans laquelle le promoteur est un promoteur répressible.

7. Molécule d'acide nucléique isolé selon la revendication 1, dans laquelle la séquence de déstabilisation d'ARNm est en 3' de ladite séquence d'acide nucléique codant la protéine rapporteur.

8. Molécule d'acide nucléique isolé selon la revendication 1, dans laquelle la séquence d'acide nucléique codant au moins la protéine rapporteur, est optimisée pour l'expression dans une cellule hôte.

9. Molécule d'acide nucléique isolé selon la revendication 1, dans laquelle la protéine rapporteur code une luciférase.

10. Molécule d'acide nucléique isolé selon la revendication 9, dans laquelle la protéine rapporteur code une luciférase de coléoptère.

11. Molécule d'acide nucléique isolé selon la revendication 10, dans laquelle la protéine rapporteur code une luciférase de taupin.

12. Molécule d'acide nucléique isolé selon la revendication 9, dans laquelle la protéine rapporteur code une protéine luciférase d'anthozoaire.

13. Molécule d'acide nucléique isolé selon la revendication 1, dans laquelle la séquence d'acide nucléique comprend la SEQ ID NO:47, la SEQ ID NO:48, la SEQ ID NO:49, la SEQ ID NO:66, la SEQ ID NO:69, la SEQ ID NO:70, la SEQ ID NO:71, la SEQ ID NO:72, la SEQ ID NO:73, la SEQ ID NO:74, la SEQ ID NO:75, la SEQ ID NO:76, la SEQ ID NO:77, la SEQ ID NO:78, la SEQ ID NO:79, la SEQ ID NO:80 ou un fragment de celles-ci qui code un polypeptide de fusion doté de sensiblement la même activité que le polypeptide de Fusion entier correspondant codé par la SEQ ID NO:47, la SEQ ID NO:48, la SEQ ID NO:49, la SEQ ID NO:66, la SEQ ID NO:69, la SEQ ID NO:70, la SEQ ID NO:71, la SEQ ID NO:72, la SEQ ID NO:73, la SEQ ID NO:74, la SEQ ID NO:75, la SEQ ID NO:76, la SEQ ID NO:77, la SEQ ID NO:78, la SEQ ID NO:79 ou la SEQ ID NO:80.

14. Molécule d'acide nucléique isolé selon la revendication 1, dans laquelle une séquence hétérologue de déstabilisation de protéine est une séquence PEST.

15. Molécule d'acide nucléique isolé selon la revendication 1, dans laquelle une séquence hétérologue de déstabilisation de protéine provient de ta terminaison C d'une ornithine décarboxylase mammalienne.

16. Molécule d'acide nucléique isolé selon la revendication 1, dans laquelle une séquence hétérologue de déstabilisation de protéine est une séquence d'ornithine décarboxylase mutante.

17. Molécule d'acide nucléique isolé selon la revendication 16, dans laquelle la séquence d'ornithine décarboxylase mutante a une substitution d'acide aminé à une position correspondant à la position 426, 427, 428, 430, 431, 433, 434, 439 ou 448 d'ornithine décarboxylase murine, où les résidus 423 à 461 correspondent à la SEQ ID NO:81.

18. Molécule d'acide nucléique isolé selon la revendication 1, dans laquelle une séquence hétérologue de déstabilisation de protéine se trouve à la terminaison C de la protéine rapporteur.

19. Molécule d'acide nucléique isolé selon la revendication 8, qui code un polypeptide de fusion doté d'une demi-vie d'expression d'environ 20 minutes.

20. Molécule d'acide nucléique isolé selon la revendication 8, qui code un polypeptide de fusion doté d'une demi-vie d'expression d'environ 30 minutes.

21. Vecteur comprenant la molécule d'acide nucléique selon la revendication 1.

22. Vecteur selon la revendication 21, dans lequel la molécule d'acide nucléique est liée de manière opérationnelle à un promoteur régulable.

23. Vecteur selon la revendication 21, dans lequel le promoteur est un promoteur répressible.

24. Vecteur selon la revendication 21, dans lequel la molécule d'acide nucléique comprend la SEQ ID NO:49, la SEQ ID NO:75, la SEQ ID NO:76, la SEQ ID NO:77, la SEQ ID NO:78, la SEQ ID NO:79, la SEQ ID NO:80 ou un fragment de celles-ci qui code un polypeptide de fusion doté de sensiblement la même activité que le polypeptide de fusion entier correspondant codé par la SEQ ID NO:49, la SEQ ID NO:75, la SEQ ID NO:76, la SEQ ID NO:77, la SEQ ID NO:78, la SEQ ID NO:79 ou la SEQ ID NO:80.

25. Polypeptide de fusion codé par la molécule d'acide nucléique selon la revendication 1, dans lequel la protéine rapporteur est une luciférase, de la chloramphénicol acétyltransférase ou de la bêta-glucuronidase.

26. Polypeptide de fusion selon la revendication 25, dans lequel la protéine rapporteur est une luciférase.

27. Cellule hôte comprenant le vecteur selon la revendication 21.

28. Cellule hôte selon la revendication 27, qui est transfectée de manière stable par le vecteur qui code un polypeptide de fusion comprenant une protéine luminescente.

29. Cellule hôte selon la revendication 28, dans laquelle le signal émis par la cellule hôte comprenant le vecteur, est plus fort que le signal émis par une cellule hôte correspondante comprenant un vecteur qui est dépourvu d'une ou de plusieurs des séquences de déstabilisation.

30. Lignée cellulaire stable comprenant le vecteur selon la revendication 21, dans laquelle le signal émis par la protéine rapporteur est égal à ou plus fort qu'un signal émis par une lignée cellulaire stable correspondante comprenant un vecteur qui est dépourvu d'une ou de plusieurs des séquences hétérologues de déstabilisation.

31. Procédé de détection d'une protéine rapporteur dans une cellule, comprenant:
a) mettre une cellule en contact avec le vecteur selon la revendication 21; et
b) détecter ou déterminer la présence ou la quantité de la cellule rapporteur dans la cellule ou lysat de la même.
